# EUROPEAN PATENT APPLICATION

(11) **EP 1 072 593 A1**
(43) Date of publication of application: **31.01.2001**
(21) Application number: 99910777.4
(22) Date of filing: 30.03.1999
(51) Int. Cl.: C07D 211/14, C07D 405/04, C07D 405/08, C07F 7/10, C09K 19/34, C09K 19/42, G02F 1/13

(54) **LIQUID CRYSTALLINE COMPOUND HAVING PIPERIDINE RING, LIQUID CRYSTAL COMPOSITION AND LIQUID CRYSTAL DISPLAY ELEMENT**

(30) Priority: 10.04.1998 JP 11619898
(71) Applicant: CHISSO CORPORATION, Osaka-shi, Osaka-fu 530-0005 (JP)
(72) Inventor: TAMURA, Norio, Chiba 290-0003 (JP); FUJITA, Atsuko, Chiba 266-0011 (JP); MATSUI, Shuichi, Chiba 290-0003 (JP); TAKEUCHI, Hiroyuki, Chiba 290-0022 (JP); TOMI, Yoshitaka, Chiba 290-0003 (JP); TAKESHITA, Fusayuki, Chiba 299-0261 (JP); NAKAGAWA, Etsuo, Chiba 290-0056 (JP)
(74) Representative: HOFFMANN - EITLE
(86) International application number: JP9901630
(87) International publication number: WO9952871

(57) **Abstract**

The present invention relates to a novel liquid crystalline compound represented by Formula (1): 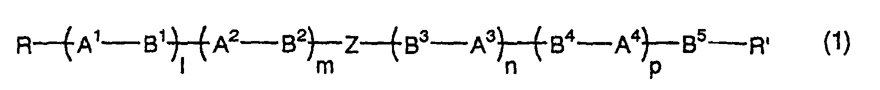 wherein R represents an alkyl group having 1 to 10 carbon atoms or a fluoroalkyl group having 1 to 10 carbon atoms, and R' represents an alkyl group having 1 to 10 carbon atoms, a fluoroalkyl group having 1 to 10 carbon atoms, a fluorine atom, a chlorine atom, a bromine atom or a cyano group, in which any methylene groups which are not adjacent to each other in these alkyl groups and any methylene groups and/or fluoromethylene groups which are not adjacent to each other in the fluoroalkyl groups may be substituted with oxygen atoms, sulfur atoms or -CH=CH-; the rings A¹, A², A³ and A⁴ each independently represent 1,4-cyclohexylene, 1,4-phenylene in which 1 to 2 hydrogen atoms may be substituted with fluorine atoms, dioxane-2,5-diyl, pyrimidine-2,5-diyl, pyridine-2,5-diyl, piperidine-1,4-diyl or 1-sila-1,4-cyclohexylene; B¹, B², B³, B⁴ and B⁵ each independently represent a single bond, -CH₂CH₂-, -CH=CH-, -OCH₂-, -CH₂O-, -(CH₂)₄-, -OCO- or -COO-; l, m, n and p each independently represent 0 or 1; and Z represents one group selected from the following moieties (I) to (V): 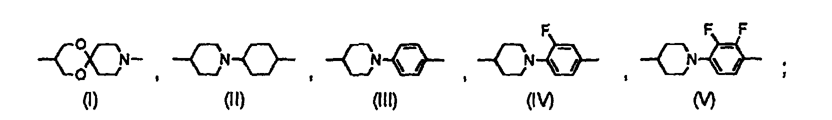 provided that
when Z is the moiety (II), l + m + n + p > 0, and the rings A¹, A², A³ and A⁴ each independently represent 1,4-phenylene in which 1 to 2 hydrogen atoms are substituted with fluorine atoms, pyrimidine-2,5-diyl, pyridine-2,5-diyl, piperidine-1,4-diyl or 1-sila-1,4-cyclohexylene; and that when Z is the moiety (III) and l = m = n = p = 0, R is an alkyl group, and R' is not a fluorine atom or a cyano group when B⁵ is a single bond. The present invention further relates to a liquid crystal composition comprising the above compound and a liquid crystal display element comprising the above composition.

The liquid crystalline compound of the present invention has a particularly large dielectric anisotropy and refractive anisotropy and a low viscosity and is excellent in a low temperature compatibility.

## Description

### Technical Field

The present invention relates to a liquid crystalline compound and a liquid crystal composition, more specifically to a liquid crystalline compound having a piperidine ring as a component of a liquid crystal composition, a liquid crystal composition comprising the said compound and a liquid crystal display element comprising the said composition. In the present application, the term "liquid crystalline compound" is used as a generic name for a compound showing a liquid crystal phase and a compound showing no liquid crystal phase but useful as a component of a liquid crystal composition.

### Background Art

A liquid crystal display element makes use of optical anisotropy and dielectric anisotropy of a liquid crystalline compound and is divided into a twisted nematic (TN) mode, a super twisted nematic (STN) mode, a dynamic scattering (DS) mode, a guest-host (GH) mode, a DAP mode, etc. according to electro-optical effects from a viewpoint of a display mode. From a viewpoint of a driving mode, it is also divided into modes of static driving, time shearing driving, active matrix driving, two-frequency driving, and the like.

In recent years, a display element having a particularly high quality has been required, which has grown in demand for a display element of active matrix driving represented by a thin film transistor (TFT) mode. In order to increase a response speed to a change in an electric field and to reduce a driving voltage, a liquid crystal material having a lower viscosity and a larger dielectric anisotropy (Δε) has further been required.

Recently, attentions have been paid to an in-plane switching (IPS) mode and a vertical aligning (VA) mode which can obtain a broad angle of visibility. It is recognized that a liquid crystal material having a largely negative dielectric anisotropy (Δε) is suited to a liquid crystal composition used for the above modes, and such a material has been required to be developed.

A liquid crystalline substance used for such a liquid crystal display element is required to show a liquid crystal phase in a wide temperature range and to be stable against heat, light, moisture, air, electric field and electromagnetic radiation. However, all of such requirements can not be satisfied with a single compound at present, and plural kinds or, in some cases, 20 kinds or more of liquid crystalline compounds are mixed and used for display media. Recently, display devices have been used in a severe environment, e.g., at very low temperatures in many cases, and therefore, improvement in a low temperature compatibility has further been required in addition to the various properties described above.

In a display element of an STN mode, a thin liquid crystal cell may be used in order to improve a response speed and a contrast. In a liquid crystal cell, a (Δn·d) value which is a product of a refractive anisotropy (Δn) and a cell thickness (d) has to be maintained constant, and therefore, a liquid crystalline compound having a large Δn is required in a certain case.

Several liquid crystalline compounds having a piperidine skeleton in a molecule are synthesized for the purpose of improving a dielectric anisotropy. Disclosed are, for example, compounds represented by Formulas (13) to (15) in DE4234627A1, JP57-98580A and JP57-193455A, compounds represented by Formula (16) or (17) in DE4234627A1 and compounds represented by Formula (18) in JP58-216157A: 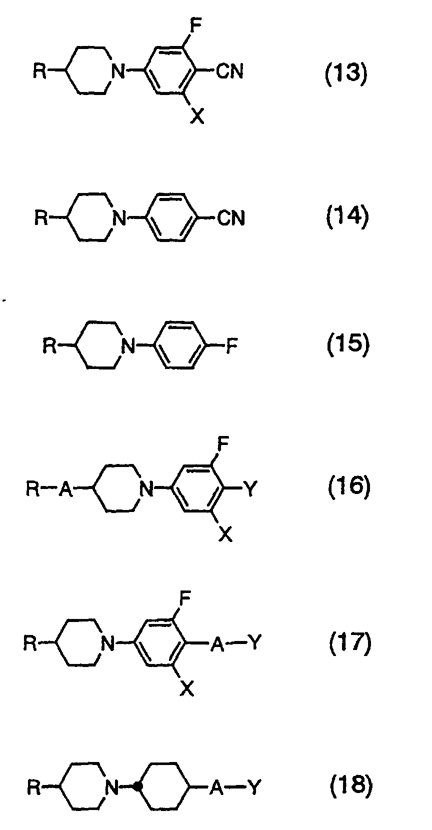 wherein R represents an alkyl group or an alkoxy group; A represents 1,4-cyclohexylene, 1,4-phenylene in which a hydrogen atom may be substituted with a fluorine atom, dioxane-2,5-diyl or the like; X represents a hydrogen atom or a fluorine atom; and Y represents a fluorine atom, a cyano group or the like.

However, the compounds represented by Formulas (13) to (15) and (18) do not show a liquid crystal phase. Further, electro-optical characteristics of the compounds represented by Formula (16) or (17) are not described at all in DE4234627A1.

### Disclosure of the Invention

An object of the present invention is to solve the problems of the prior arts as described above and to provide a novel liquid crystalline compound which has a large dielectric anisotropy and refractive anisotropy and is excellent in compatibility with other liquid crystalline compounds and which has a low viscosity and is chemically and physically stable, a liquid crystal composition comprising this compound and a liquid crystal display element.

The present inventors have made intensive studies to solve the above problems and found a novel compound having improved characteristics as compared with those of publicly known liquid crystalline compounds, thus achieving the present invention.

The present application claims the following inventions.
(1) A novel liquid crystalline compound represented by Formula (1):

   R―A¹―B¹(̵A²―B²)̵Z―B³―A³―B⁴―A⁴―B⁵―R' (1)

   wherein R represents an alkyl group having 1 to 10 carbon atoms or a fluoroalkyl group having 1 to 10 carbon atoms, and R' represents an alkyl group having 1 to 10 carbon atoms, a fluoroalkyl group having 1 to 10 carbon atoms, a fluorine atom, a chlorine atom, a bromine atom or a cyano group, in which any methylene groups which are not adjacent to each other in these alkyl groups and any methylene groups and/or fluoromethylene groups which are not adjacent to each other in the fluoroalkyl groups may be substituted with oxygen atoms, sulfur atoms or -CH=CH-; the rings A¹, A², A³ and A⁴ each independently represent 1,4-cyclohexylene, 1,4-phenylene in which 1 to 2 hydrogen atoms may be substituted with fluorine atoms, dioxane-2,5-diyl, pyrimidine-2,5-diyl, pyridine-2,5-diyl, piperidine-1,4-diyl or 1-sila-1,4-cyclohexylene; B¹, B², B³, B⁴ and B⁵ each independently represent a single bond, -CH₂CH₂-, -CH=CH-, -OCH₂-, -CH₂O-, -(CH₂)₄-, -OCO- or -COO-; l, m, n and p each independently represent 0 or 1; and Z represents one group selected from the following moieties (I) to (V): 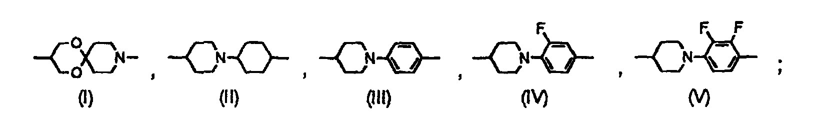 provided that
   when Z is the moiety (II), l + m + n + p > 0, and the rings A¹, A², A³ and A⁴ each independently represent 1,4-phenylene in which 1 to 2 hydrogen atoms are substituted with fluorine atoms, pyrimidine-2,5-diyl, pyridine-2,5-diyl, piperidine-1,4-diyl or 1-sila-1,4-cyclohexylene; and that when Z is the moiety (III) and l = m = n = p = 0, R is an alkyl group, and R' is not a fluorine atom or a cyano group when B⁵ is a single bond.
(2) The compound of Formula (1) as described in the item (1), wherein Z is the moiety (I).
(3) The compound of Formula (1) as described in the item (2), wherein l + m + n + p > 0; the rings A¹, A², A³ and A⁴ each independently represent 1,4-cyclohexylene, 1,4-phenylene in which 1 to 2 hydrogen atoms may be substituted with fluorine atoms, or dioxane-2,5-diyl; B⁵ is a single bond; and R' is a fluorine atom, a cyano group or a fluoroalkoxy group.
(4) The compound of Formula (1) as described in the item (1), wherein Z is the moiety (II).
(5) The compound of Formula (1) as described in the item (4), wherein any of the rings A¹, A², A³ and A⁴ is 1,4-phenylene in which 1 to 2 hydrogen atoms are substituted with fluorine atoms; B⁵ is a single bond; and R' is a fluorine atom, a cyano group or a fluoroalkoxy group.
(6) The compound of Formula (1) as described in the item (1), wherein Z is the moiety (III).
(7) The compound of Formula (1) as described in the item (6), wherein n + p > 0; the ring A³ or A⁴ is 1,4-phenylene in which 1 to 2 hydrogen atoms may be substituted with fluorine atoms; and B⁵ is a single bond.
(8) The compound of Formula (1) as described in the item (7), wherein R' is a fluorine atom, a cyano group or a fluoroalkoxy group.
(9) The compound of Formula (1) as described in the item (6), wherein l + m > 0 and n = p = 0; B⁵ is a single bond; and R' is a fluorine atom, a cyano group or a fluoroalkoxy group.
(10) The compound of Formula (1) as described in the item (1), wherein Z is the moiety (IV).
(11) The compound of Formula (1) as described in the item (1), wherein Z is the moiety (V).
(12) The compound of Formula (1) as described in the item (11), wherein n + p > 1; any of the rings A¹, A², A³ and A⁴ is 1,4-cyclohexylene, or 1,4-phenylene in which 1 to 2 hydrogen atoms may be substituted with fluorine atoms; B⁵ is a single bond; and R and R' are alkyl groups, in which any methylene groups which are not adjacent to each other may be substituted with oxygen atoms or -CH=CH-, or fluoroalkyl groups having 1 to 10 carbon atoms, in which any methylene groups and/or fluoromethylene groups which are not adjacent to each other may be substituted with oxygen atoms or -CH=CH-.
(13) The compound of Formula (1) as described in the item (11), wherein n = p = 0; B⁵ is a single bond; and R and R' each are independently alkyl groups having 1 to 10 carbon atoms, in which any methylene groups which are not adjacent to each other may be substituted with oxygen atoms or -CH=CH-, or fluoroalkyl groups having 1 to 10 carbon atoms, in which any methylene groups and/or fluoromethylene groups which are not adjacent to each other may be substituted with oxygen atoms or -CH=CH-.
(14) A liquid crystal composition comprising at least one of the liquid crystal compounds as described in any one of the items (1) to (13).
(15) A liquid crystal composition comprising at least one liquid crystalline compound as described in any one of the items (1) to (13) as a first component and at least one compound selected from the group consisting of compounds represented by Formulas (2), (3) and (4) as a second component: 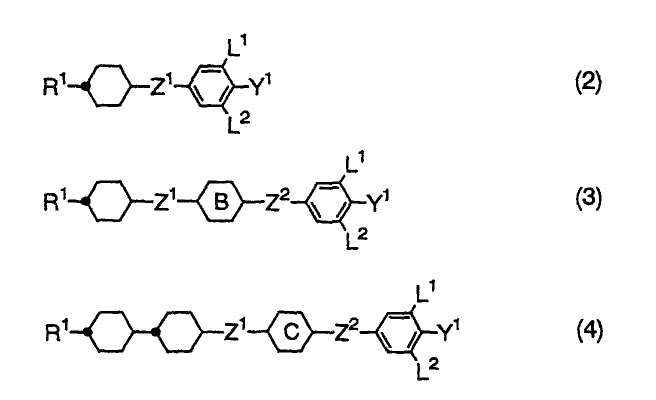 wherein R¹ represents an alkyl group having 1 to 10 carbon atoms, in which any methylene groups which are not adjacent to each other may be substituted with oxygen atoms or -CH=CH-, and in which any hydrogen atoms may be substituted with fluorine atoms; Y¹ represents a fluorine atom, a chlorine atom, -OCF₃, -OCF₂H, -CF₃, -CF₂H, -CFH₂, -OCF₂CF₂H or -OCF₂CFHCF₃ L¹ and L² each independently represent a hydrogen atom or a fluorine atom; Z¹ and Z² each independently represent -(CH₂)₂-, -(CH₂)₄-, -COO-, -CF₂O-, -OCF₂-, -CH=CH- or a single bond; the ring B represents trans-1,4-cyclohexylene, 1,3-dioxane-2,5-diyl, or 1,4-phenylene in which hydrogen atoms may be substituted with fluorine atoms; and the ring C represents trans-1,4-cyclohexylene, or 1,4-phenylene in which hydrogen atoms may be substituted with fluorine atoms.
(16) A liquid crystal composition comprising at least one compound as described in any one of the items (1) to (13) as a first component and at least one compound selected from the group consisting of compounds represented by Formulas (5) and (6) as a second component: 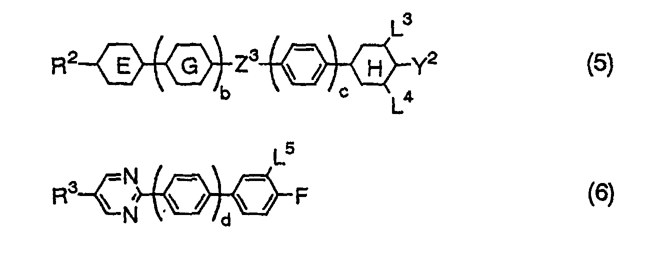 wherein R² and R³ each independently represent an alkyl group having 1 to 10 carbon atoms, in which any methylene groups which are not adjacent to each other may be substituted with oxygen atoms or -CH=CH-, and in which any hydrogen atoms may be substituted with fluorine atoms; Y² represents -CN or -C≡C-CN; the ring E represents trans-1,4-cyclohexylene, 1,4-phenylene, 1,3-dioxane-2,5-diyl or pyrimidine-2,5-diyl; the ring G represents trans-1,4-cyclohexylene, 1,4-phenylene in which hydrogen atoms may be substituted with fluorine atoms or pyrimidine-2,5-diyl; the ring H represents trans-1,4-cyclohexylene or 1,4-phenylene; Z³ represents 1,2-ethylene, -COO- or a single bond; L³, L⁴ and L⁵ each independently represent a hydrogen atom or a fluorine atom; and b, c and d each independently represent 0 or 1.
(17) A liquid crystal composition comprising at least one compound as described in any one of the items (1) to (13) as a first component, at least one compound selected from the group consisting of the compounds represented by Formulas (2), (3) and (4) as a second component and at least one compound selected from the group consisting of compounds represented by Formulas (7), (8) and (9) as a third component: 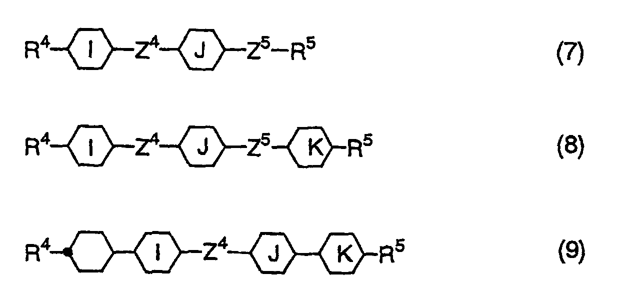 wherein R⁴ and R⁵ each independently represent an alkyl group having 1 to 10 carbon atoms, in which any methylene groups which are not adjacent to each other may be substituted with oxygen atoms or -CH=CH-, and in which any hydrogen atoms may be substituted with fluorine atoms; I, J and K each independently represent trans-1,4-cyclohexylene, pyrimidine-2,5-diyl, or 1,4-phenylene in which hydrogen atoms may be substituted with fluorine atoms; and Z⁴ and Z⁵ each independently represent -C≡C-, -COO-, -CH₂CH₂-, -CH=CH- or a single bond.
(18) A liquid crystal composition comprising at least one compound as described in any one of the items (1) to (13) as a first component and at least one compound selected from the group consisting of compounds represented by Formulas (10), (11) and (12) as a second component: 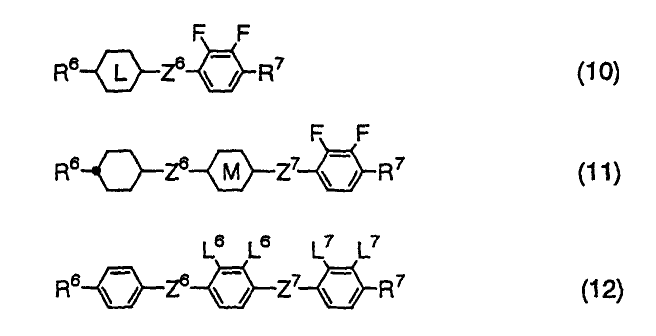 wherein R⁶ and R⁷ each independently represent an alkyl group having 1 to 10 carbon atoms, in which any methylene groups which are not adjacent to each other may be substituted with oxygen atoms or -CH=CH-, and in which any hydrogen atoms may be substituted with fluorine atoms; the rings L and M each independently represent trans-1,4-cyclohexylene or 1,4-phenylene; L⁶ and L⁷ each independently represent a hydrogen atom or a fluorine atom but do not represent hydrogen atoms at the same time; and Z⁶ and Z⁷ each independently represent -CH₂CH₂-, -CH₂O- or a single bond.
(19) A liquid crystal composition comprising at least one compound as described in any one of the items (1) to (13) as a first component, at least one compound selected from the group consisting of the compounds represented by Formulas (7), (8) and (9) as a second component and at least one compound selected from the group consisting of the compounds represented by Formulas (10), (11) and (12) as a third component.
(20) A liquid crystal composition comprising at least one compound as described in any one of the items (1) to (13) as a first component, at least one compound selected from the group consisting of the compounds represented by Formulas (5) and (6) as a second component and at least one compound selected from the group consisting of the compounds represented by Formulas (7), (8) and (9) as a third component.
(21) A liquid crystal composition comprising at least one compound as described in any one of the items (1) to (13) as a first component, at least one compound selected from the group consisting of the compounds represented by Formulas (2), (3) and (4) as a second component, at least one compound selected from the group consisting of the compounds represented by Formulas (5) and (6) as a third component and at least one compound selected from the group consisting of the compounds represented by Formulas (7), (8) and (9) as a fourth component.
(22) A liquid crystal composition further comprising at least one optically active compound in addition to the liquid crystal composition as described in any one of the items (14) to (21).
(23) A liquid crystal display element comprising the liquid crystal composition as described in any one of the items (14) to (22).

Among the liquid crystalline compounds of the present invention represented by Formula (1), compounds represented by the following Formulas (1-1) to (1-64) can be given as examples of the particularly preferred compounds. 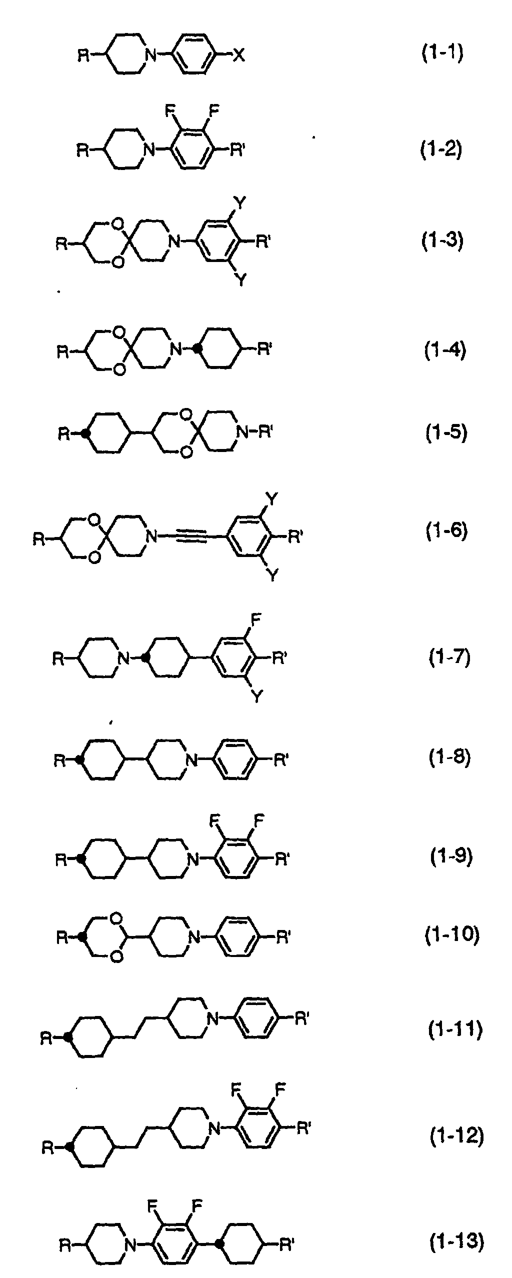 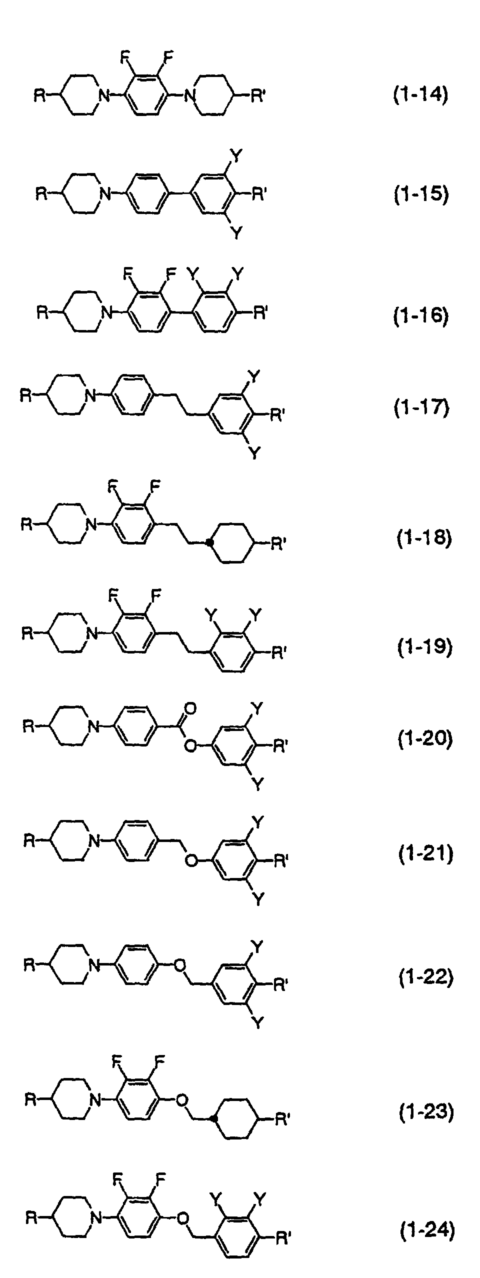 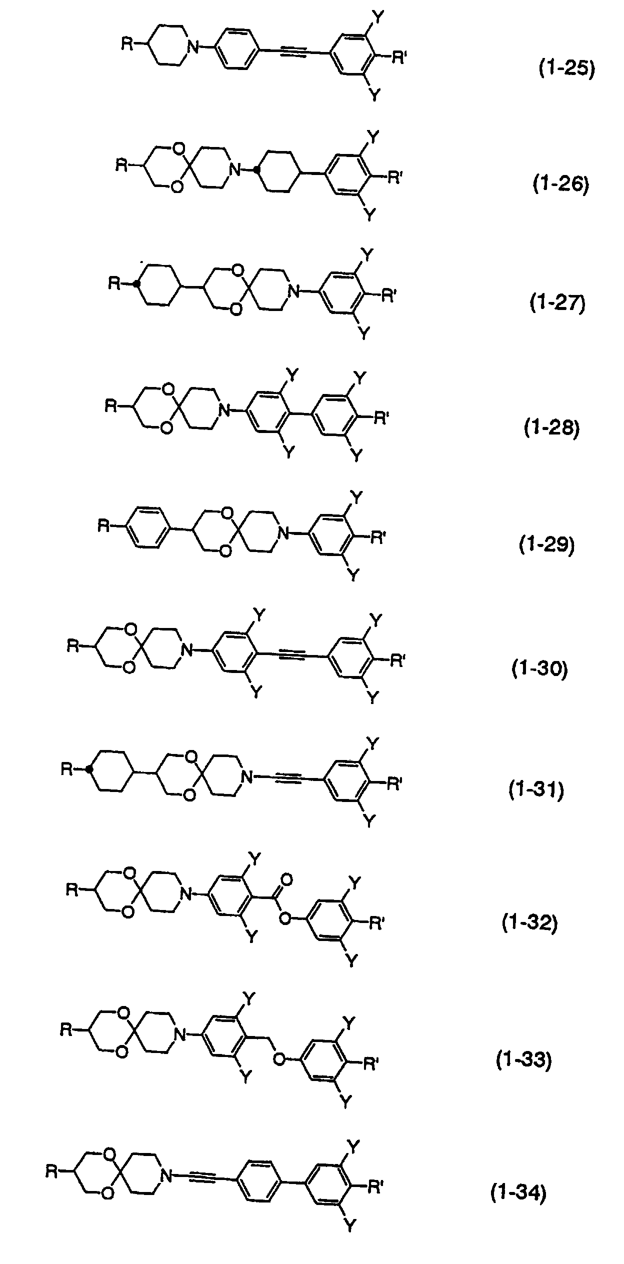 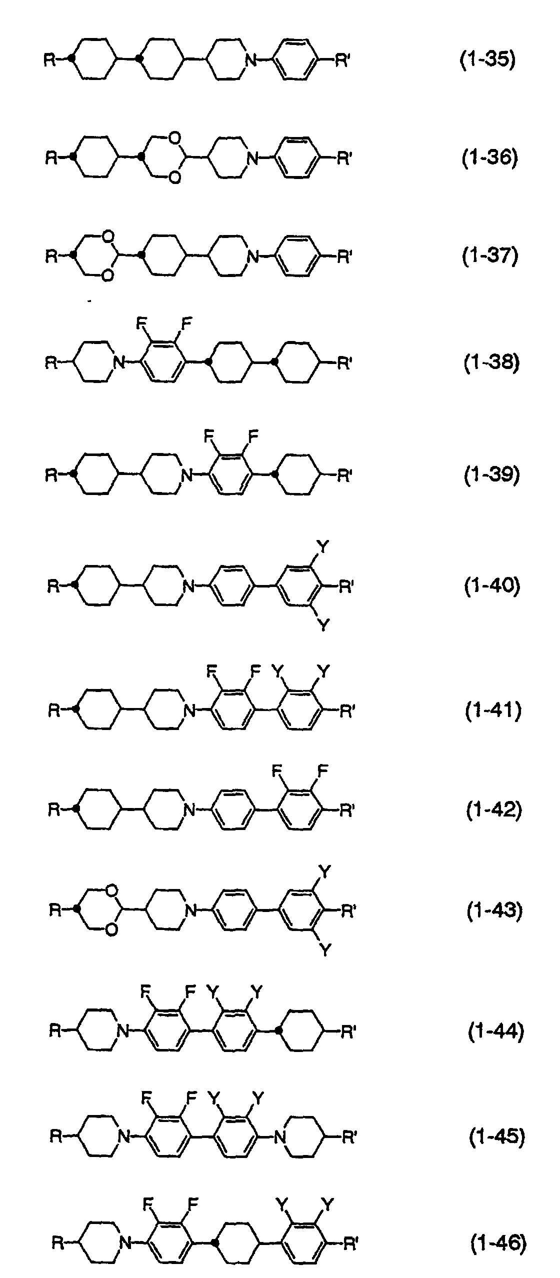 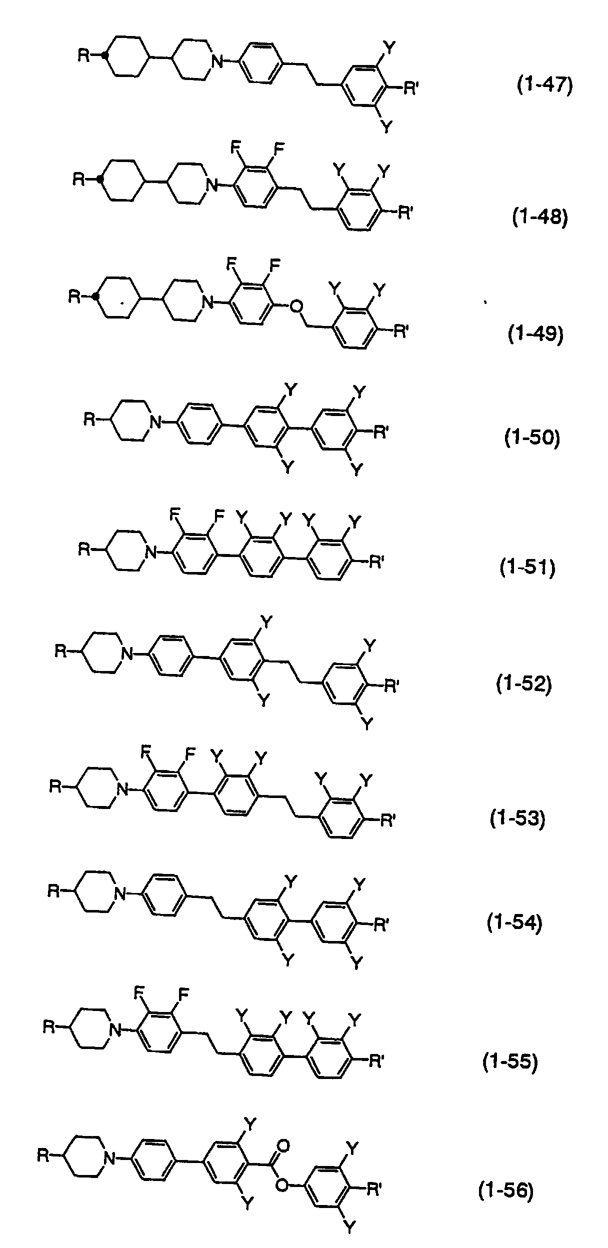 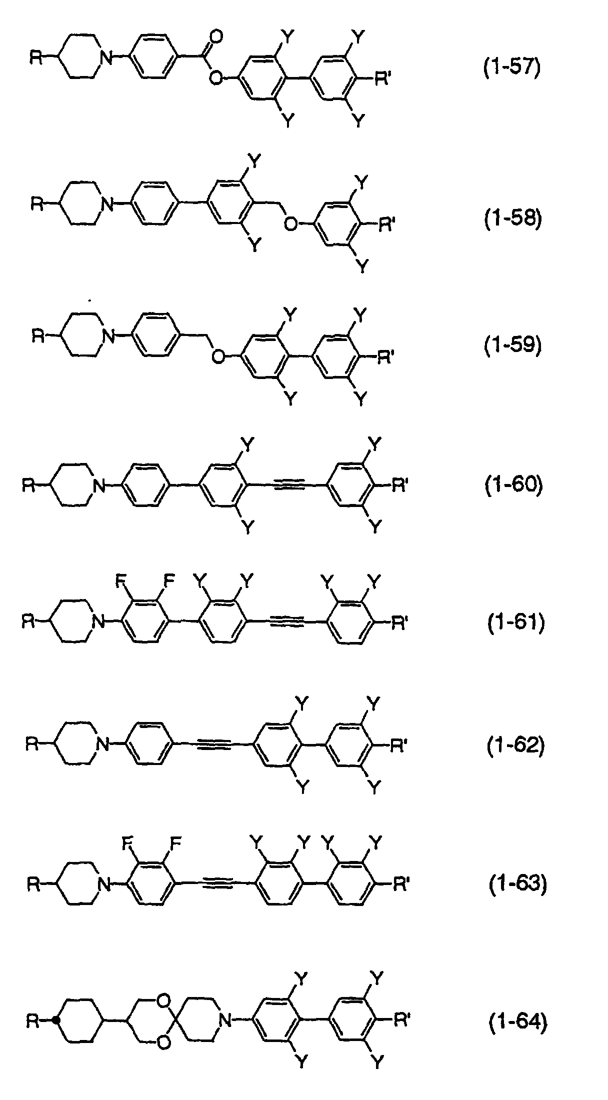 In the above formulas, R and R' have the same meanings as defined above; X represents an alkyl group or fluoroalkyl group having 1 to 10 carbon atoms, in which any methylene groups and/or fluoromethylene groups which are not adjacent to each other may be substituted with oxygen atoms or -CH=CH-, a chlorine atom or a bromine atom; and Y represents a hydrogen atom or a fluorine atom.

All these compounds of the present invention have such excellent characteristics as a very large dielectric anisotropy and refractive anisotropy, a good chemical and physical stability, a low viscosity and a good low temperature compatibility.

In particular, the dicyclic and tricyclic compounds represented by Formulas (1-1) to (1-25) have a relatively high clearing point and a low viscosity, and can therefore increase a response speed of the cell without reducing a clearing point of the liquid crystal composition. Further, the tetracyclic and pentacyclic compounds represented by Formulas (1-26) to (1-64) have a high clearing point, and can therefore expand a liquid crystal display temperature range of the liquid crystal composition and increase a response speed of the cell.

All the liquid crystal compounds of the present invention represented by Formula (1) do not necessarily show a liquid crystal phase. However, all the liquid crystalline compounds represented by Formula (1) have a good compatibility with other liquid crystalline compounds and do not markedly lower or narrow a nematic phase temperature range of the other liquid crystalline compounds when mixed with them. Accordingly, the liquid crystalline compound represented by Formula (1) is a useful component of a liquid crystal composition even if the compound itself does not show a liquid crystal phase.

The liquid crystal composition of the present invention comprises at least one liquid crystalline compound represented by Formula (1) as the first component.

The content of the compound is preferably 0.1 to 99.9% by weight based on the total weight of the liquid crystal composition in order to prepare the composition having the excellent characteristics.

The liquid crystal composition of the present invention may comprise only the first component but preferably comprises a compound obtained by mixing the first component with at least one compound (hereinafter referred to as a second A component) selected from the group consisting of the compounds represented by Formulas (2), (3) and (4) and/or at least one compound (hereinafter referred to as a second B component) selected from the group consisting of the compounds represented by Formulas (5) and (6) or a compound obtained by mixing the above components with at least one compound selected from the group consisting of the compounds represented by Formulas (7), (8) and (9) as the third component. An optically active compound and publicly known other liquid crystalline compounds can further be mixed therewith as other components for the purpose of controlling the threshold voltage, the liquid crystal temperature range, the refractive anisotropy (Δn), the Δε and the viscosity.

Among the second A components described above, given below are compounds represented by Formulas (2-1) to (2-9) as preferable examples of the compounds represented by Formula (2), compounds represented by Formulas (3-1) to (3-69) as preferable examples of the compounds represented by Formula (3) and compounds represented by Formulas (4-1) to (4-24) as preferable examples of the compounds represented by Formula (4), respectively. 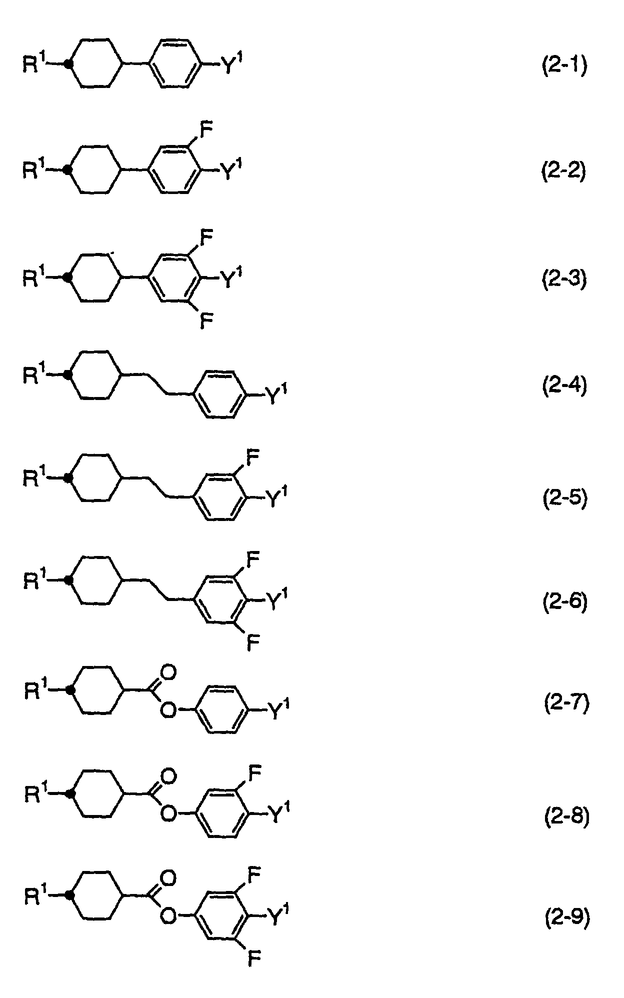 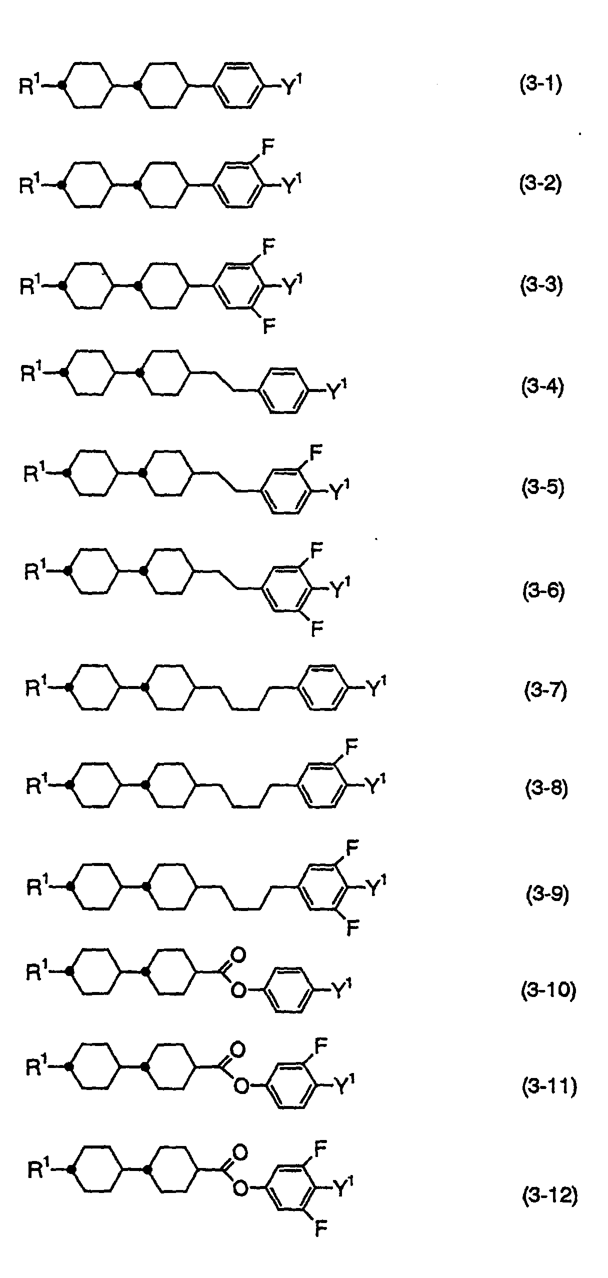 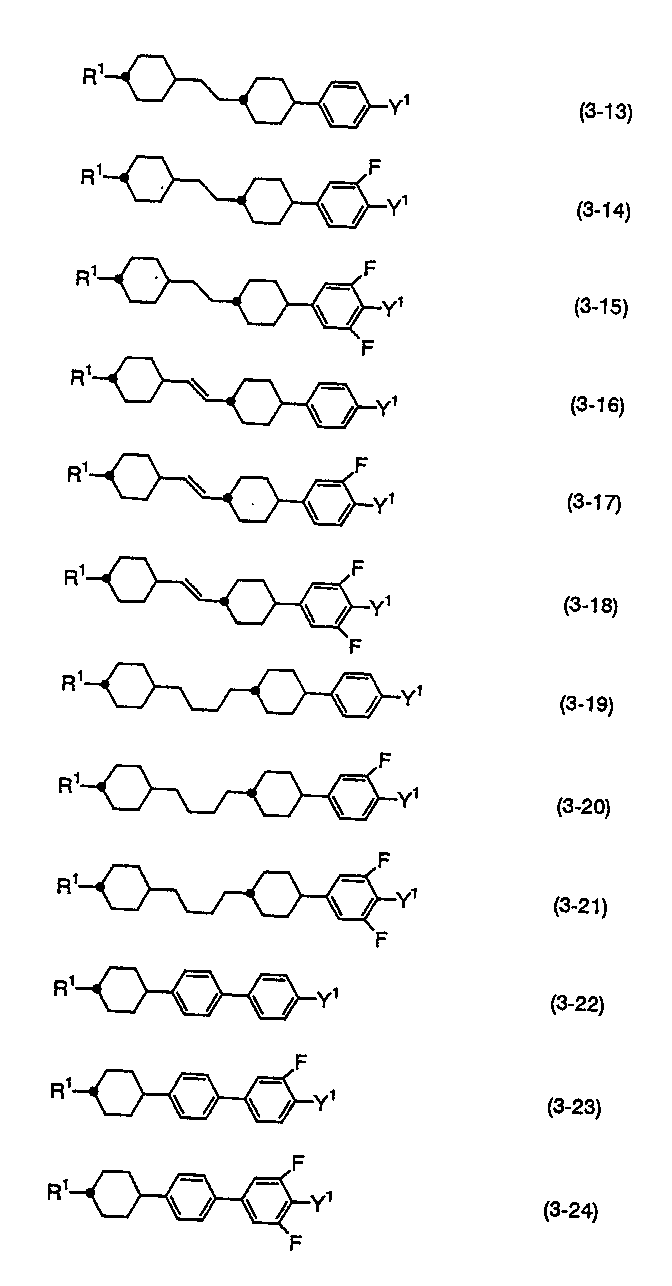 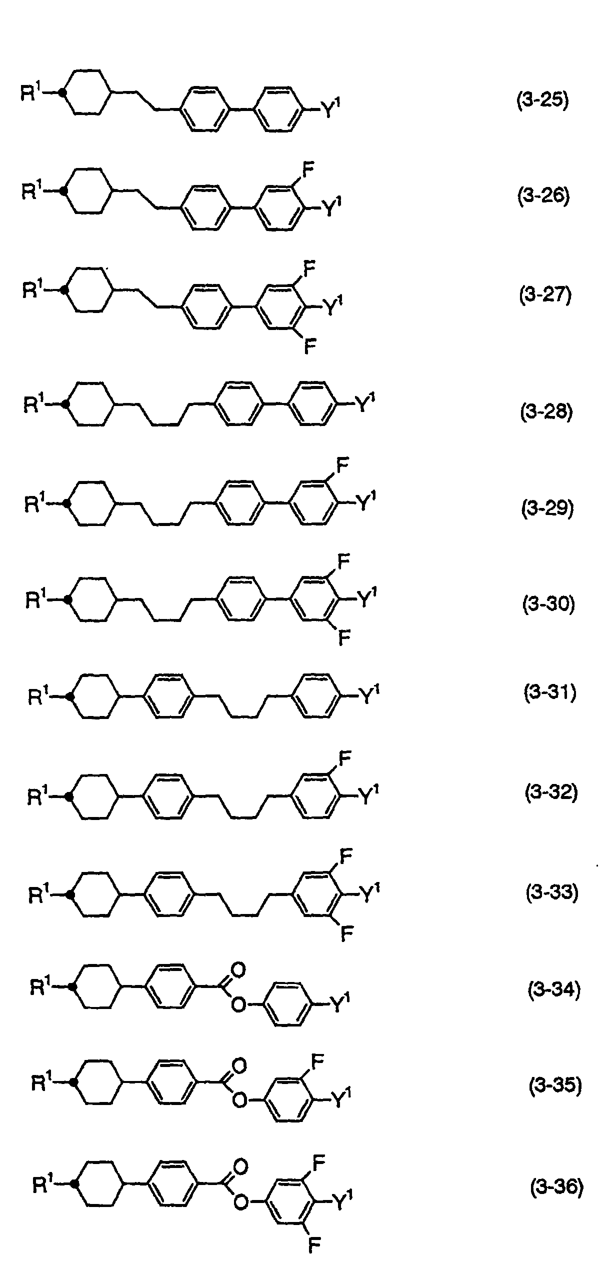 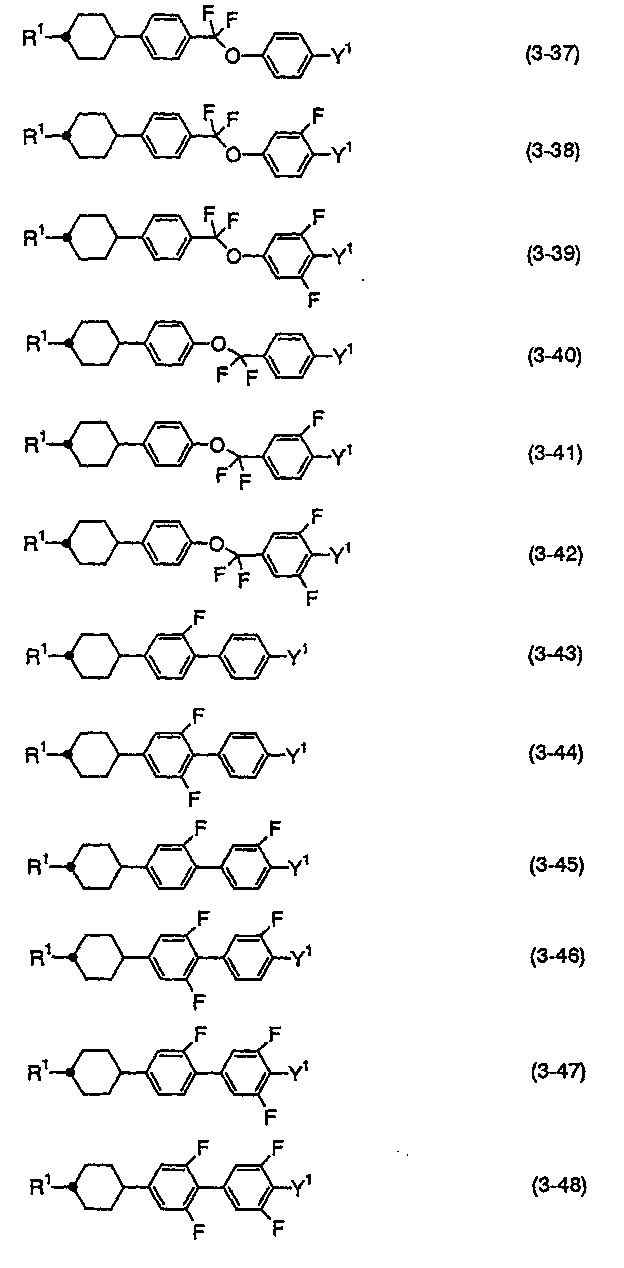 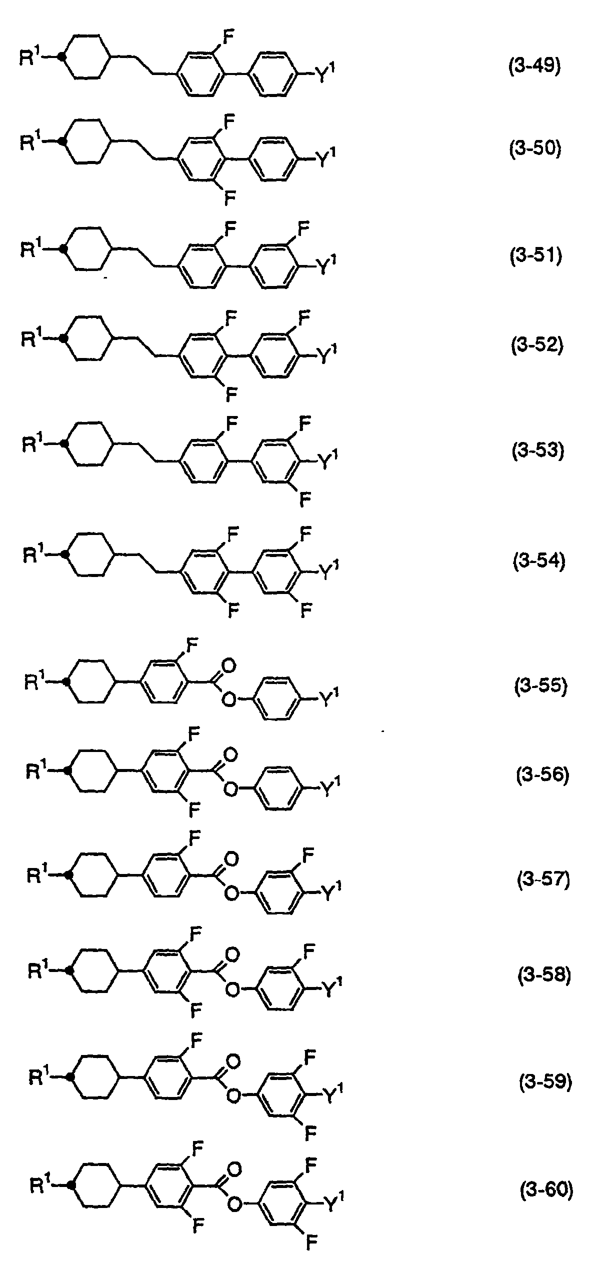 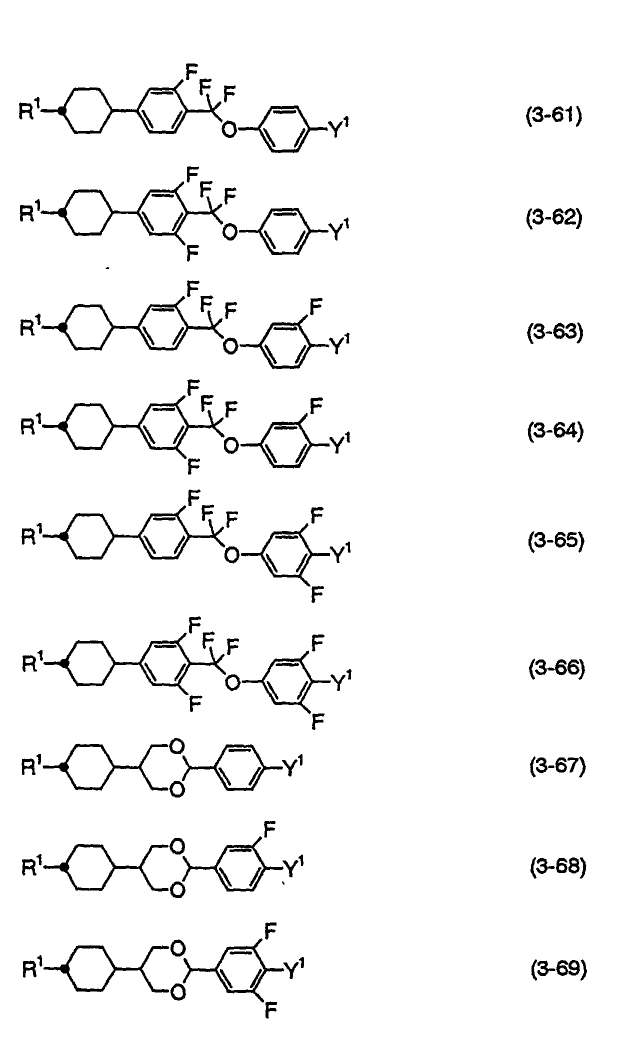 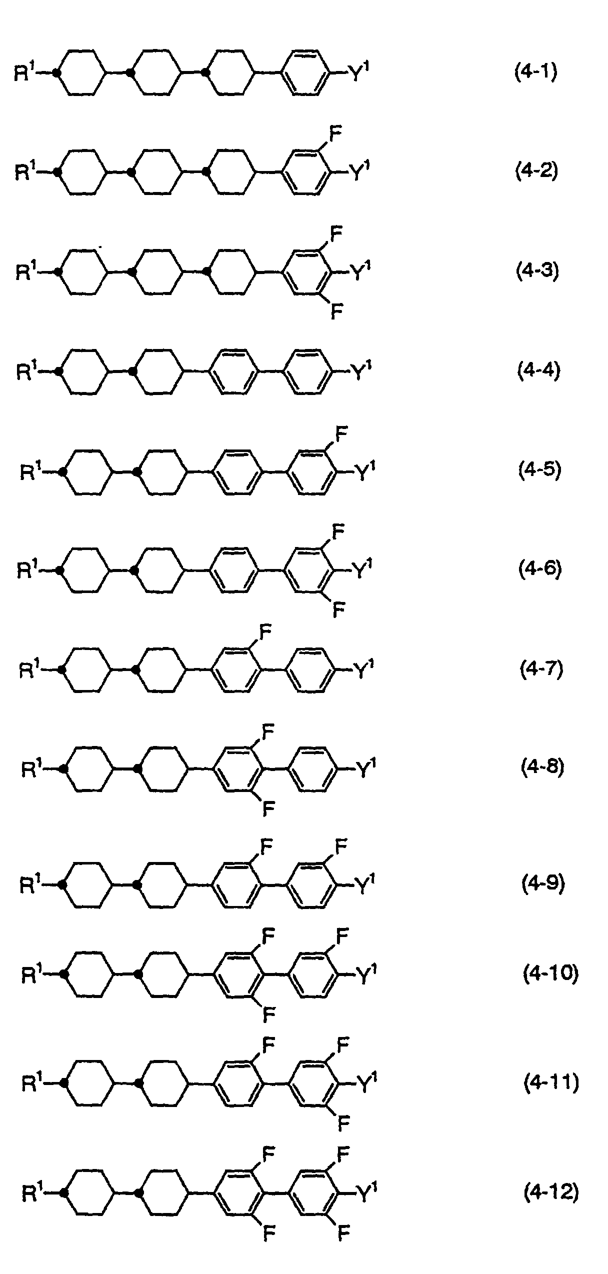 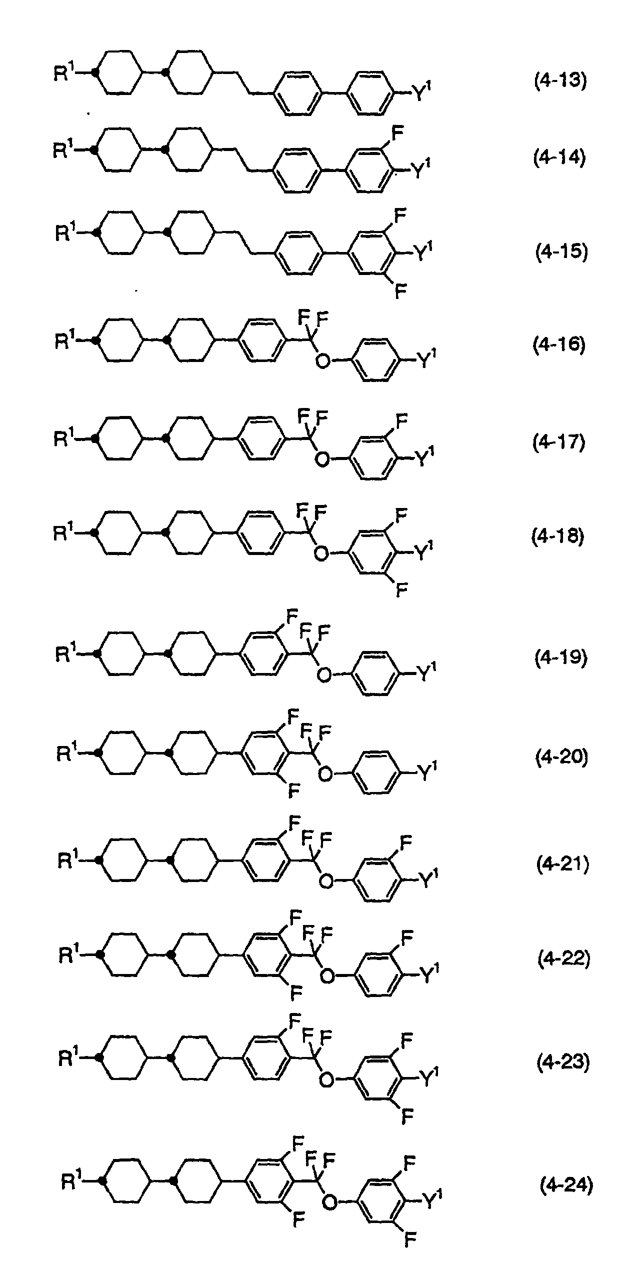 In the above formulas, R¹ and Y¹ have the same meanings as defined above.

All the compounds represented by Formulas (2) to (4) have a positive Δε (P type compounds) and are very excellent in a thermal stability and a chemical stability, so that they are useful when preparing a liquid crystal composition for TFT which is required to have a high reliability such as a high voltage-holding rate (a large resistivity).

When preparing a liquid crystal composition for TFT, the concentration of the compounds represented by Formulas (2) to (4) is suitably in a range of 1 to 99.9% by weight, preferably 10 to 97% by weight and more preferably 40 to 95% by weight based on the total weight of the liquid crystal composition. In this case, the compounds represented by Formulas (7) to (9) may further be added for the purpose of controlling the viscosity.

The compounds represented by Formulas (2) to (4) can also be used when preparing a liquid crystal composition for STN or TN. These compounds have a relatively small effect to reduce a threshold voltage of a liquid crystal composition, and in this case, the concentration is preferably 50% by weight or less based on the total weight of the liquid crystal composition.

Among the second B components described above, given below are compounds represented by Formulas (5-1) to (5-40) as preferable examples of the compounds represented by Formula (5) and compounds represented by Formulas (6-1) to (6-3) as preferable examples of the compounds represented by Formula (6), respectively. 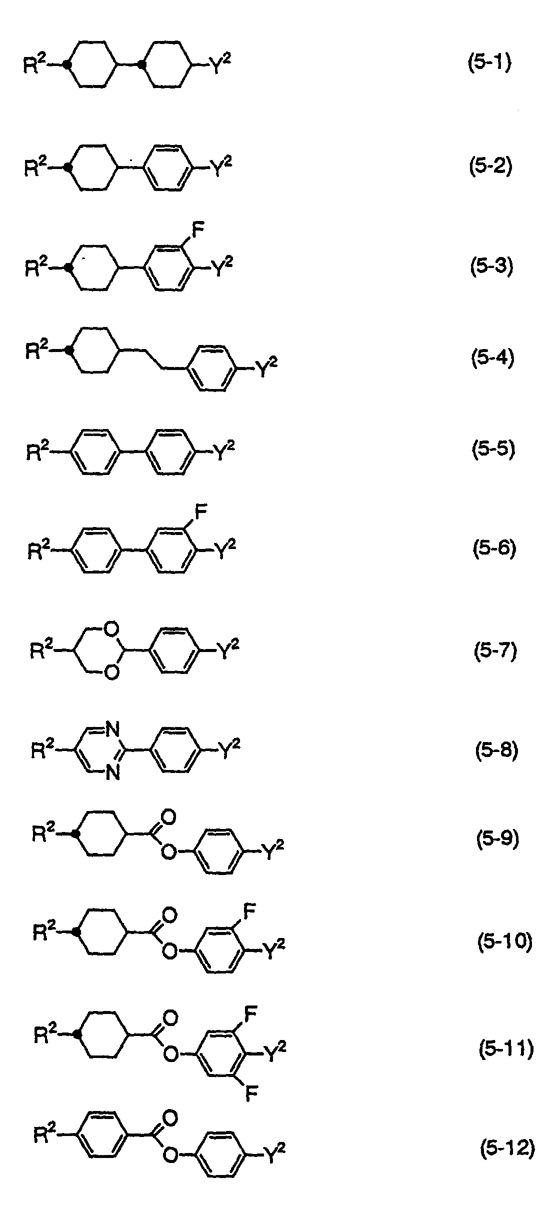 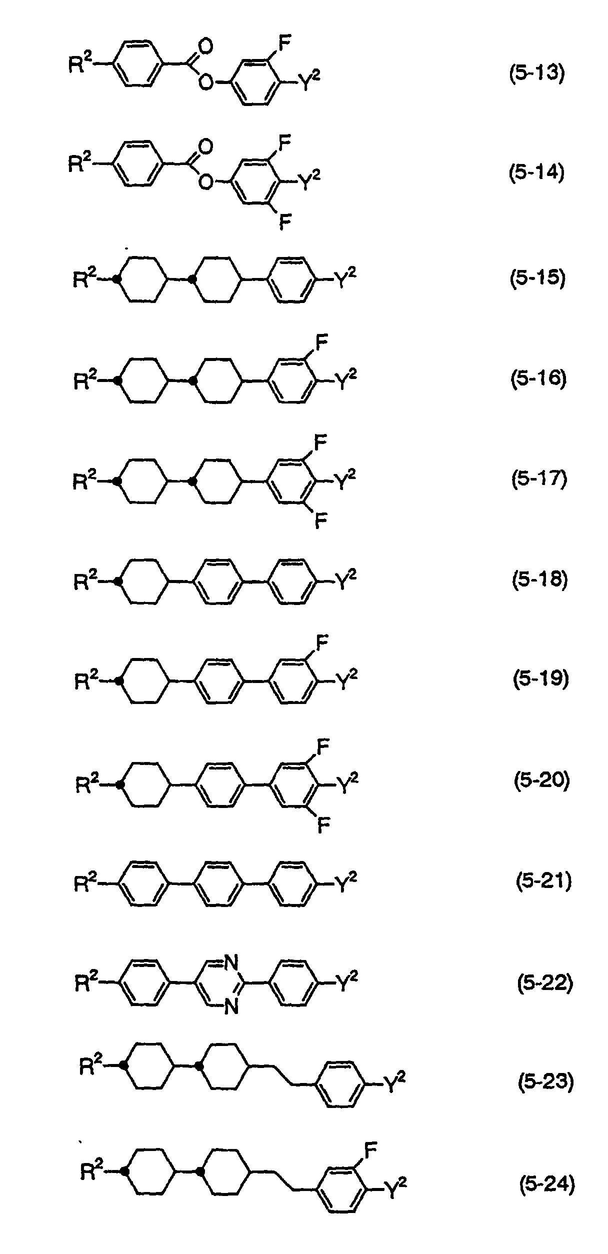 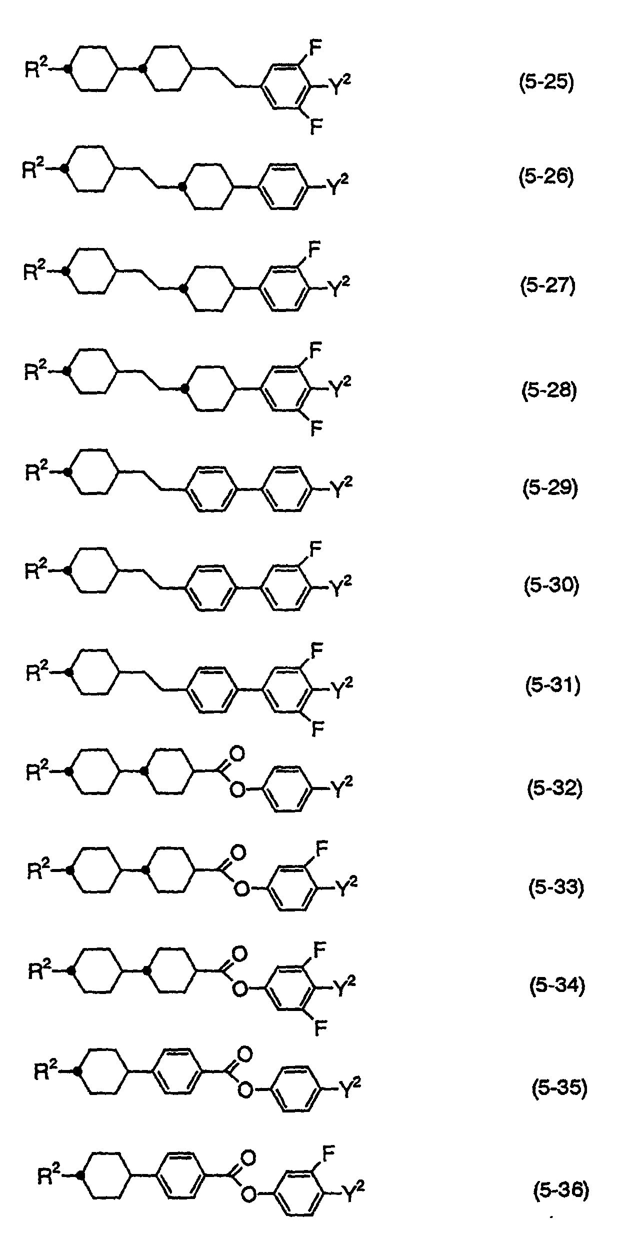 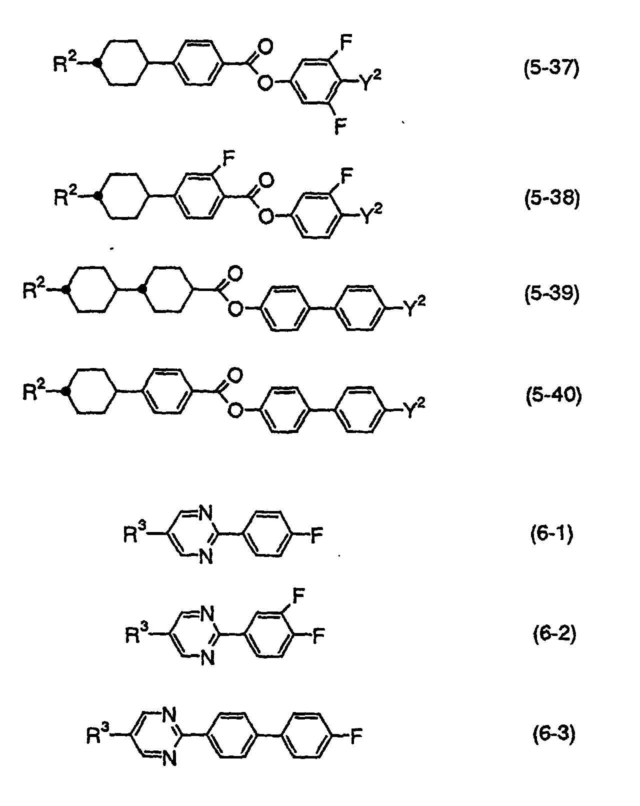 In the above formulas, R², Y² and R³ have the same meanings as defined above.

All these compounds represented by Formulas (5) and (6) have a largely positive Δε and are used particularly for the purpose of reducing a threshold voltage of the liquid crystal composition. Further, they are also used for the purpose of improving a steepness of a transmittance-voltage curve of a liquid crystal composition for STN or TN as well as controlling the Δn and expanding the nematic range such as elevating the clearing point, and they are useful particularly when preparing these liquid crystal compositions.

These compounds reduce a threshold voltage of the liquid crystal composition as the concentration thereof in the liquid crystal composition is increased, but on the other hand, they increase the viscosity. Accordingly, as long as the liquid crystal composition satisfies the required viscosity, the higher concentration is more preferred because it enables the display element to be driven at a lower voltage. When preparing the liquid crystal composition for STN or TN, the concentration of the compound represented by Formula (5) or (6) is suitably in a range of 0.1 to 99.9% by weight, preferably 10 to 97% by weight and more preferably 40 to 95% by weight based on the total weight of the liquid crystal composition.

Among the third components described above, given below are compounds represented by Formulas (7-1) to (7-11) as preferable examples of the compound represented by Formula (7), compounds represented by Formulas (8-1) to (8-18) as preferable examples of the compound represented by Formula (8) and compounds represented by Formulas (9-1) to (9-6) as preferable examples of the compound represented by Formula (9), respectively. 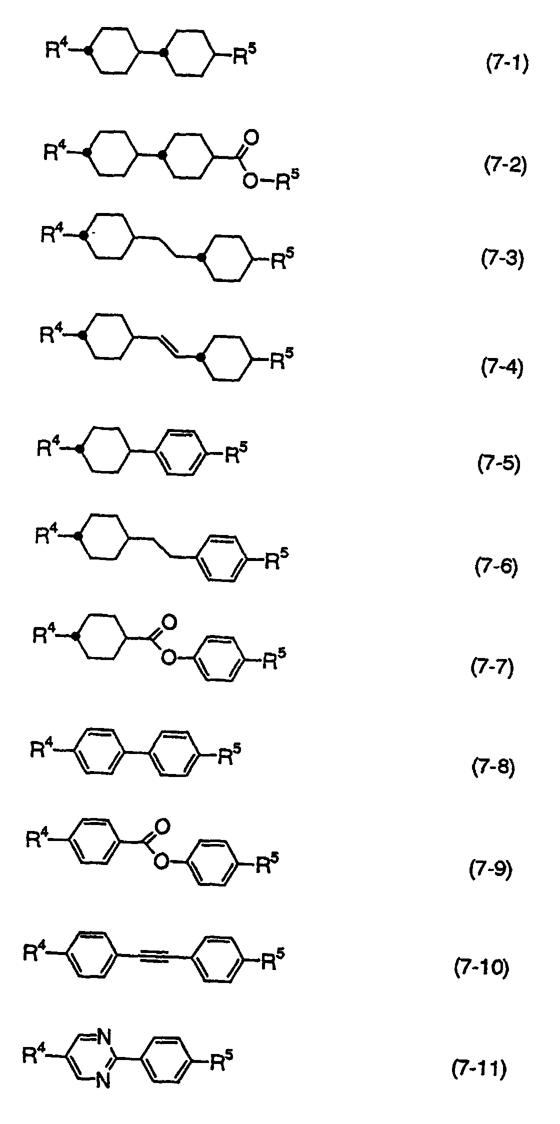 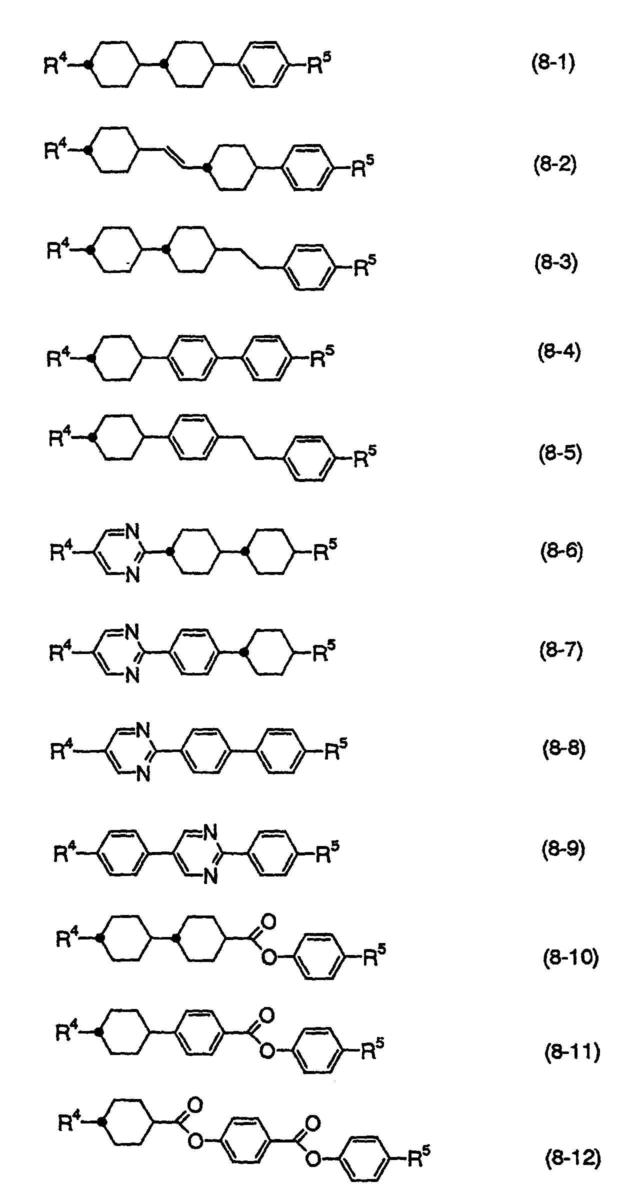 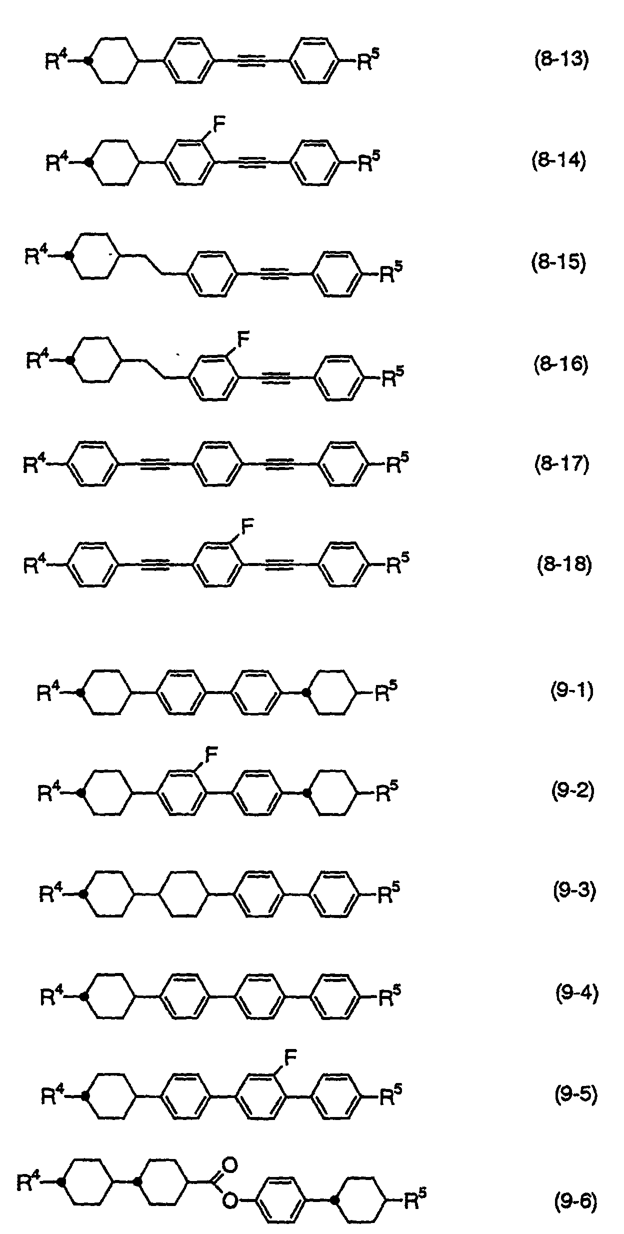 In the above formulas, R⁴ and R⁵ have the same meanings as defined above.

All the compounds represented by Formulas (7) to (9) have a small absolute value of the Δε. The compounds represented by Formula (7) are used primarily for the purpose of controlling the viscosity or the Δn, and the compounds represented by Formulas (8) and (9) are used for the purpose of expanding the nematic range such as elevating the clearing point and controlling the Δn.

These compounds elevate a threshold voltage of the liquid crystal composition as the concentration thereof in the liquid crystal composition is increased, but on the other hand, they decrease the viscosity. Accordingly, the higher concentration is desirable as long as the liquid crystal composition satisfies the required threshold voltage. When preparing the liquid crystal composition for TFT, the concentration of the compounds represented by Formulas (7) to (9) is suitably 40% by weight or less, preferably 35% by weight or less, based on the total weight of the liquid crystal composition. When preparing the liquid crystal composition for STN or TN, the concentration of the above compounds is suitably 70% by weight or less, and preferably 60% by weight or less.

The following compounds can be given as preferable examples of the compounds represented by Formulas (10) to (12). 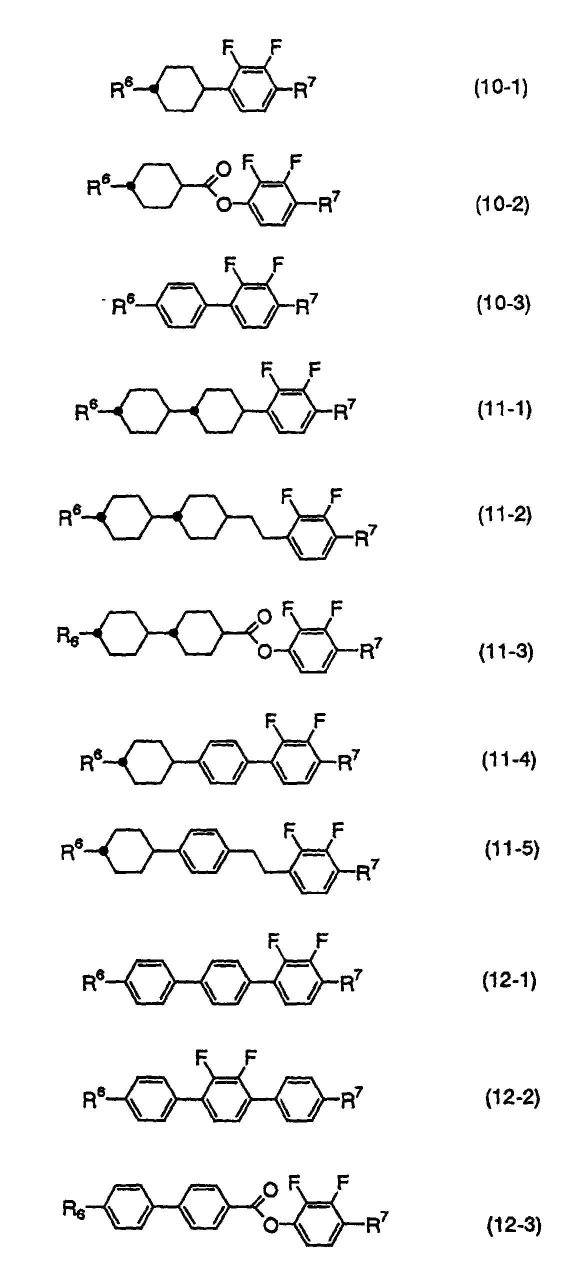 In the above formulas, R⁶ and R⁷ have the same meanings as defined above.

All the compounds represented by Formulas (10) to (12) have a negative Δε (N type compounds). The dicyclic compounds represented by Formula (10) are used primarily for the purpose of controlling the threshold voltage, the viscosity or the refractive anisotropy. The compounds represented by Formula (11) are used for the purpose of expanding the nematic range or controlling the refractive anisotropy. The compounds represented by Formula (12) are used for the purpose of extending the nematic range, reducing the threshold voltage or increasing the refractive anisotropy.

The compounds represented by Formulas (10) to (12) are mainly used for the N type composition. These compounds reduce a threshold voltage of the liquid crystal composition as the concentration thereof in the liquid crystal composition is increased, but they increase the viscosity. Accordingly, the smaller concentration is desirable as long as the liquid crystal composition satisfies the required threshold voltage. However, these compounds have an absolute value of the dielectric anisotropy of 5 or less, and therefore, the concentration of less than 40% by weight may prevent driving at a low voltage.

When preparing the N type liquid crystal composition for TFT, the concentration of the compound represented by Formulas (10) to (12) is a preferably 40% by weight or more, more preferably 50 to 95% by weight, based on the total weight of the liquid crystal composition.

The compounds represented by Formulas (10) to (12) may also be added to the P type liquid crystal composition for the purpose of controlling the elastic constant and a steepness of the voltage-transmittance curve (V-T curve). In this case, the concentration of these compounds is preferably 35% by weight or less.

The compounds represented by Formulas (1) to (12) are not different in characteristics thereof even if the atoms constituting them are substituted with isotopes thereof, and therefore, they may be substituted with the isotopes.

An optically active compound is usually added to the liquid crystal composition of the present invention for the purpose of inducing a spiral structure of the liquid crystal composition to control the required twist angle and thus preventing reverse twist except for a specific case of a liquid crystal composition for an OCB (optically compensated birefringence) mode and the like. Any publicly known optically active compounds used for such a purpose can be used for the liquid crystal composition of the present invention. Optically active compounds represented by the following Formulas (Op-1) to (Op-10) can be given as the preferred compounds.

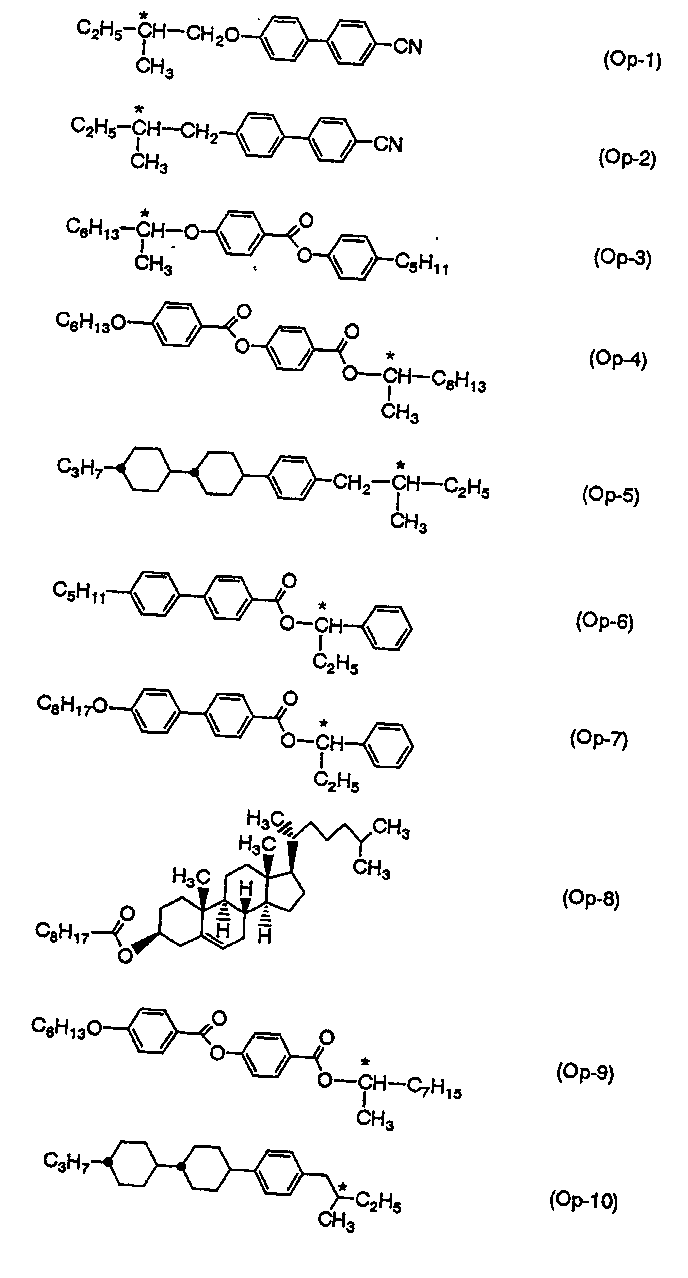

A pitch length of twist in the liquid crystal composition of the present invention is controlled by adding these optically active compounds. The pitch of twist is preferably controlled in a range of 40 to 200 µm in the case of the liquid crystal compositions for TFT and TN, 60 to 20 µm in the case of the liquid crystal composition for STN, and 1.5 to 4 µm in the case of the liquid crystal composition for bistable TN, respectively. Two or more kinds of the optically active compounds may be added for the purpose of controlling a temperature dependency of the pitch.

The liquid crystal composition of the present invention can also be used as a liquid crystal composition for a guest-host (GH) mode by adding a dichromatic pigment of a merocyanine base, a styryl base, an azo base, an azomethine base, an azoxy base, a quinophthalone base, an anthraquinone base, a tetrazine base or the like. It can also be used as a liquid crystal composition for NCAP prepared by putting a nematic liquid crystal into a microcapsule, a polymer dispersion type liquid crystal display element (PDLCD) which is represented by a polymer network liquid crystal display element (PNLCD) prepared by introducing a three-dimensional polymer network into a liquid crystal, electrically controlled birefringence (ECB) and dynamic scattering (DS).

The liquid crystal composition of the present invention is prepared by conventional methods. In general, it is prepared by a method in which various components are dissolved each other at a high temperature.

The following composition examples 1 to 17 can be shown as nematic liquid crystal compositions containing the liquid crystal composition of the present invention thus prepared.

In the respective compounds contained in the composition examples, a left terminal group, a bonding group, a ring structure and a right terminal group are represented by the symbols as defined in the following Table 1. The numbers given to the compounds of the present invention are the same as those in the examples described later, and the concentrations (%) of the compounds mean % by weight unless otherwise described.

The characteristic data in the composition examples are shown by NI (nematic-isotropic liquid transition temperature or clearing point), η (viscosity: measured at 20°C), Δn (refractive anisotropy: measured at 25°C) and Vth (threshold voltage: measured at 25°C).

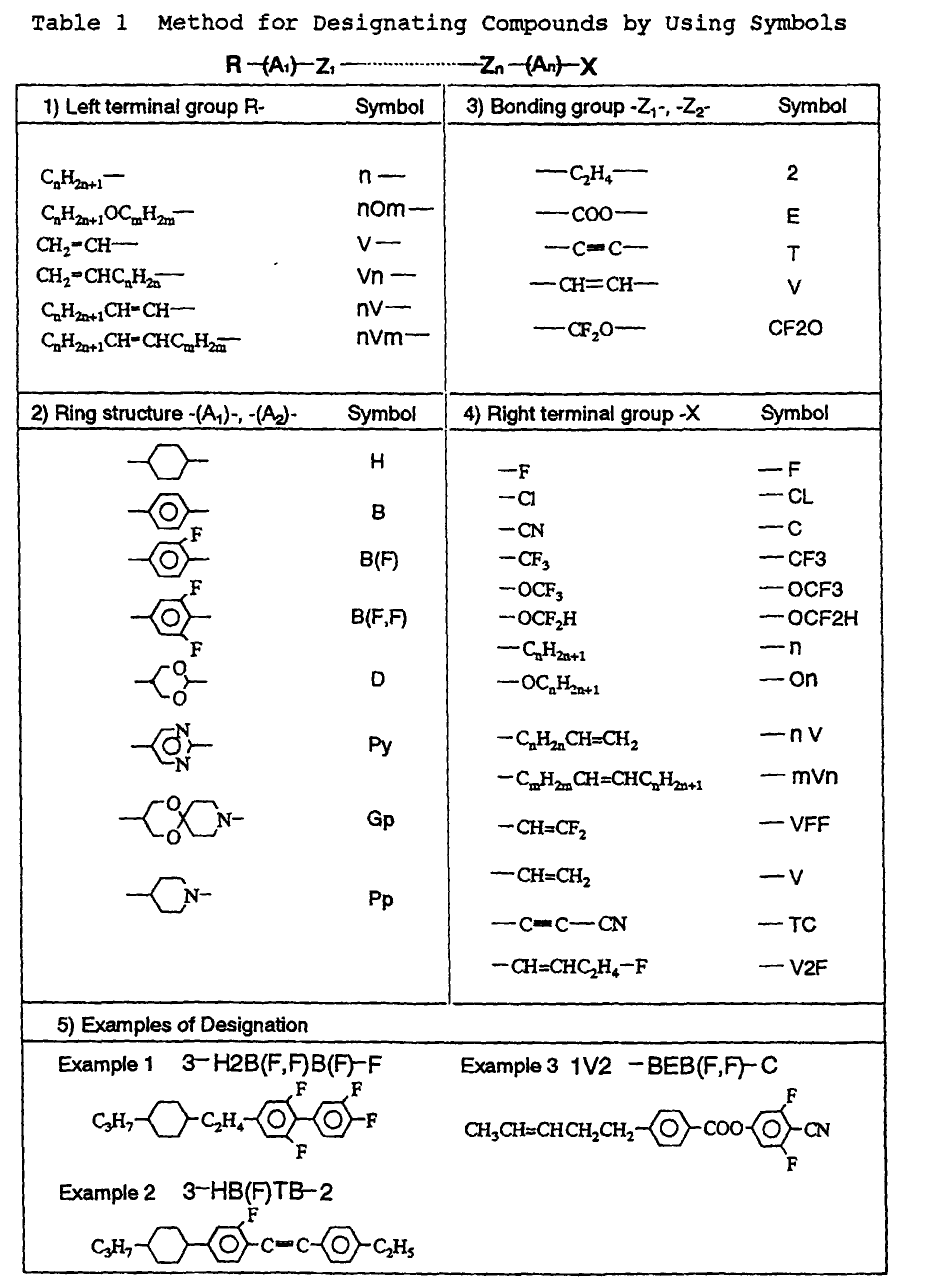

### Composition Example 1

| | |
|---|---|
| 2-GpB-C (No. 10) | 5.0% |
| 3-GpB-C (No. 11) | 5.0% |
| 4-GpB-C (No. 12) | 5.0% |
| 5-GpB-C (No. 13) | 5.0% |
| 1V2-BEB(F,F)-C | 5.0% |
| 3-HB-C | 5.0% |
| 1-BTB-3 | 5.0% |
| 2-BTB-1 | 10.0% |
| 3-HH-4 | 11.0% |
| 3-HHB-1 | 11.0% |
| 3-HHB-3 | 9.0% |
| 3-H2BTB-2 | 4.0% |
| 3-H2BTB-3 | 4.0% |
| 3-H2BTB-4 | 4.0% |
| 3-HB(F)TB-2 | 6.0% |
| 3-HB(F)TB-3 | 6.0% |

This composition had the following characteristics.
NI = 86.0 (°C)
η = 40.0 (mPa·s)
Δn = 0.162
Vth = 2.05 (V)

A composition prepared by adding 0.8 part by weight of the optically active compound of Formula (Op-4) to 100 parts by weight of the composition 1 had a pitch of 11.8 µm at 25°C.

### Composition Example 2

| | |
|---|---|
| 2-GpB(F,F)-F (No. 23) | 4.0% |
| 3-GpB(F,F)-F (No. 24) | 4.0% |
| 4-GpB(F,F)-F (No. 25) | 4.0% |
| 2O1-BEB(F)-C | 5.0% |
| 5O1-BEB(F)-C | 4.0% |
| 1V2-BEB(F,F)-C | 16.0% |
| 3-HB-O2 | 10.0% |
| 3-HH-4 | 3.0% |
| 3-HHB-F | 3.0% |
| 3-HHB-1 | 8.0% |
| 3-HHB-O1 | 4.0% |
| 3-HBEB-F | 4.0% |
| 3-HHEB-F | 7.0% |
| 5-HHEB-F | 7.0% |
| 3-H2BTB-2 | 4.0% |
| 3-H2BTB-3 | 4.0% |
| 3-H2BTB-4 | 4.0% |
| 3-HB(F)TB-2 | 5.0% |

This composition had the following characteristics
NI = 79.3 (°C)
η = 47.0 (mPa·s)
Δn = 0.127
Vth = 1.23 (V)

### Composition Example 3

| | |
|---|---|
| 3-PpBB(F)-F (No. 270) | 5.0% |
| 3-HB-C | 12.0% |
| 3-HB-O2 | 15.0% |
| 2-BTB-1 | 3.0% |
| 3-HHB-1 | 8.0% |
| 3-HHB-F | 4.0% |
| 3-HHB-O1 | 5.0% |
| 3-HHB-3 | 14.0% |
| 3-HHEB-F | 4.0% |
| 5-HHEB-F | 4.0% |
| 2-HHB(F)-F | 7.0% |
| 3-HHB(F)-F | 7.0% |
| 5-HHB(F)-F | 7.0% |
| 3-HHB(F,F)-F | 5.0% |

This composition had the following characteristics.
NI = 104.4 (°C)
η = 18.0 (mPa·s)
Δn = 0.104
Vth = 2.50 (V)

### Composition Example 4

| | |
|---|---|
| 3-GpB-C (No. 11) | 5.0% |
| 3-BEB(F)-C | 8.0% |
| 3-HB-C | 3.0% |
| V-HB-C | 8.0% |
| 1V-HB-C | 8.0% |
| 3-HB-O2 | 3.0% |
| 3-HH-2V | 14.0% |
| 3-HH-2V1 | 7.0% |
| V2-HHB-1 | 15.0% |
| 3-HHB-1 | 5.0% |
| 3-HHEB-F | 7.0% |
| 3-H2BTB-2 | 6.0% |
| 3-H2BTB-3 | 6.0% |
| 3-H2BTB-4 | 5.0% |

This composition had the following characteristics.
NI = 97.7 (°C)
η = 23.4 (mPa·s)
Δn = 0.133
Vth = 2.15 (V)

### Composition Example 5

| | |
|---|---|
| 3-GpB-C (No. 11) | 3.0% |
| 5-BEB(F)-C | 5.0% |
| V-HB-C | 11.0% |
| 5-PyB-C | 6.0% |
| 4-BB-3 | 11.0% |
| 3-HH-2V | 10.0% |
| 5-HH-V | 11.0% |
| V-HHB-1 | 7.0% |
| V2-HHB-1 | 15.0% |
| 3-HHB-1 | 6.0% |
| 1V2-HBB-2 | 10.0% |
| 3-HHEBH-3 | 5.0% |

This composition had the following characteristics.
NI = 87.1 (°C)
η = 19.4 (mPa·s)
Δn = 0.116
Vth = 2.11 (V)

### Composition Example 6

| | |
|---|---|
| 2-GpB-C (No. 10) | 5.0% |
| 3-GpB-C (No. 11) | 5.0% |
| V2-HB-TC | 10.0% |
| 3-HB-TC | 10.0% |
| 5-HB-C | 7.0% |
| 5-BB-C | 3.0% |
| 2-BTB-1 | 10.0% |
| 2-BTB-O1 | 5.0% |
| 3-HH-4 | 5.0% |
| 3-HHB-1 | 10.0% |
| 3-HHB-3 | 11.0% |
| 3-H2BTB-2 | 3.0% |
| 3-H2BTB-3 | 3.0% |
| 3-HB(F)TB-2 | 3.0% |
| 5-BTB(F)TB-3 | 10.0% |

This composition had the following characteristics.
NI = 98.3 (°C)
η = 28.7 (mPa·s)
Δn = 0.205
Vth = 2.02 (V)

### Composition Example 7

| | |
|---|---|
| 3-GpB-C (No. 11) | 5.0% |
| 1V2-BEB(F,F)-C | 6.0% |
| 3-HB-C | 13.0% |
| 2-BTB-1 | 10.0% |
| 5-HH-VFF | 30.0% |
| 1-BHH-VFF | 8.0% |
| 1-BHH-2VFF | 11.0% |
| 3-H2BTB-2 | 5.0% |
| 3-H2BTB-3 | 4.0% |
| 3-H2BTB-4 | 4.0% |
| 3-HHB-1 | 4.0% |

This composition had the following characteristics.
NI = 80.1 (°C)
η = 19.3 (mPa·s)
Δn = 0.130
Vth = 1.99 (V)

### Composition Example 8

| | |
|---|---|
| 2-GpB(F)-F (No. 17) | 4.0% |
| 3-GpB(F)-F (No. 18) | 4.0% |
| 4-GpB(F)-F (No. 19) | 4.0% |
| 5-GpB(F)-F (No. 20) | 5.0% |
| 3-HHB(F)-F | 17.0% |
| 5-HHB(F)-F | 16.0% |
| 2-H2HB(F)-F | 10.0% |
| 3-H2HB(F)-F | 5.0% |
| 5-H2HB(F)-F | 10.0% |
| 2-HBB(F)-F | 6.0% |
| 3-HBB(F)-F | 6.0% |
| 5-HBB(F)-F | 13.0% |

This composition had the following characteristics.
NI = 72.5 (°C)
η = 38.1 (mPa·s)
Δn = 0.080
Vth = 1.97 (V)

A composition prepared by adding 0.3 part by weight of the optically active compound of Formula (Op-9) to 100 parts by weight of the composition 8 had a pitch of 79.0 µm at 25°C.

### Composition Example 9

| | |
|---|---|
| 3-GpB(F,F)-F (No. 24) | 5.0% |
| 7-HB(F,F)-F | 3.0% |
| 3-HB-O2 | 7.0% |
| 2-HHB(F)-F | 10.0% |
| 3-HHB(F)-F | 10.0% |
| 5-HHB(F)-F | 10.0% |
| 2-HBB(F)-F | 9.0% |
| 3-HBB(F)-F | 9.0% |
| 5-HBB(F)-F | 16.0% |
| 2-HBB-F | 4.0% |
| 3-HBB-F | 4.0% |
| 5-HBB-F | 3.0% |
| 5-HBB(F,F)-F | 10.0% |

This composition had the following characteristics.
NI = 78.3 (°C)
η = 28.6 (mPa·s)
Δn = 0.109
Vth = 1.79 (V)

### Composition Example 10

| | |
|---|---|
| 2-GpB(F,F)-OCF3 (No. 27) | 5.0% |
| 3-GpB(F,F)-OCF3 (No. 28) | 5.0% |
| 4-GpB(F,F)-OCF3 (No. 29) | 4.0% |
| 3-HB-CL | 10.0% |
| 5-HB-CL | 4.0% |
| 7-HB-CL | 5.0% |
| 2-HBB(F)-F | 8.0% |
| 3-HBB(F)-F | 8.0% |
| 4-HHB-CL | 8.0% |
| 5-HHB-CL | 8.0% |
| 3-H2HB(F)-CL | 4.0% |
| 3-HBB(F,F)-F | 10.0% |
| 5-H2BB(F,F)-F | 9.0% |
| 3-HB(F)VB-2 | 4.0% |
| 3-HB(F)VB-3 | 4.0% |

This composition had the following characteristics.
NI = 64.7 (°C)
η = 32.0 (mPa·s)
Δn = 0.113
Vth = 1.94 (V)

### Composition Example 11

| | |
|---|---|
| 3-PpBB(F)-F (No. 270) | 4.0% |
| 4-PpBB(F)-F (No. 271) | 4.0% |
| 3-HHB(F,F)-F | 9.0% |
| 3-H2HB(F,F)-F | 8.0% |
| 4-H2HB(F,F)-F | 8.0% |
| 3-HBB(F,F)-F | 21.0% |
| 5-HBB(F,F)-F | 20.0% |
| 3-H2BB(F,F)-F | 10.0% |
| 5-HHBB(F,F)-F | 3.0% |
| 5-HHEBB-F | 2.0% |
| 3-HH2BB(F,F)-F | 3.0% |
| 1O1-HBBH-4 | 4.0% |
| 1O1-HBBH-5 | 4.0% |

This composition had the following characteristics.
NI = 100.1 (°C)
η = 33.4 (mPa·s)
Δn = 0.125
Vth = 1.70 (V)

A composition prepared by adding 0.25 part by weight of the optically active compound of Formula (Op-10) to 100 parts by weight of the composition 11 had a pitch of 64.0 µm at 25°C.

### Composition Example 12

| | |
|---|---|
| 3-PpBB(F,F)-OCF3 (No. 283) | 5.0% |
| 4-PpBB(F,F)-OCF3 (No. 284) | 5.0% |
| 5-HB-F | 12.0% |
| 6-HB-F | 9.0% |
| 7-HB-F | 7.0% |
| 2-HHB-OCF3 | 7.0% |
| 3-HHB-OCF3 | 7.0% |
| 4-HHB-OCF3 | 7.0% |
| 5-HHB-OCF3 | 5.0% |
| 3-HH2B-OCF3 | 4.0% |
| 5-HH2B-OCF3 | 4.0% |
| 3-HHB(F,F)-OCF3 | 5.0% |
| 3-HBB(F)-F | 10.0% |
| 3-HH2B(F)-F | 3.0% |
| 3-HB(F)BH-3 | 3.0% |
| 5-HBBH-3 | 3.0% |
| 3-HHB(F,F)-OCF2H | 4.0% |

This composition had the following characteristics.
NI = 80.3 (°C)
η = 19.0 (mPa·s)
Δn = 0.095
Vth = 2.20 (V)

### Composition Example 13

| | |
|---|---|
| 3-PpBB(F)-F (No. 270) | 5.0% |
| 4-PpBB(F)-F (No. 271) | 5.0% |
| 5-PpBB(F)-F (No. 272) | 5.0% |
| 3-H2HB(F,F)-F | 7.0% |
| 5-H2HB(F,F)-F | 8.0% |
| 3-HHB(F,F)-F | 10.0% |
| 4-HHB(F,F)-F | 5.0% |
| 3-HH2B(F,F)-F | 9.0% |
| 5-HH2B(F,F)-F | 9.0% |
| 3-HBB(F,F)-F | 15.0% |
| 3-HBEB(F,F)-F | 2.0% |
| 4-HBEB(F,F)-F | 2.0% |
| 5-HBEB(F,F)-F | 2.0% |
| 3-HHEB(F,F)-F | 10.0% |
| 4-HHEB(F,F)-F | 3.0% |
| 5-HHEB(F,F)-F | 3.0% |

This composition had the following characteristics.
NI = 90.2 (°C)
η = 28.6 (mPa·s)
Δn = 0.102
Vth = 1.75 (V)

### Composition Example 14

| | |
|---|---|
| 3-PpBB(F)-F (No. 270) | 6.0% |
| 4-PpBB(F)-F (No. 271) | 6.0% |
| 7-HB(F,F)-F | 3.0% |
| 3-H2HB(F,F)-F | 12.0% |
| 4-H2HB(F,F)-F | 10.0% |
| 5-H2HB(F,F)-F | 10.0% |
| 3-HHB(F,F)-F | 5.0% |
| 4-HHB(F,F)-F | 5.0% |
| 3-HH2B(F,F)-F | 15.0% |
| 5-HH2B(F,F)-F | 10.0% |
| 3-HBB(F,F)-F | 12.0% |
| 3-HBCF2OB(F,F)-F | 6.0% |

This composition had the following characteristics.
NI = 75.8 (°C)
η = 23.1 (mPa·s)
Δn = 0.093
Vth = 1.50 (V)

### Composition Example 15

| | |
|---|---|
| 3-PpBB(F)-F (No. 270) | 5.0% |
| 7-HB(F,F)-F | 5.0% |
| 3-H2HB(F,F)-F | 12.0% |
| 4-H2HB(F,F)-F | 10.0% |
| 3-HHB(F,F)-F | 10.0% |
| 4-HHB(F,F)-F | 5.0% |
| 3-HBB(F,F)-F | 5.0% |
| 3-HHEB(F,F)-F | 10.0% |
| 4-HHEB(F,F)-F | 3.0% |
| 5-HHEB(F,F)-F | 3.0% |
| 2-HBEB(F,F)-F | 3.0% |
| 3-HBEB(F,F)-F | 5.0% |
| 5-HBEB(F,F)-F | 3.0% |
| 3-HDB(F,F)-F | 15.0% |
| 3-HHBB(F,F)-F | 6.0% |

This composition had the following characteristics.
NI = 79.1 (°C)
η = 33.5 (mPa·s)
Δn = 0.087
Vth = 1.42 (V)

### Composition Example 16

| | |
|---|---|
| 3-PpBB(F)-F (No. 270) | 5.0% |
| 4-PpBB(F)-F (No. 271) | 5.0% |
| 5-H4HB(F,F)-F | 7.0% |
| 5-H4HB-OCF3 | 15.0% |
| 3-H4HB(F,F)-CF3 | 8.0% |
| 5-H4HB(F,F)-CF3 | 10.0% |
| 3-HB-CL | 6.0% |
| 5-HB-CL | 4.0% |
| 2-H2BB(F)-F | 5.0% |
| 5-HVHB(F,F)-F | 5.0% |
| 3-HHB-OCF3 | 5.0% |
| 3-H2HB-OCF3 | 5.0% |
| V-HHB(F)-F | 5.0% |
| 3-HHB(F)-F | 5.0% |
| 5-HHEB-OCF3 | 2.0% |
| 3-HBEB(F,F)-F | 5.0% |
| 5-HH-V2F | 3.0% |

This composition had the following characteristics.
NI = 71.8 (°C)
η = 23.9 (mPa·s)
Δn = 0.098
Vth = 1.78 (V)

### Composition Example 17

| | |
|---|---|
| 3-PpBB(F)-F (No. 270) | 5.0% |
| 5-HB-CL | 12.0% |
| 3-HH-4 | 7.0% |
| 3-HB-O2 | 20.0% |
| 3-H2HB(F,F)-F | 8.0% |
| 3-HHB(F,F)-F | 8.0% |
| 3-HBB(F,F)-F | 6.0% |
| 2-HHB(F)-F | 5.0% |
| 3-HHB(F)-F | 5.0% |
| 2-H2HB(F)-F | 2.0% |
| 3-H2HB(F)-F | 1.0% |
| 5-H2HB(F)-F | 2.0% |
| 3-HHBB(F,F)-F | 4.0% |
| 3-HBCF2OB-OCF3 | 4.0% |
| 5-HBCF2OB(F,F)-CF3 | 4.0% |
| 3-HHB-1 | 3.0% |
| 3-HHB-O1 | 4.0% |

This composition had the following characteristics.
NI = 71.4 (°C)
η = 15.0 (mPa·s)
Δn = 0.090
Vth = 2.10 (V)

The compound of the present invention represented by Formula (1) can be produced by publicly known ordinary organic synthetic processes as shown below.

### (A) Process for producing the compound of Formula (1) wherein Z is the moiety (I):

As shown in Formula (A-1), a spiro compound (20) can readily be synthesized by subjecting diol (19) and 4-piperidone chloride monohydrate to dehydration-condensation in the presence of an acid catalyst such as toluenesulfonic acid according to a method described in J. Chem. Soc., Perkin Trans. I, 158, (1979). This diol (19) can be synthesized according to descriptions in J. Org. Chem., 32, 113 (1967). This spiro compound (20) is further converted according to (A-2) to (A-4), whereby the compound represented by Formula (1) can easily be synthesized.

As shown in Formula (A-2), in the case of the compound of Formula (1) wherein B³ is a single bond and the ring A³ is cyclohexylene, the spiro compound (20) is reacted with ketone (21) according to a method described in JP58-216157A, and the resulting enamine derivative (22) is hydrogenated to be converted into the compound represented by Formula (1).

The compound of Formula (1), wherein the ring A³ is dioxane-2,5-diyl, piperidine-1,4-diyl or 1,4-silacyclohexylene, can also be synthesized by the same method. This ketone (21) can readily be synthesized according to methods described in ORGANIC FUNCTIONAL GROUP PREPARATIONS, Vol. 1, p. 206, ACADEMIC PRESS, 4th Edition Experimental Chemical Course, Vol. 21, p. 149 (Maruzen) and JP07-70148A.

As shown in Formula (A-3), the compound of Formula (1), wherein B³ is a single bond and the ring A³ is 1,4-phenylene in which 1 to 2 hydrogen atoms may be substituted with fluorine atoms, pyrimidine-2,5-diyl or pyridine-2,5-diyl, can be synthesized by a coupling reaction of the spiro compound (20) with a compound represented by Formula (23) according to a method described in J. Am. Chem. Soc., 118, 7215 (1996), J. Am. Chem. Soc., 118, 7217 (1996), J. Org. Chem., 62, 1264 (1997), J. Org. Chem., 62, 1268 (1997) or Tetrahedron Lett., 38, 6359, 6363, 6367 (1997).

Preferred catalysts used in this reaction are compounds obtained by combining zero valent or divalent palladium such as dibenzylidenepalladium (hereinafter abbreviated as "Pd₂(dba)₃") and palladium acetate with 2,2-bis(diphenylphosphino)-1,1'-binaphthyl (hereinafter abbreviated as "BINAP") or tri-o-tolylphosphine as a ligand, and sodium t-butoxide and cesium carbonate are preferred as bases. Toluene and tetrahydrofuran (hereinafter abbreviated as "THF") are preferably used as solvents.

The compound represented by Formula (23) can readily be synthesized according to methods described in ORGANIC FUNCTIONAL GROUP PREPARATIONS, Vol. 1, p. 148, ACADEMIC PRESS, New Experimental Chemical Course, vol. 14, p. 307 (Maruzen) and J. Am. Chem. Soc., 108, 3033 (1986).

As shown in Formula (A-4), the compound of Formula (1), wherein B³ is a bonding group other than a single bond, can readily be synthesized by reacting the spiro compound (20) with the compound (23) in the presence of a base such as potassium carbonate, sodium carbonate, potassium hydroxide, sodium hydroxide or n-butyl lithium according to a method described in ORGANIC FUNCTIONAL GROUP PREPARATIONS, Vol. 1, p. 378, ACADEMIC PRESS, 4th Edition Experimental Chemical Course, Vol. 21, p. 284 (Maruzen) or Angew. Chem., Int. Ed., 6, 767 (1967). 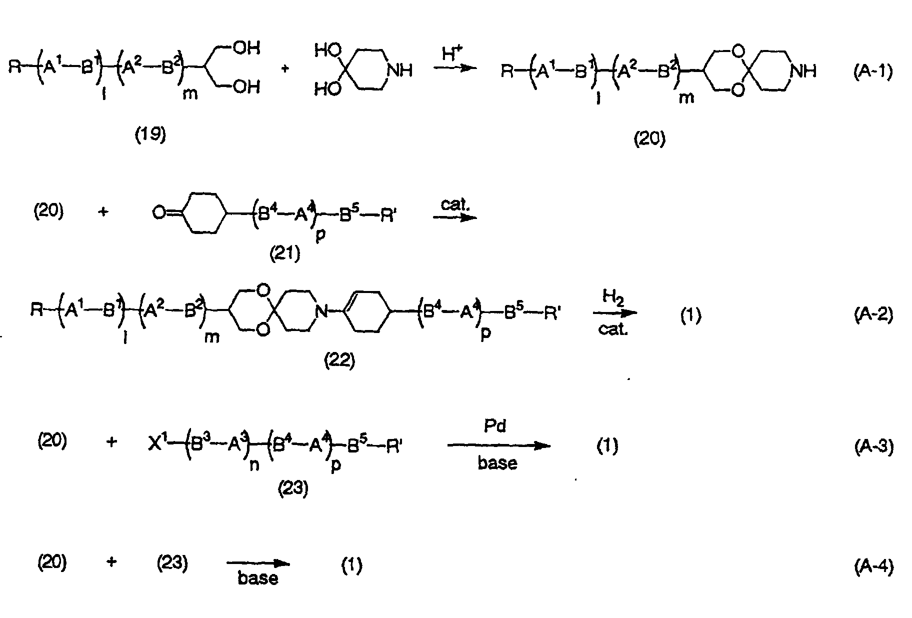 In the formulas, R, R', A¹, A², A³, A⁴, B¹, B², B³, B⁴, B⁵, l, m, n and p have the same meanings as defined above, and X¹ represents a halogen atom or a trifluoromethanesulfonyloxy group.

### (B) Process for producing the compound in Formula (1) wherein Z is the moiety (II):

As shown in Formula (B-1), an enamine derivative (26) can easily be synthesized by subjecting a piperidine derivative (24) and ketone (25) to dehydration-condensation by the same method as in the above (A-2). This enamine derivative (26) can be converted into the compound represented by Formula (1) by hydrogenating it.

As shown in Formula (B-2), the compound represented by Formula (1) can readily be synthesized from the piperidine derivative (24) and the compound (27) by the same method as in the above (A-4).

In this case, the piperidine derivative (24) can be synthesized by a method shown in DE4234627A1, the ketone (25) can be synthesized by the same method as the ketone (21), and the compound (27) can be synthesized by the same method as the compound (23) or a method shown in Tetrahedron Lett., 35, 1051 (1994). 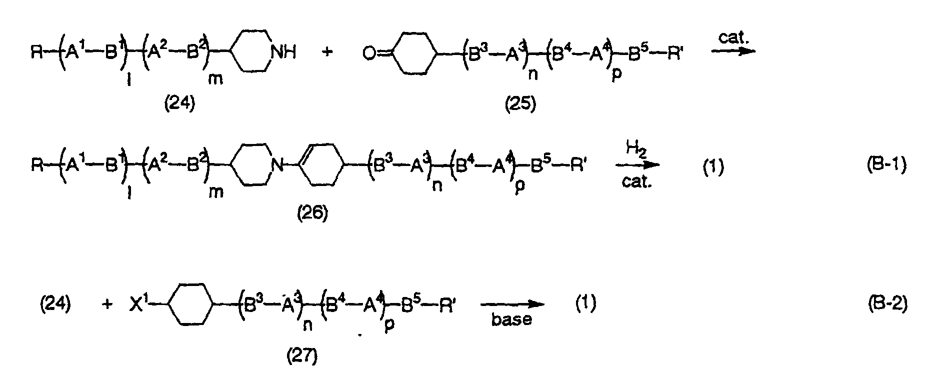 In the formulas, R, R', A¹, A², A³, A⁴, B¹, B², B³, B⁴, B⁵, l, m, n, p and X¹ have the same meanings as defined above.

### (C) Process for producing the compound of Formula (1) wherein Z is the moiety (III), (IV) or (V):

As shown in Formula (C-1), the compound represented by Formula (1) can readily be synthesized by subjecting the piperidine derivative (24) and a compound (28) to a coupling reaction in the presence of a palladium catalyst by the same method as in the above (A-3).

As shown in Formula (C-2), the piperidine derivative (24) is reacted with p-bromoiodobenzene according to a method described in J. Org. Chem., 62, 6066 (1997) to prepare a compound represented by Formula (29), and then the compound (29) is converted in succession according to a method described in DE4234627A1, whereby the compound represented by Formula (1) can be synthesized. 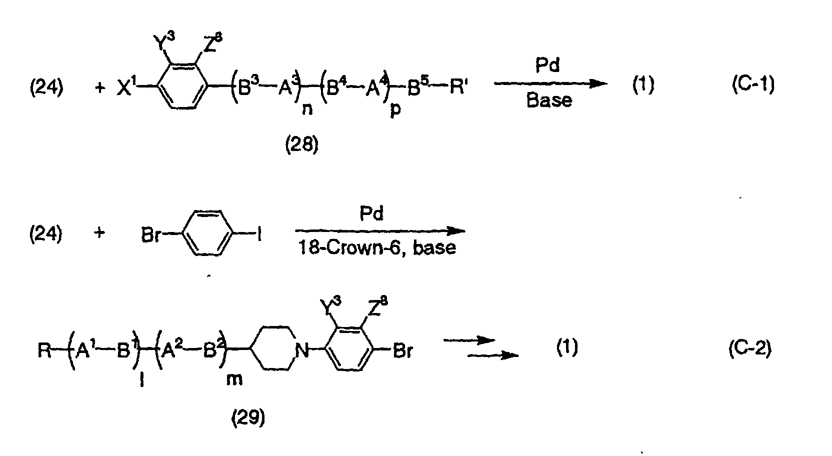 In the formulas, R, R', A¹, A², A³, A⁴, B¹, B², B³, B⁴, B⁵, l, m, n, p and X¹ have the same meanings as defined above, and Y³ and Z⁸ each independently represent a hydrogen atom or a fluorine atom.

In any reactions described above, the compound represented by Formula (1) can be isolated and refined by ordinary operations such as extraction, dehydration, distillation, recrystallization and column chromatography.

### Best Mode for Carrying Out the Invention

The present invention shall be explained in detail with reference to the following examples, but the present invention shall by no means be restricted by these examples.

In the respective examples, M⁺ represents a molecular ion peak in a mass spectrum (MS). C represents a crystal; N represents a nematic phase; S represents a smectic phase; and I represents an isotropic liquid. Those parenthesized in the phase transition point show monotropic liquid crystal phases, and a unit of a phase transition temperature is °C in all cases.

### Example 1

### Synthesis of 1-(4-cyanophenyl)-4-piperidone 2-propylpropyleneacetal (the compound of Formula (1), wherein Z is the moiety (I); R is propyl; l = m = n = 0 and p = 1; the ring A⁴ is 1,4-phenylene; B⁴ and B⁵ are single bonds; and R' is cyano: Compound No. 11)

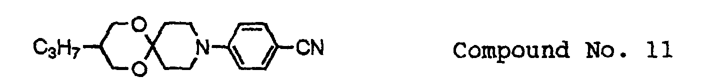

A mixture of 7.9 g (67 mmol) of 2-propylpropylene glycol, 10 g (65 mmol) of 4-piperidone hydrate hydrochloride and 0.5 g (2.6 mmol) of p-toluenesulfonic acid hydrate was heated and refluxed in 150 ml of toluene for 1.5 hour while removing the resulting water. After cooling down, a 2M-sodium hydroxide aqueous solution was added until the pH became 14, and the organic layer was separated. The aqueous layer was extracted with 100 ml of toluene, and the toluene layer was added to the above organic layer, followed by drying on anhydrous sodium carbonate. After removing the sodium carbonate by filtration, the solvent was distilled off under reduced pressure, and the residue was distilled under reduced pressure to obtain 11 g of 4-piperidone 2-propylpropyleneacetal as a colorless liquid. (Yield: 84%).

4.0 g (20 mmol) of 4-piperidone 2-propylpropyleneacetal, 3.1 g (17 mmol) of p-boromocyanobenzene, 39 mg (0.043 mmol) of Pd₂(dba)₃ and 80 mg (0.13 mmol) of BINAP were added to 70 ml of toluene, and then 2.1 g (22 mmol) of sodium t-butoxide was added to react them at 80°C for 4 hours under argon atmosphere. After cooling down, the reaction mixture was washed with 70 ml of water, and the organic layer was separated. The aqueous layer was extracted with 70 ml of toluene, and the toluene layer was added to the above organic layer, followed by drying on anhydrous sodium carbonate. After removing the sodium carbonate by filtration, the solvent was distilled off under reduced pressure, and the residue was refined by means of flash column chromatography (silica gel/toluene : ethyl acetate = 20 : 1) and then recrystallization (toluene) to obtain 1.2 g of the desired product. (Yield: 23%).

The structure of this compound was supported well by the various spectral data thereof and those of the compounds at the respective stages in the reaction.
Phase transition point: C·96.3·I
MS m/e = 300 (M⁺)
¹H-NMR (90 MHz) δ (ppm): 7.47, 6.84 (d, 4H, J=9.0 Hz), 3.3-4.0 (m, 8H), 1.8-2.2 (m, 5H), 0.8-1.5 (m, 7H)

### Example 2

### Synthesis of 1-(3,4-difluorophenyl)-4-piperidone 2-propylpropyleneacetal (the compound of Formula (1), wherein Z is the moiety (I); R is propyl; l = m = n = 0 and p = 1; B⁴ is a single bond; the ring A⁴ is 3-fluoro-1,4-phenylene; B⁵ is a single bond; and R' is a fluorine atom: Compound No. 18)

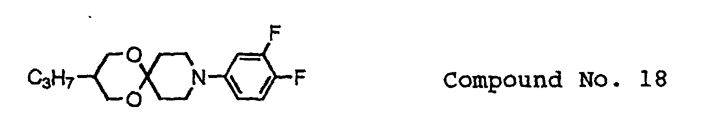

5.0 g (25 mmol) of 4-piperidone 2-propylpropyleneacetal, 4.1 g (21 mmol) of 3,4-difluoroboromobenzene, 49 mg (0.054 mmol) of Pd₂(dba)₃ and 97 mg (0.16 mmol) of BINAP were added to 70 ml of toluene, and then 2.3 g (24 mmol) of sodium t-butoxide was added to react them at 80°C for 3 hours under argon atmosphere. After cooling down, the reaction mixture was washed with 70 ml of water, and the organic layer was separated. The aqueous layer was extracted with 70 ml of toluene, and the toluene layer was added to the above organic layer, followed by drying on anhydrous sodium carbonate. After removing the sodium carbonate by filtration, the solvent was distilled off under reduced pressure, and the residue was refined by means of flash column chromatography (silica gel/toluene : ethyl acetate = 50 : 1) and then recrystallization (heptane) to obtain 2.3 g of the desired product. (Yield: 35%).

The structure of this compound was supported well by the various spectral data thereof.
Phase transition point: C·34.2·I
MS m/e = 311 (M⁺)
¹H-NMR (90 MHz) δ (ppm): 6.4-7.2 (m, 3H), 3.4-4.0 (m, 4H), 3.0-3.4 (m, 4H), 1.8-2.2 (m, 5H), 1.1-1.5 (m, 3H), 0.91 (t, 3H, J=6.5 Hz)
¹⁹F-NMR (60 MHz) δ (ppm): -137.3 (1F), -150.6 (1F)

### Example 3

### Synthesis of 1-(3,4,5-trifluorophenyl)-4-piperidone 2-propylpropyleneacetal (the compound of Formula (1), wherein Z is the moiety (I); R is propyl; l = m = n = 0 and p = 1; B⁴ is a single bond; the ring A⁴ is 3,5-difluoro-1,4-phenylene; B⁵ is a single bond; and R' is a fluorine atom: Compound No. 24)

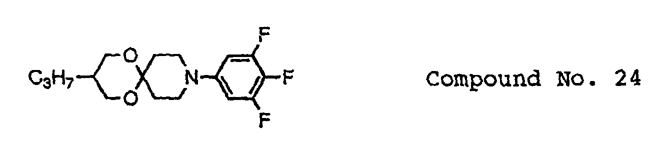

5.0 g (25 mmol) of 4-piperidone 2-propylpropyleneacetal, 4.5 g (21 mmol) of 3,4,5-trifluoroboromobenzene, 49 mg (0.054 mmol) of Pd₂(dba)₃ and 97 mg (0.16 mmol) of BINAP were added to 80 ml of toluene, and then 2.3 g (24 mmol) of sodium t-butoxide was added to react them at 80°C for 2 hours under argon atmosphere. After cooling down, the reaction mixture was washed with 70 ml of water, and the organic layer was separated. The aqueous layer was extracted with 70 ml of toluene, and the toluene layer was added to the above organic layer, followed by drying on anhydrous sodium carbonate. After removing the sodium carbonate by filtration, the solvent was distilled off under reduced pressure, and the residue was refined by means of flash column chromatography (silica gel/toluene : ethyl acetate = 50 : 1) and then recrystallization (heptane) to obtain 2.5 g of the desired product. (Yield: 36%).

The structure of this compound was supported well by the various spectral data thereof.
Phase transition point: C·53.8·I
MS m/e = 329 (M⁺)
¹H-NMR (90 MHz) δ (ppm): 6.3-6.7 (m, 2H), 3.4-4.0 (m, 4H), 3.0-3.4 (m, 4H), 1.8-2.3 (m, 5H), 1.1-1.5 (m, 3H), 0.91 (t, 3H, J=6.5 Hz)
¹⁹F-NMR (60 MHz) δ (ppm): -135.2 (2F), -169.3 (1F)

### Example 4

### Synthesis of 1-(4-trifluoromethyl-3,5-trifluorophenyl)-4-piperidone 2-propylpropyleneacetal (the compound of Formula (1), wherein Z is the moiety (I); R is propyl; l = m = n = 0 and p = 1; B⁴ is a single bond; the ring A⁴ is 3,5-difluoro-1,4-phenylene; B⁵ is a single bond; and R' is trifluoromethoxy: Compound No. 28)

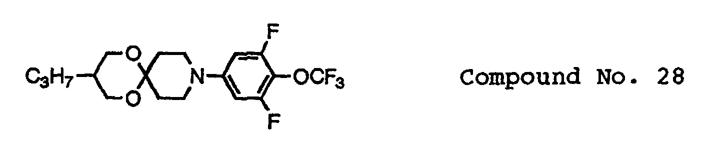

5.0 g (25 mmol) of 4-piperidone 2-propylpropyleneacetal, 5.9 g (21 mmol) of 4-trifluoromethyl-3,5-trifluoroboromobenzene, 49 mg (0.054 mmol) of Pd₂(dba)₃ and 97 mg (0.16 mmol) of BINAP were added to 80 ml of toluene, and then 2.8 g (29 mmol) of sodium t-butoxide was added to react them at 80°C for 3 hours under argon atmosphere. After cooling down, the reaction mixture was washed with 70 ml of water, and the organic layer was separated. The aqueous layer was extracted with 70 ml of toluene, and the toluene layer was added to the above organic layer, followed by drying on anhydrous sodium carbonate. After removing the sodium carbonate by filtration, the solvent was distilled off under reduced pressure, and the residue was refined by means of flash column chromatography (silica gel/toluene: ethyl acetate = 50 : 1) and then recrystallization (heptane) to obtain 3.8 g of the desired product. (Yield: 45%).

The structure of this compound was supported well by the various spectral data thereof.
Phase transition point: C·47.1·I
MS m/e = 395 (M⁺).
¹H-NMR (90 MHz) δ (ppm): 6.43 (dt, 2H, J=15.0, 2.0 Hz), 3.1-4.0 (m, 8H), 1.8-2.2 (m, 5H), 1.1-1.5 (m, 3H), 0.91 (t, 3H, J=7.1 Hz)
¹⁹F-NMR (60 MHz) δ (ppm): -60.8 (3F), -125.8 (2F)

### Example 5

### Synthesis of 4-(3,4-difluorophenyl)-1-(4-propylpiperidyl)benzene (the compound of Formula (1), wherein Z is the moiety (III); R is propyl; l = m = n = 0 and p = 1; the ring A⁴ is 3-fluoro-1,4-phenylene; B⁴ and B⁵ are single bonds; and R' is a fluorine atom: Compound No. 270)

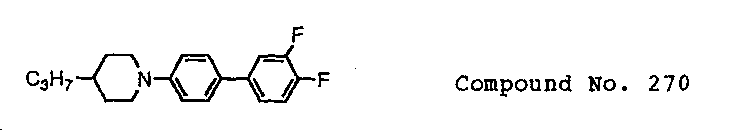

22 g (0.18 mol) of 4-propylpyridine and 0.80 g of platinum oxide were added to 150 ml of acetic acid, and the mixture was stirred at room temperature overnight under a hydrogen pressure of 8 kg/cm². After distilling the solvent off under reduced pressure, 60 ml of a 4M-sodium hydroxide aqueous solution was added, and the solution was extracted with 150 ml of toluene. The aqueous layer was further extracted twice with each 100 ml of toluene, and the organic layers were put together and dried on anhydrous sodium carbonate. After removing the sodium carbonate by filtration, the solvent was distilled off. The residue was distilled under reduced pressure (64°C/21.5 mmHg) to obtain 15 g of 4-propylpiperidine. (Yield: 65%).

7.5 g (59 mmol) of 4-propylpiperidine, 13.8 g (48.8 mmol) of 4-boromoiodobenzene, 120 mg (0.13 mmol) of Pd₂(dba)₃, 230 mg (0.37 mmol) of BINAP and 13 g (49 mmol) of 18-crown-6 were added to 70 ml of THF, and then 6.6 g (69 mmol) of sodium t-butoxide to react them at room temperature for 19 hours under argon atmosphere. After filtering the reaction solution, 100 ml of water was added, and the solution was extracted twice with each 100 ml of toluene. The organic layers were put together and dried on anhydrous sodium carbonate. After removing the sodium carbonate by filtration, the solvent was distilled off under reduced pressure. The residue was refined by means of flash column chromatography (silica gel/toluene : ethyl acetate = 20 : 1 → 2 : 1) to obtain 10.4 g of 4-(4-propylpiperidyl)bromobenzene. (Yield: 76%).

4.0 g (14 mmol) of the 4-(4-propylpiperidyl)bromobenzene, 4.5 g (28 mmol) of 3,4-difluorophenylboric acid, 1.0 g of 5% palladium activated carbon and 12 g (0.11 mol) of sodium carbonate were added to 120 ml of a mixed solvent of toluene : alcohol : water (1 : 1 : 1), and the mixture was heated and refluxed for 9 hours. Then, 4.5 g (28 mmol) of boric acid and 12 g (0.11 mol) of sodium carbonate were added to the reaction mixture, which was further heated and refluxed for 5 hours. After cooling down, 100 ml of toluene and 100 ml of water were added to the reaction mixture, and the organic layer was separated. The aqueous layer was extracted with 100 ml of toluene, and the toluene layer was added to the above organic layer, followed by drying on anhydrous sodium carbonate. After removing the sodium carbonate by filtration, the solvent was distilled off under reduced pressure, and the residue was refined by means of flash column chromatography (silica gel/heptane : toluene = 5 : 1 → 1 : 1) and then recrystallization (heptane) to obtain 1.5 g of the desired product as a colorless crystal. (Yield: 33%).

The structure of this compound was supported well by the various spectral data thereof and those of the compounds at the respective stages in the reaction.
Phase transition point: C·112.9(·SA·106.0)·N·117.5·I
MS m/e = 315 (M⁺)
¹H-NMR (90 MHz) δ (ppm): 6.9-7.5 (m, 7H), 3.5-3.9 (m, 2H), 2.5-3.0 (m, 2H), 1.1-2.0 (m, 9H), 0.92 (t, 3H, J=5.9 Hz)
¹⁹F-NMR (60 MHz) δ (ppm): -138.4 (1F), -142.5 (1F)

### Example 6

### Synthesis of 4-(4-trifluoromethyl-3,5-difluorophenyl)-1-(4-propylpiperidyl)benzene (the compound of Formula (1), wherein Z is the moiety (III); R is propyl; l = m = n = 0 and p = 1; B⁴ is a single bond; the ring A⁴ is 3,5-difluoro-1,4-phenylene; B⁵ is a single bond; and R' is trifluoromethoxy: Compound No. 283)

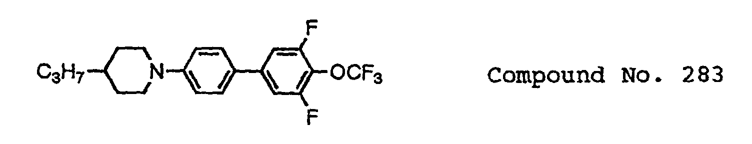

3.0 g (11 mmol) of the 4-(4-propylpiperidyl)bromobenzene obtained in Example 5, 5.1 g (21 mmol) of 4-trifluoromethyl-3,5-difluorophenylboric acid, 1.0 g of 5% palladium activated carbon and 4.5 g (42 mmol) of sodium carbonate were added to 90 ml of a mixed solvent of toluene : alcohol : water (1 : 1 : 1), and the mixture was heated and refluxed for 21 hours.

After cooling down, 100 ml of toluene and 100 ml of water were added to the reaction mixture, and the organic layer was separated. The aqueous layer was extracted with 100 ml of toluene, and the toluene layer was added to the above organic layer, followed by drying on anhydrous sodium carbonate. After removing the sodium carbonate by filtration, the solvent was distilled off under reduced pressure, and the residue was refined by means of flash column chromatography (silica gel/heptane : toluene = 3 : 1) and then recrystallization (heptane) to obtain 2.0 g of the desired product as a colorless crystal. (Yield: 47%).

The structure of this compound was supported well by the various spectral data thereof.
Phase transition point: C·107.8·I
MS m/e = 399 (M⁺)
¹H-NMR (90 MHz) δ (ppm): 6.8-7.5 (m, 6H), 3.6-4.0 (m, 2H), 2.5-3.0 (m, 2H), 1.1-2.0 (m, 9H), 0.92 (t, 3H, J=6.2 Hz)
¹⁹F-NMR (60 MHz) δ (ppm): -60.3 (3F), -125.7 (2F)

### Example 7

### Synthesis of 1-(4-propylpiperidyl)-4-(trans-4-propylcyclohexyl)-2,3-difluorobenzene (the compound of Formula (1), wherein Z is the moiety (V); R and R' are propyl; l = m = n = 0 and p = 1; both B⁴ and B⁵ are single bonds; and the ring A⁴ is trans-1,4-cyclohexylene: Compound No. 391)

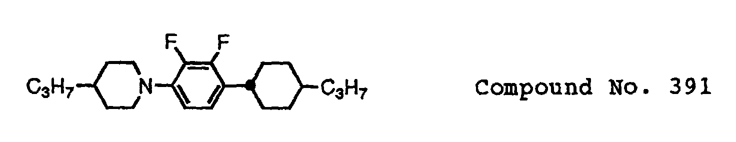

A mixture of 5.6 g (18 mmol) of 1-(4-propylcyclohexyl)-4-bromo-2,3-difluorobenzene synthesized by the same method as described in JP02-004723A, 2.9 g (23 mmol) of 4-propylpiperidine, 40 mg (0.043 mmol) of Pd₂(dba)₃ and 83 mg (0.13 mmol) of BINAP was dissolved in 80 ml of toluene, and then 1.9 g (20 mmol) of sodium t-butoxide was added to react them at 80°C for 3 hours under argon atmosphere.

After cooling down, the reaction mixture was washed with 70 ml of water, and the organic layer was separated. The aqueous layer was extracted with 70 ml of toluene, and the toluene layer was added to the above organic layer, followed by drying on anhydrous sodium carbonate. After removing the sodium carbonate by filtration, the solvent was distilled off under reduced pressure, and the residue was refined by means of flash column chromatography (silica gel/heptane → toluene) and then recrystallization (ethanol) to obtain 1.0 g of the desired product. (Yield: 15%).
Phase transition point: C·81.8·N·125.0·I
MS m/e = 363 (M⁺)
¹H-NMR (90 MHz) δ (ppm): 6.5-7.0 (m, 2H), 3.2-3.6 (m, 2H), 2.4-3.0 (m, 2H), 0.6-2.0 (m, 29H)
¹⁹F-NMR (60 MHz) δ (ppm): -145.1 (1F), -149.5 (1F)

### Example 8

### Synthesis of 1-(4-propylpiperidyl)-4-butoxy-2,3-difluorobenzene (the compound of Formula (1), wherein Z is the moiety (V); R is propyl, and R' is butoxy; l = m = n = p = 0; B⁵ is a single bond: Compound No. 381)

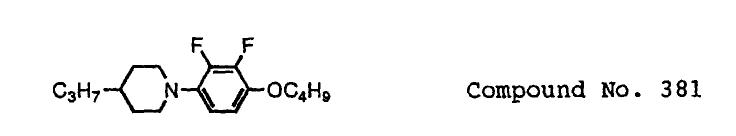

A mixture of 4.0 g (15 mmol) of 1-butoxy-4-bromo-2,3-difluorobenzene synthesized by the same method as described in JP02-004723A, 2.5 g (20 mmol) of 4-propylpiperidine, 35 mg (0.038 mmol) of Pd₂(dba)₃ and 70 mg (0.11 mmol) of BINAP was dissolved in 60 ml of toluene, and then 2.0 g (21 mmol) of sodium t-butoxide was added to react them at 80°C for 4 hours under argon atmosphere.

After cooling down, the reaction mixture was washed with 70 ml of water, and the organic layer was separated. The aqueous layer was extracted with 70 ml of toluene, and the toluene layer was added to the above organic layer, followed by drying on anhydrous sodium carbonate. After removing the sodium carbonate by filtration, the solvent was distilled off under reduced pressure, and the residue was refined by means of flash column chromatography (silica gel/heptane: toluene = 1 : 1) and then recrystallization (ethanol) to obtain 1.0 g of the desired product. (Yield: 21%).
Phase transition point: C·34.4(·N·9.3)·I
MS m/e = 311 (M⁺)
¹H-NMR (90 MHz) δ (ppm): 6.5-7.0 (m, 2H), 3.98 (t, 2H, J=6.4 HZ), 3.2-3.6 (m, 2H), 2.4-3.0 (m, 2H), 0.6-2.0 (m, 19H)
¹⁹F-NMR (60 MHz) δ (ppm): -146.9 (1F), -158.4 (1F)

### Example 9

### Synthesis of 4-(4-(4-propylcyclohexyl)-1-piperidyl)toluene (the compound of Formula (1), wherein Z is the moiety (III); R is propyl, and R' is methyl; l = 1, m = n = p = 0; B¹ and B⁵ are single bonds; and A¹ is trnas-1,4-cyclohexylene: Compound No. 249-1)

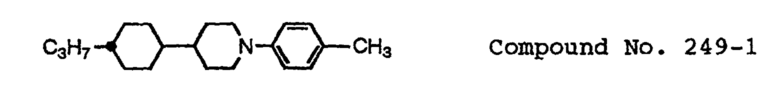

50 g (440 mmol) of 3-chloropyridine was subjected to lithiation using 480 mmol of LDA (lithium diisopropylamide) by the same method as described in Heterocycles, 35, 151 (1993), and then it was reacted with 62 g (440 mmol) of 4-propylcyclohexanone to obtain a crude product of 1-((2-chloro)-4-pyridyl)-4-propylcyclohexanol. 22 ml of conc. sulfuric acid was added thereto, and the solution was put into 1.3 liter of toluene and refluxed while removing the resulting water. After cooling down, 400 ml of a 2M sodium hydroxide aqueous solution was added thereto. After separating the organic layer, the aqueous layer was further extracted with 400 ml of toluene, and the organic layers were put together and washed with 400 ml of purified water. The organic layer was dried on anhydrous magnesium sulfate and then filtered, and the solvent was distilled off under reduced pressure. The residue was refined by means of column chromatography (toluene: ethyl acetate = 10 : 1) to obtain 61 g of 3-chloro-4-(4-propyl)-1-cyclohexenyl)pyridine. (Yield: 58%).

60 g (250 mmol) of this compound was hydrogenated in 600 ml of ethanol in the presence of 27 g (250 mmol) of sodium carbonate using 6.0 g of 5% Pd/C as a catalyst. After filtering the reaction mixture, the solvent was distilled off under reduced pressure, and the residue was extracted in the same manner as described above in a toluene-water system to obtain a crude product of 4-(4-propyl)cyclohexylpyridine. This was refined by distilling under reduced pressure (134°C/3.8 mmHg).

12 g (59 mmol) of this compound was subjected to hydrogenation reaction (hydrogen pressure: 8 kg/cm²) in 200 ml of acetic acid using 300 mg of platinum oxide as a catalyst. The catalyst was filtered off, and the solvent was distilled off under reduced pressure. Then, 200 ml of a 10M-sodium hydroxide aqueous solution was added, and the solution was extracted twice with each 200 ml of toluene. The organic layer was dried on anhydrous sodium carbonate and then filtered, and the solvent was distilled off under reduced pressure to obtain 4-(4-propyl)cyclohexylpiperidine. This compound was used for the subsequent reaction as it was without purifying.

3.3 g (34 mmol) of sodium t-butoxide was added to a mixture of 5.0 g (24 mmol) of this piperidine derivative, 4.1 g (24 mmol) of 4-bromotoluene, 66 mg (0.072 mmol) of Pd₂(dba)₃ and 134 mg (0.21 mmol) of BINAP to react them in 40 ml of toluene at 80°C for 3 hours under helium atmosphere. After cooling down, the reaction solution was filtered, and 60 ml of water was added, followed by extracting with 60 ml of toluene. The organic layer was dried on anhydrous sodium sulfate and then filtered, and the solvent was distilled off under reduced pressure. The residue was refined by means of column chromatography (heptane : toluene = 1 : 1 → toluene) and recrystallization (heptane) to obtain 0.33 g of 4-(4-(4-propylcyclohexyl)-1-piperidyl)toluene as a colorless crystal. (Yield: 4.6%).
Phase transition point: C·91.0·SB·125.4·SA·127.9·N·161.6·I
MS m/e = 299 (M⁺)
¹H-NMR (500 MHz) δ (ppm): 7.05 (d, 2H, J=8.50 Hz), 6.86 (d, 2H, 8.60 Hz), 3.61-3.63 (m, 2H), 2.55-2.57 (m, 2H), 2.26 (s, 3H), 0.85-1.78 (m, 22H)

### Example 10

### Synthesis of 4-(4-(4-propylphenyl)-1-piperidyl)-1-cyanobenzene (the compound of Formula (1), wherein Z is the moiety (III); R is propyl, and R' is cyano; l = 1, m = n = p = 0; B¹ and B⁵ are single bonds; and A¹ is 1,4-phenylene: Compound No. 254)

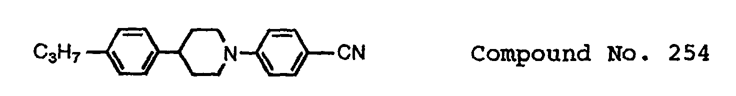

4-(4-propylphenyl)-1-piperidine was synthesized according to a method described in J. Am. Chem. Soc., 106, 3270 (1984). Specifically, a Grignard reagent prepared from 16 g (80 mmol) of 4-propylbromobenzene and 2.1 g (86 mmol) of magnesium using 80 ml of THF as a solvent according to a conventional method was added to 200 ml of a THF solution of 9.7 ml (120 mmol) of pyridine and 760 mg (4.0 mmol) of copper iodide at -20°C or lower, and the solution was stirred at -20°C for 15 minutes and at room temperature for another 15 minutes. 300 ml of a saturated ammonium chloride aqueous solution was added to the reaction mixture, and then the organic layer was separated. The aqueous layer was extracted with 300 ml of toluene, and then the organic layers were put together and washed with 300 ml of a saturated sodium chloride. The organic layer was dried on anhydrous magnesium sulfate and then filtered, and the solvent was distilled off under reduced pressure. The resulting residue was dissolved in 200 ml of ethanol, and 3.2 g of 5% Pd/C was added to carry out hydrogenation (hydrogen pressure: 8 kg/cm²) at room temperature for 2 days. After finishing the reaction, the catalyst was filtered, and the solvent was distilled off under reduced pressure. 100 ml of a 25% hydrogen bromide/acetic acid solution was added to the resulting residue to react them at room temperature for 24 hours. After the solvent was distilled off from the resulting reaction solution under reduced pressure, 500 ml of a 2M-sodium hydroxide aqueous solution and 300 ml of toluene were added to the residue, and the solution was stirred. The organic layer was separated, and then the aqueous layer was extracted with 200 ml of toluene. The organic layer was dried on anhydrous sodium carbonate, and then the solvent was distilled off under reduced pressure. The residue was refined by distilling under reduced pressure (131°C/2.3 mmHg) to obtain 11 g of the desired 4-(4-propylphenyl)-1-piperidine. (Yield: 67%).

1.2 g (12 mmol) of sodium t-butoxide was added to a mixture of 2.0 g (9.8 mmol) of this piperidine derivative, 1.8 g (9.9 mmol) of 4-cyanobromobenzene, 23 mg (0.025 mmol) of Pd₂(dba)₃ and 134 mg (0.080 mmol) of BINAP to react them in 25 ml of toluene at 80°C for 3 hours under helium atmosphere. After cooling down, the reaction solution was treated in the same manner as in the above example, and the residue was refined by means of column chromatography (toluene) and recrystallization (ethanol) to obtain 1.8 g of 4-(4-(4-propylphenyl)-1-piperidyl)-1-cyanobenzene as a colorless crystal. (Yield: 60%).
Phase transition point: C·119.1·I
MS m/e = 304 (M⁺)
¹H-NMR (500 MHz) δ (ppm): 7.47-7.51 (m, 2H), 7.13 (s, 4H), 6.89-6.91 (m, 2H), 3.96-3.99 (m, 2H), 2.94-3.00 (m, 2H), 2.68-2.74 (m, 1H), 2.54-2.58 (m, 2H), 1.57-1.97 (m, 6H), 0.94 (t, 3H, J=7.35 Hz).

### Example 11

### Synthesis of 3,5-difluoro-4-cyanophenoxy 4-(4-propyl-1-piperidyl)benzoate (the compound of Formula (1), wherein Z is the moiety (III); R is propyl, and R' is cyano; l = m = n = 0 and p = 1; B⁴ is -COO-; A⁴ is 1,4-phenylene which is substituted with fluorine atoms at the 3- and 5-positions; and B⁵ is a single bond: Compound No. 297)

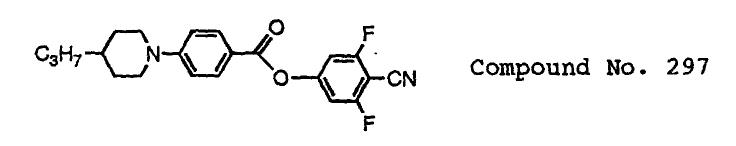

9.4 ml of n-BuLi dissolved in 60 ml of THF was added to 3.3 g (12 mmol) of 4-(4-propylpiperidyl)bromobenzene obtained in Example 5 at -60°C or lower, and then about 2 g of dry ice was added at -40°C or lower. The solution was stirred at room temperature for 3 hours, and then the solvent was distilled off under reduced pressure. 50 ml of a 3N-hydrochloric acid was added to the residue, and the solution was filtered. The resulting crystal was dried under reduced pressure to obtain 2.8 g of 4-(4-propylpiperidyl)benzoic acid (yield: 98%). This compound was used for the subsequent reaction as it was without purifying.

Dicyclohexylcarbodimide (DCC) dissolved in 10 ml of CH₂Cl₂ was added to a CH₂Cl₂ solution (40 ml) of a mixture of 2.8 g (12 mmol) of the resulting benzoic acid derivative, 2.4 g (15 mmol) of 3,5-difluoro-4-cyanophenol and 2.1 ml (15 mmol) of Et₃N at 0°C. After stirring the solution at room temperature overnight, 20 ml of ethyl acetate was added, and the solution was stirred for 5 minutes, followed by distilling the solvent off under reduced pressure. The residue was refined by means of column chromatography (toluene : ethyl acetate = 30 : 1) and recrystallization (ethanol) to obtain 2.1 g of 3,5-difluoro-4-cyanophenoxy 4-(4-propyl-1-piperidyl)benzoate as a colorless crystal. (Yield: 48%).
Phase transition point: C·94.5·N·130.9·I
MS m/e = 384 (M⁺)
¹H-NMR (90 MHz) δ (ppm): 7.97 (d, 2H, J=5.31 Hz), 6.8-7.1 (m, 4H), 3.8-4.1 (m, 2H), 2.7-3.1 (m, 2H), 0.8-1.9 (m, 12H)

### Example 12

### Synthesis of 2,3-difluoro-4-ethoxy-1-(4-propyl-1-piperidyl)benzene (the compound of Formula (1), wherein Z is the moiety (V); R is propyl, and R' is ethoxy; l = m = n = p = 0; and B⁵ is a single bond: Compound No. 380)

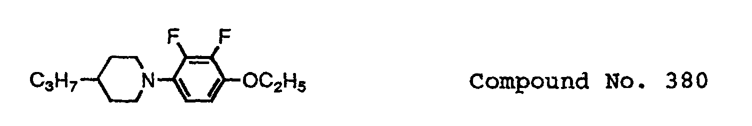

7.0 g (44 mmol) of 2,3-difluoroethoxybezene was subjected to lithiation at -50°C or lower with 32 ml (44 mmol) of a sec-BuLi 1.38M-solution using 100 ml of THF as a solvent by the same method as described in JP61-112070. After stirring the solution at -70°C for 15 minutes, 2.3 ml (45 mmol) of bromine was added at -50°C or lower, and the solution was stirred at room temperature for 2 hours. The reaction mixture was treated according to a conventional method and further refined by distillation under reduced pressure (88°C/5.0 mmHg), whereby 7.2 g of the desired 4-bromo-2,3-difluoroethoxybenzene was obtained. (Yield: 69%).

5.3 g (55 mmol) of sodium t-butoxide was added to a mixture of 10 g (42 mmol) of this bromide, 8.1 g (64 mmol) of 4-propylpiperidine obtained in Example 5, 97 mg (0.10 mmol) of Pd₂(dba)₃ and 197 mg (0.32 mmol) of BINAP, and 100 ml of toluene was further added as a solvent to react them at 100°C for 5 hours under argon atmosphere. After cooling down the reaction mixture, it was treated in the same manner as in the above example, and the residue was refined by means of column chromatography (heptane : toluene 1 : 1 → toluene) and recrystallization (heptane) to obtain 2.9 g of the desired 2,3-difluoro-4-ethoxy-1-(4-propyl-1-piperidyl)benzene. (Yield: 24%).
Phase transition point: C·50.2(·N·10.0)·I
MS m/e = 283 (M⁺)
¹H-NMR (500 MHz) δ (ppm): 6.58-6.64 (m, 2H), 4.06 (q, 2H, J=6.95 Hz), 3.32 (d, 2H, J=11.9 Hz), 2.59 (t, 2H, J=11.4 Hz), 1.76 (d, 2H, J=11.2 Hz), 1.20-1.43 (m, 10H), 0.91 (t, 3H, 7.05 Hz)
¹⁹F-NMR (470 MHz) δ (ppm): -147.2 to -147.3 (m, 1F), -158.7 to -158.8 (m, 1F)

### Example 13

### Synthesis of 2,3-difluoro-4-ethoxy-1-(4-(4-propylcyclohexyl)-1-piperidyl)benzene (the compound of Formula (1), wherein Z is the moiety (V); R is propyl, and R' is ethoxy; l = 1, m = n = p = 0; B¹ and B⁵ are single bonds; and A¹ is trnas-1,4-cyclohexylene: Compound No. 384)

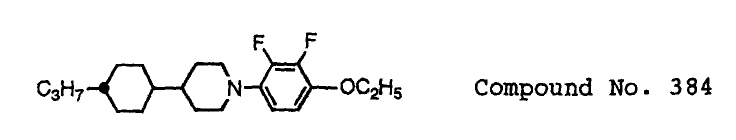

5.0 g (24 mmol) of 4-(4-propyl)cyclohexylpiperidine synthesized in Example 8 was reacted with 7.0 g (29 mmol) of 4-bromo-2,3-difluoroethoxybenzene in the same manner as in Example 11 using 3.3 g (34 mmol) of sodium t-butoxide as a base, 66 mg (0.072 mmol) of Pd₂(dba)₃ as a catalyst and 134 mg (0.21 mmol) of BINAP, and 0.57 g of the desired compound was obtained by taking the same refining step as described above. (Yield: 6.5%).
Phase transition point: C·81.3·N·167.0·I
MS m/e = 365 (M⁺)
¹H-NMR (500 MHz) δ (ppm): 6.58-6.64 (m, 2H), 4.06 (q, 2H, J=7.10 Hz), 3.35 (d, 2H, J=11.5 Hz), 2.56 (t, 2H, J=9.90 Hz), 0.89-1.78 (m, 22H), 0.88 (t, 3H, J=7.45 Hz)
¹⁹F-NMR (470 MHz) δ (ppm): -147.2 to -147.3 (m, 1F), -158.6 to -158.8 (m, 1F)

The following compounds (Nos. 1 to 470) can be produced according to Examples 1 to 13 as described above.

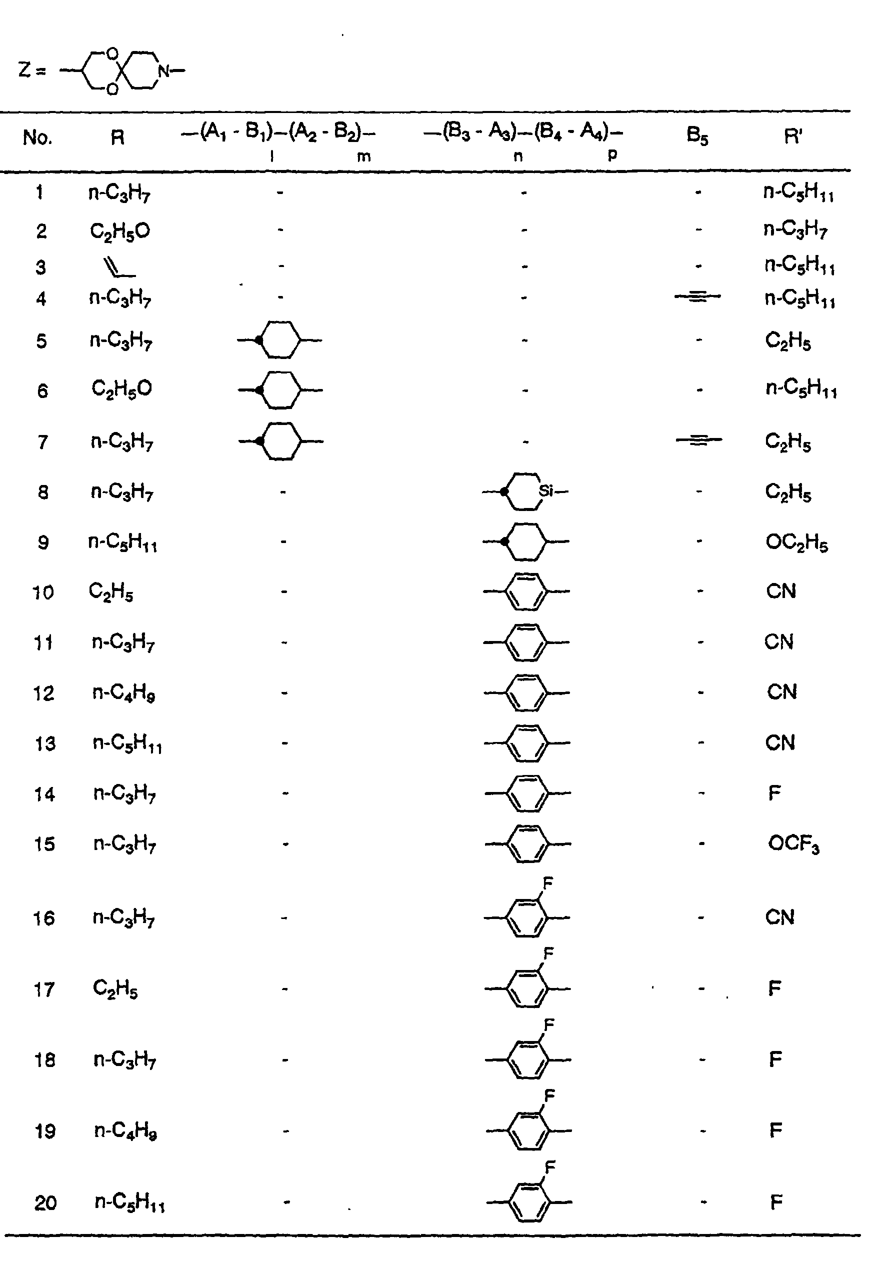

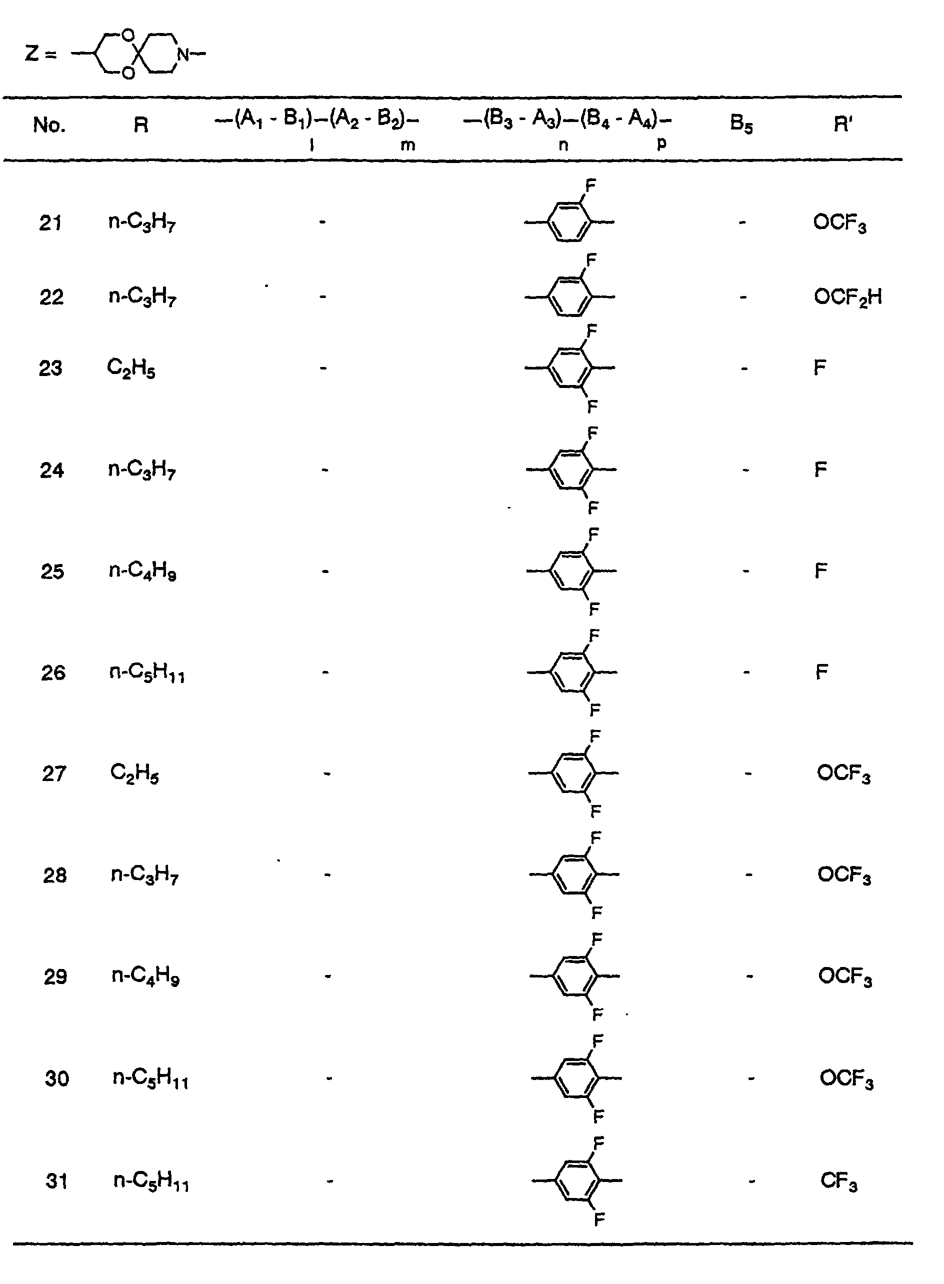

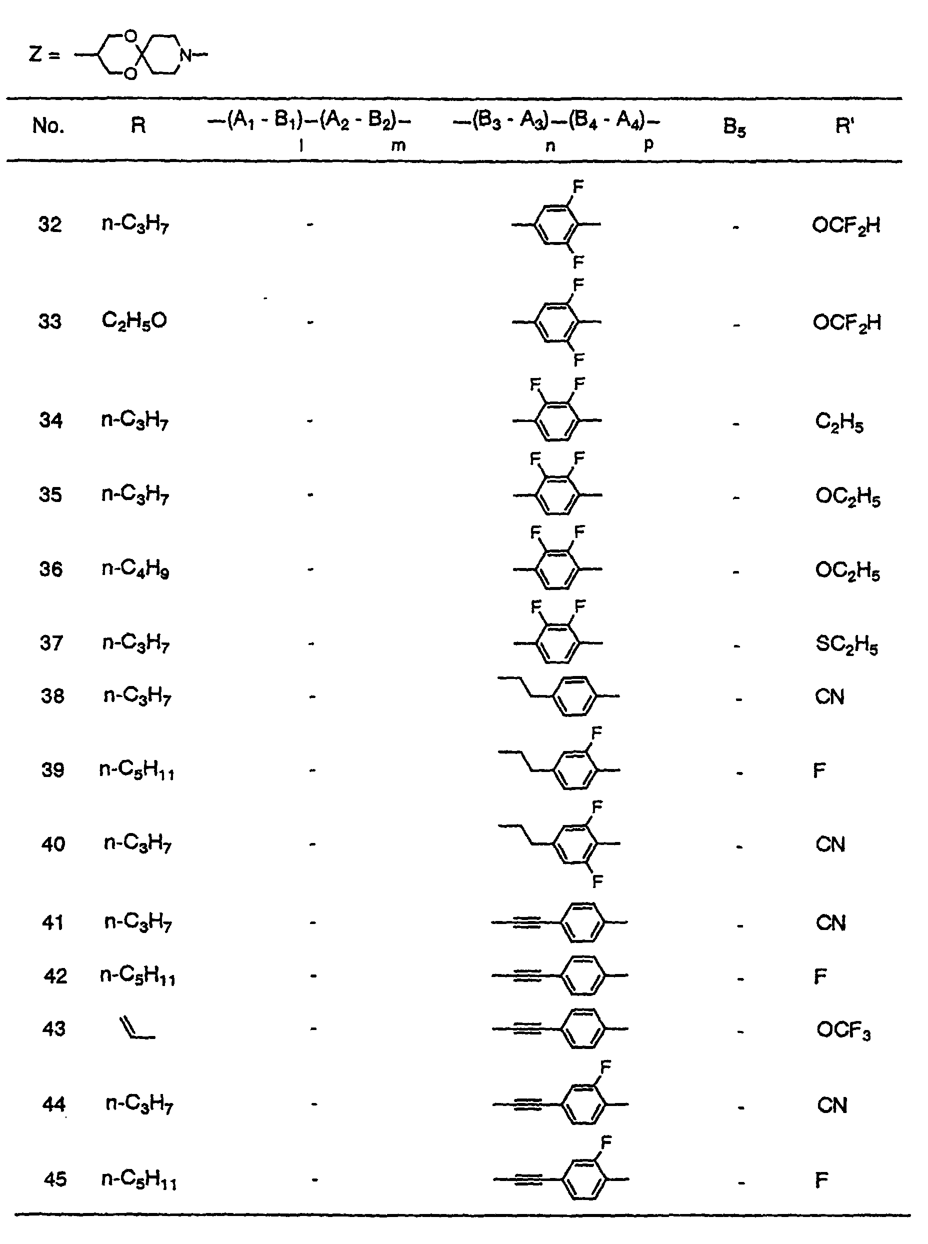

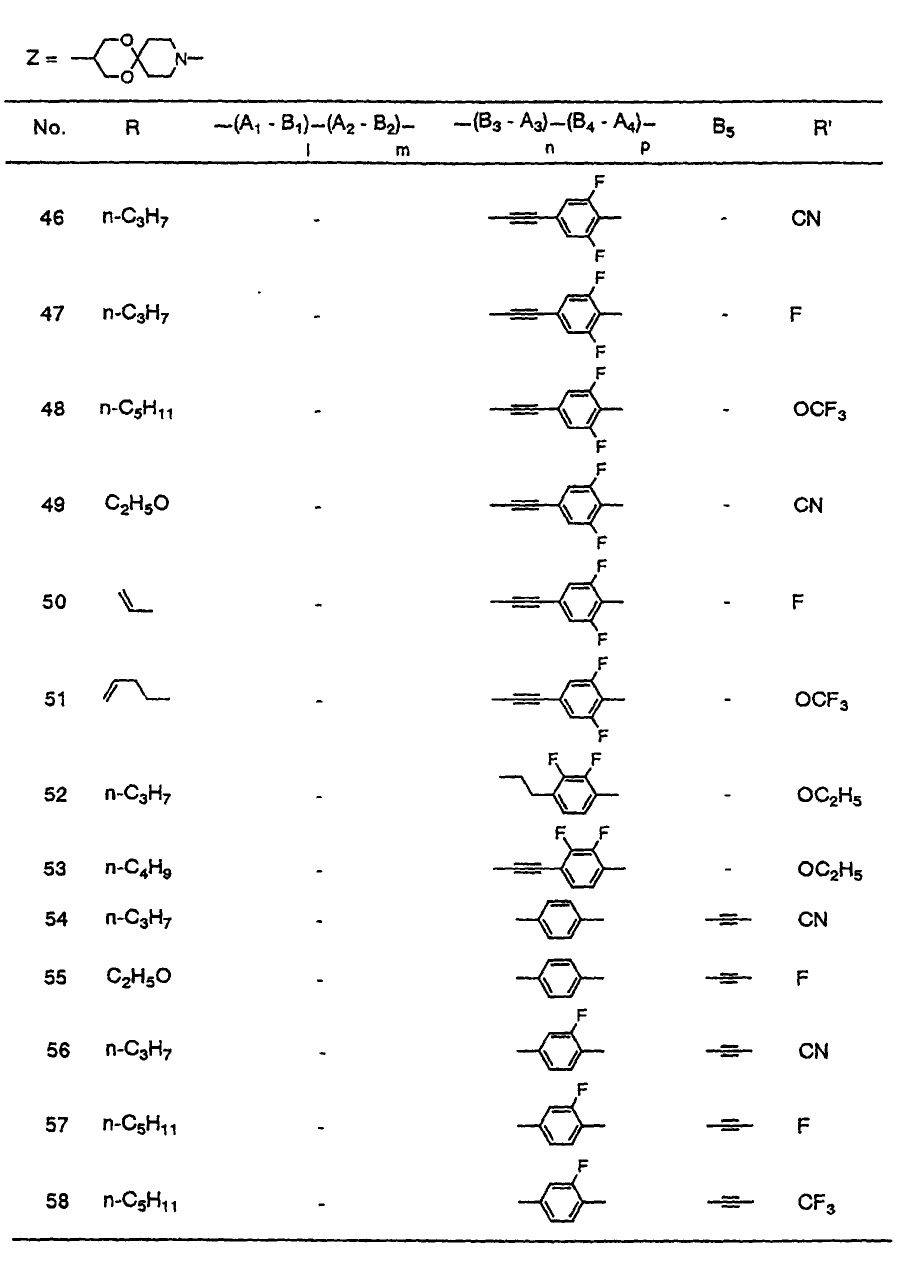

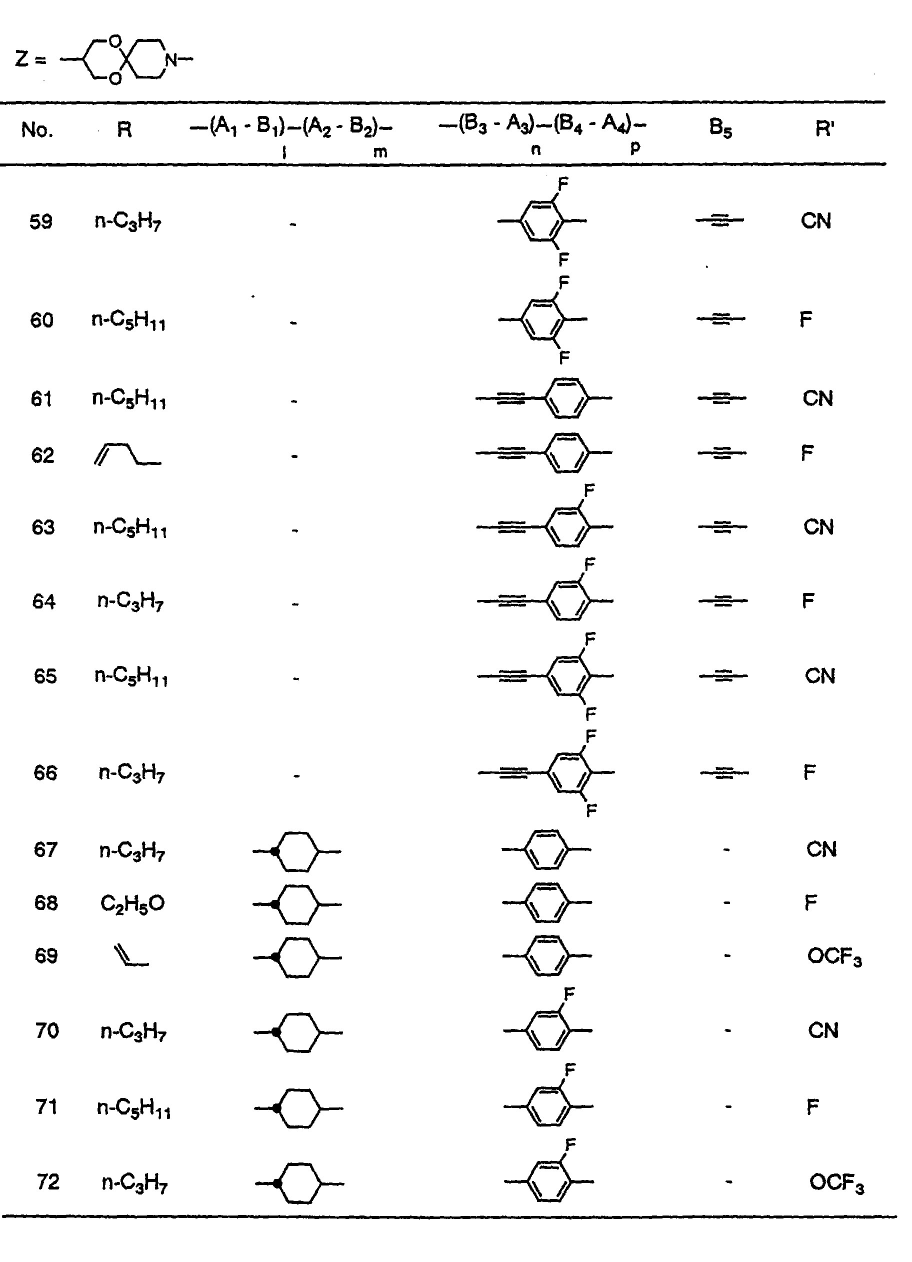

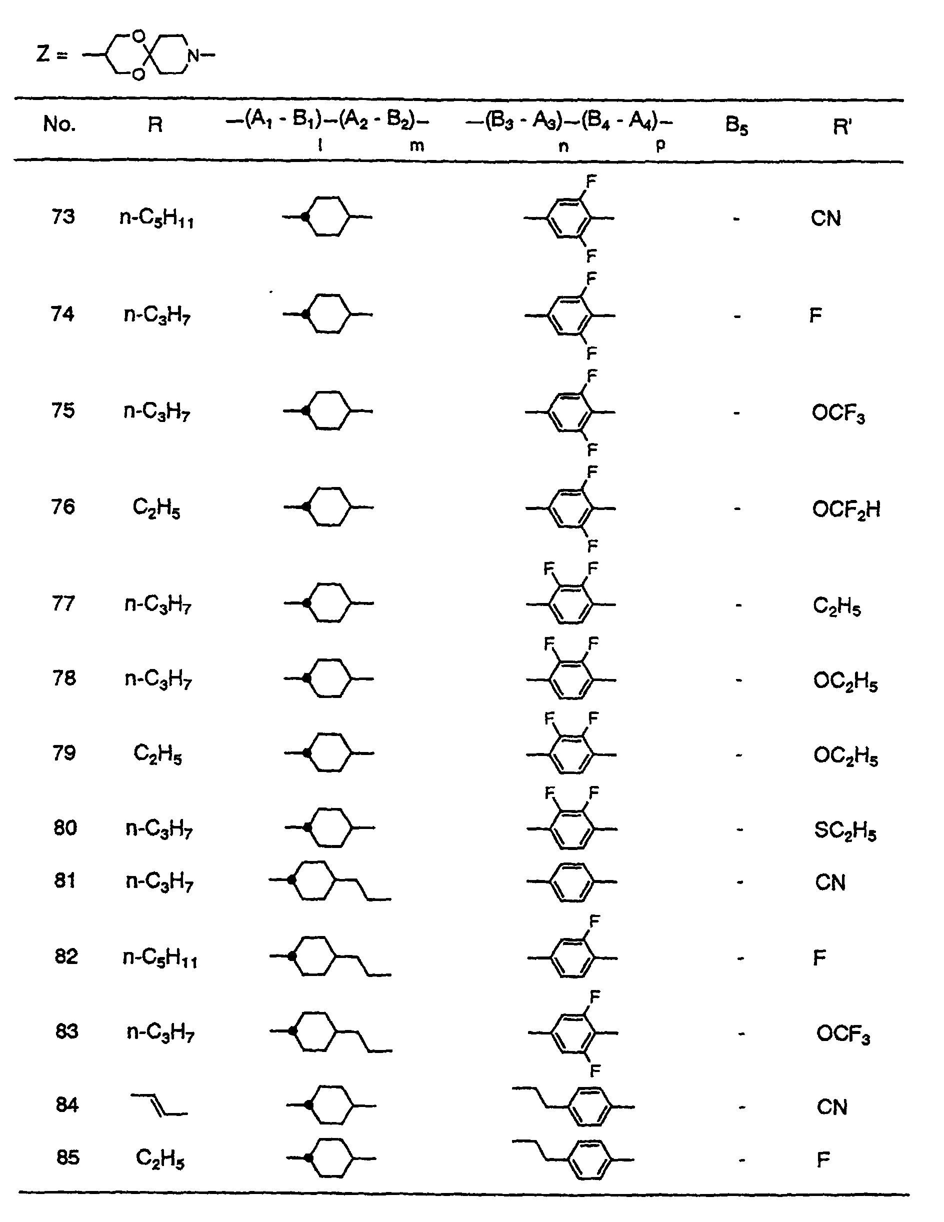

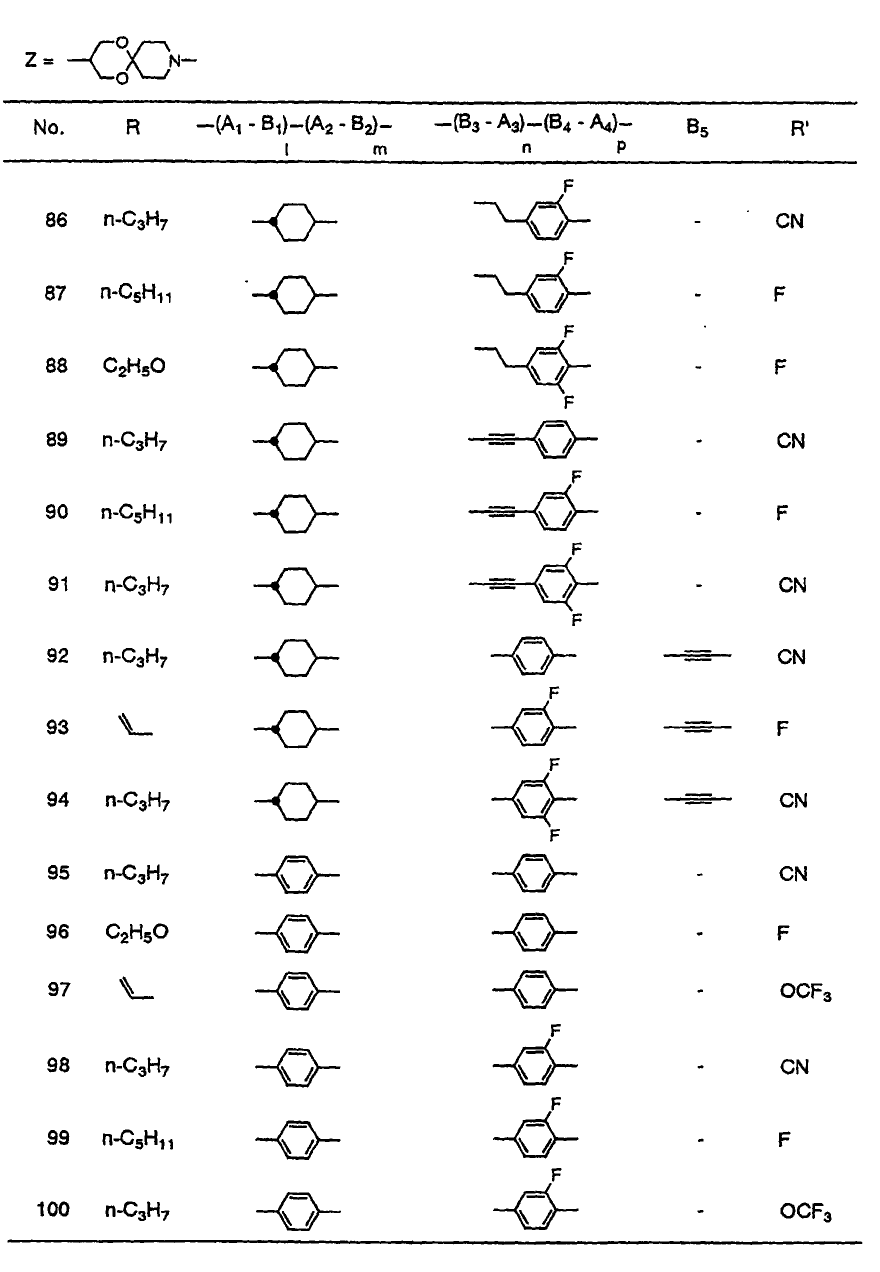

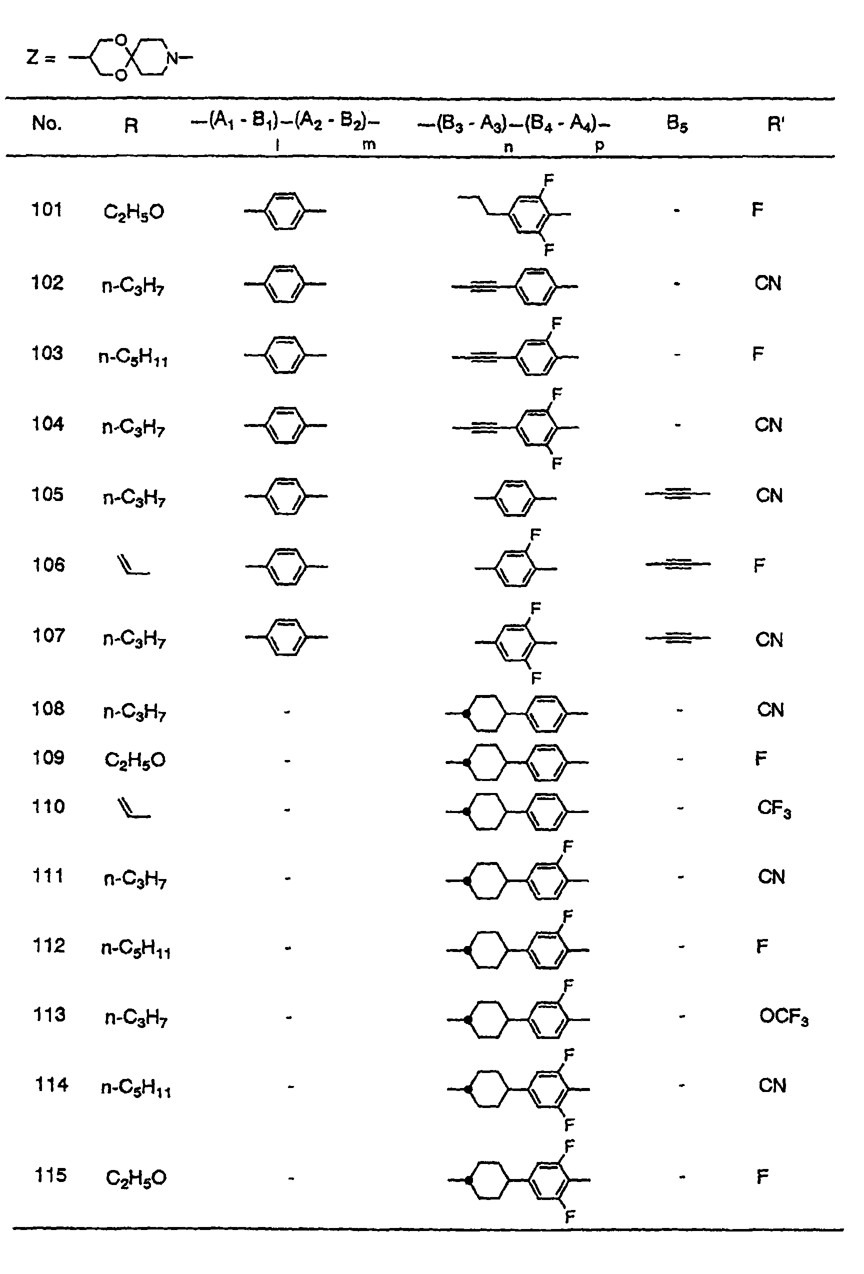

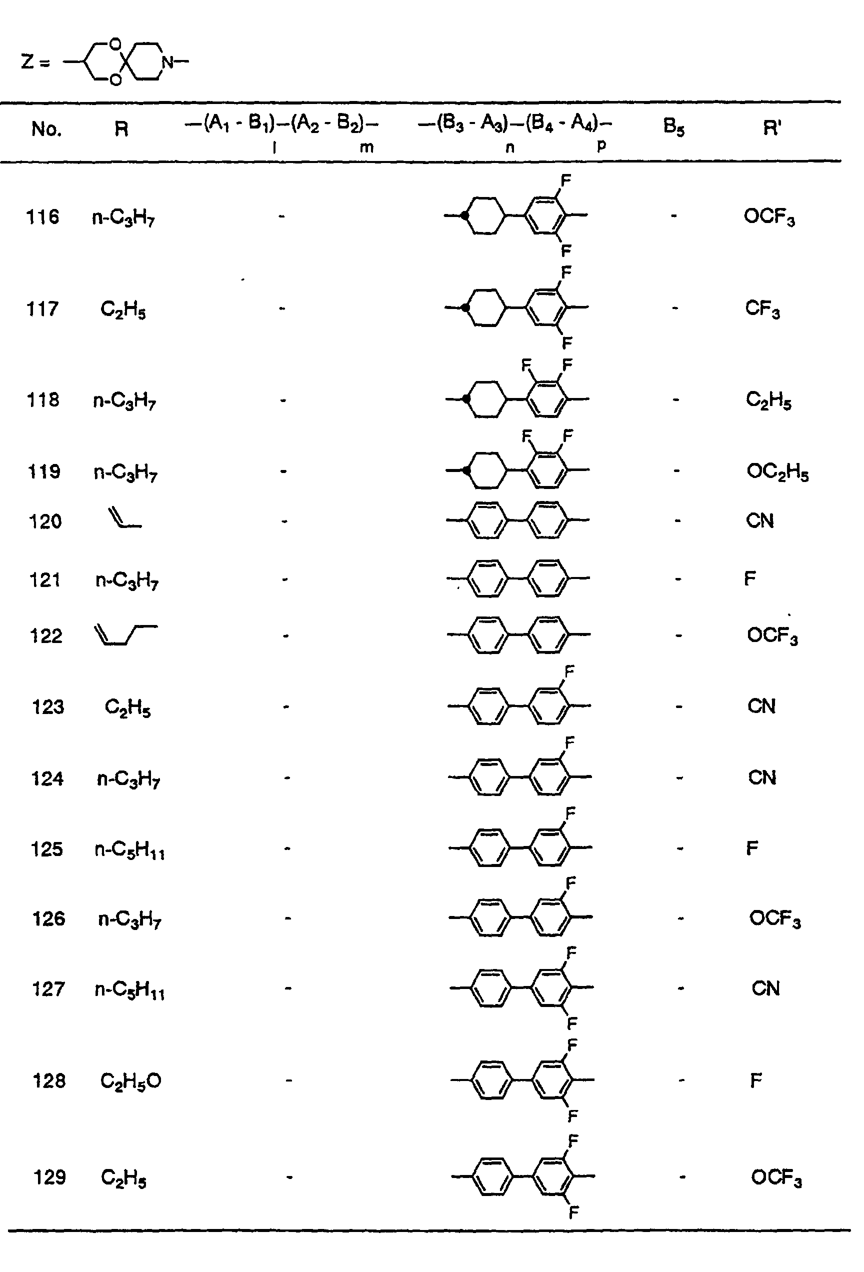

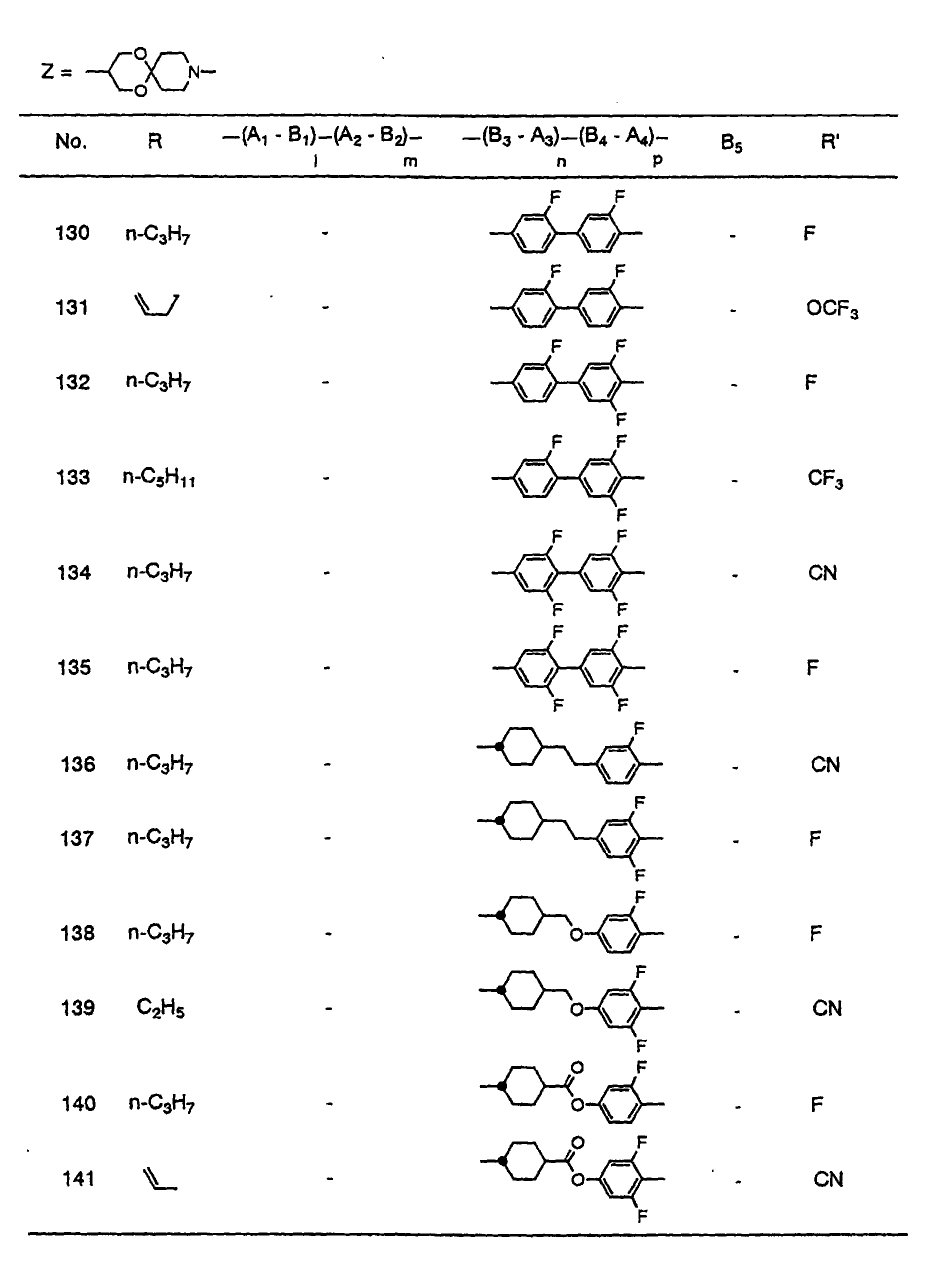

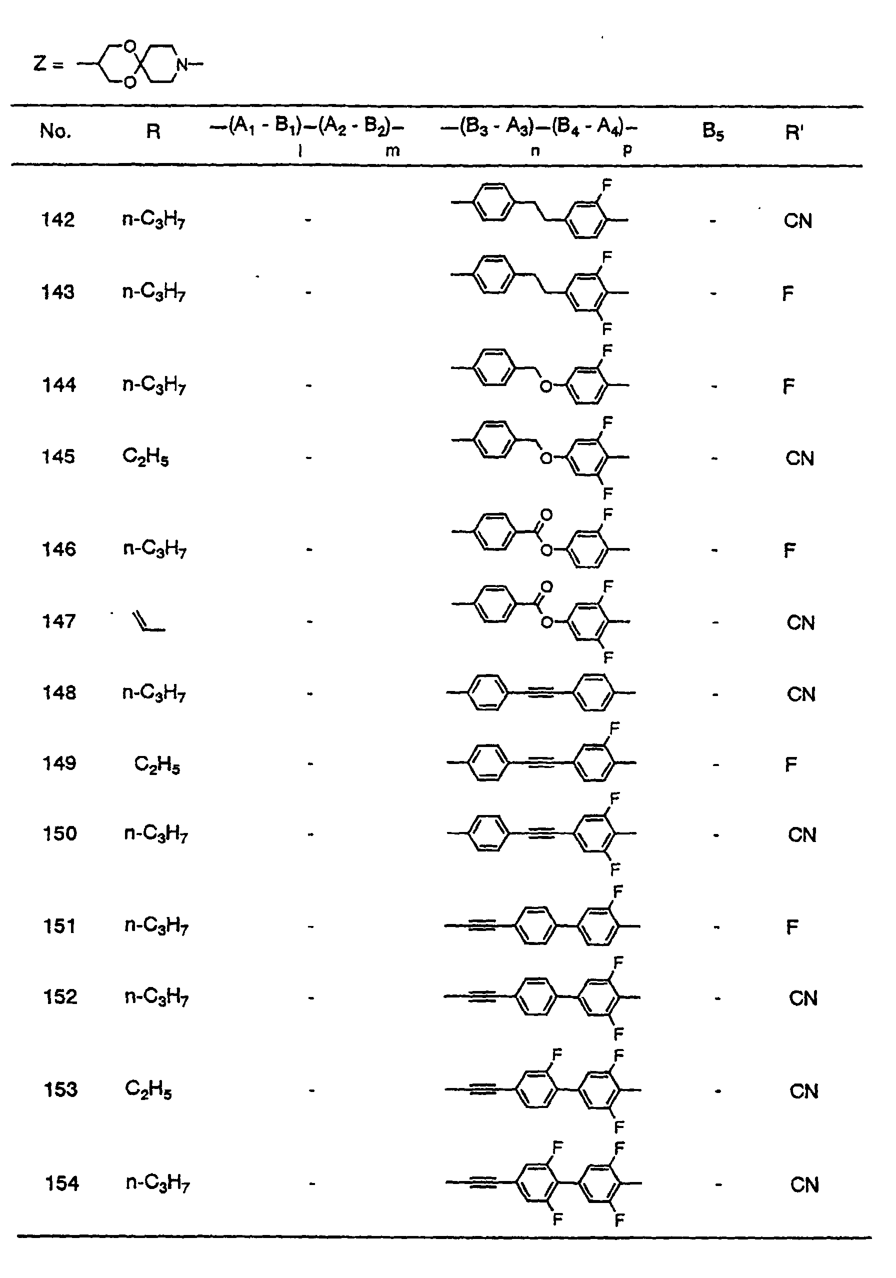

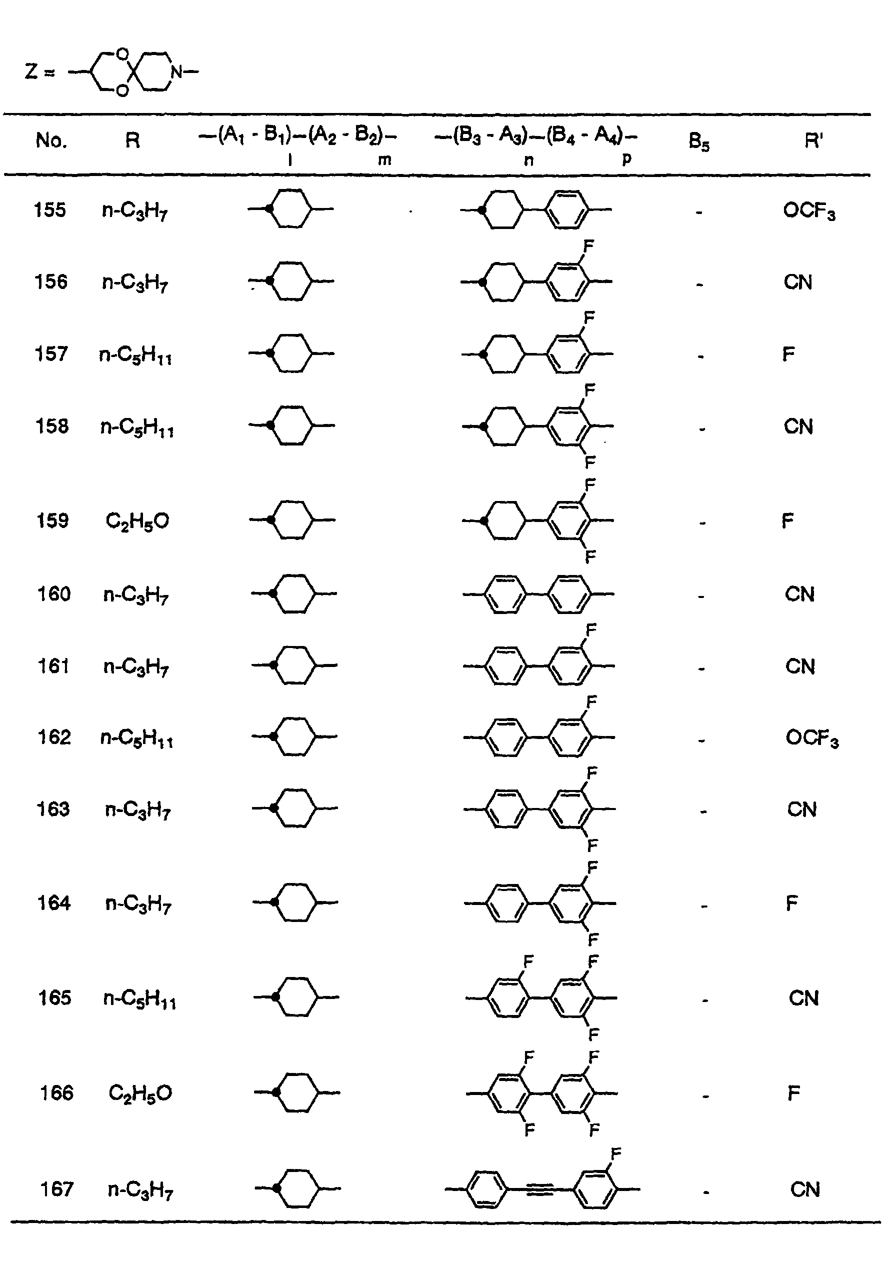

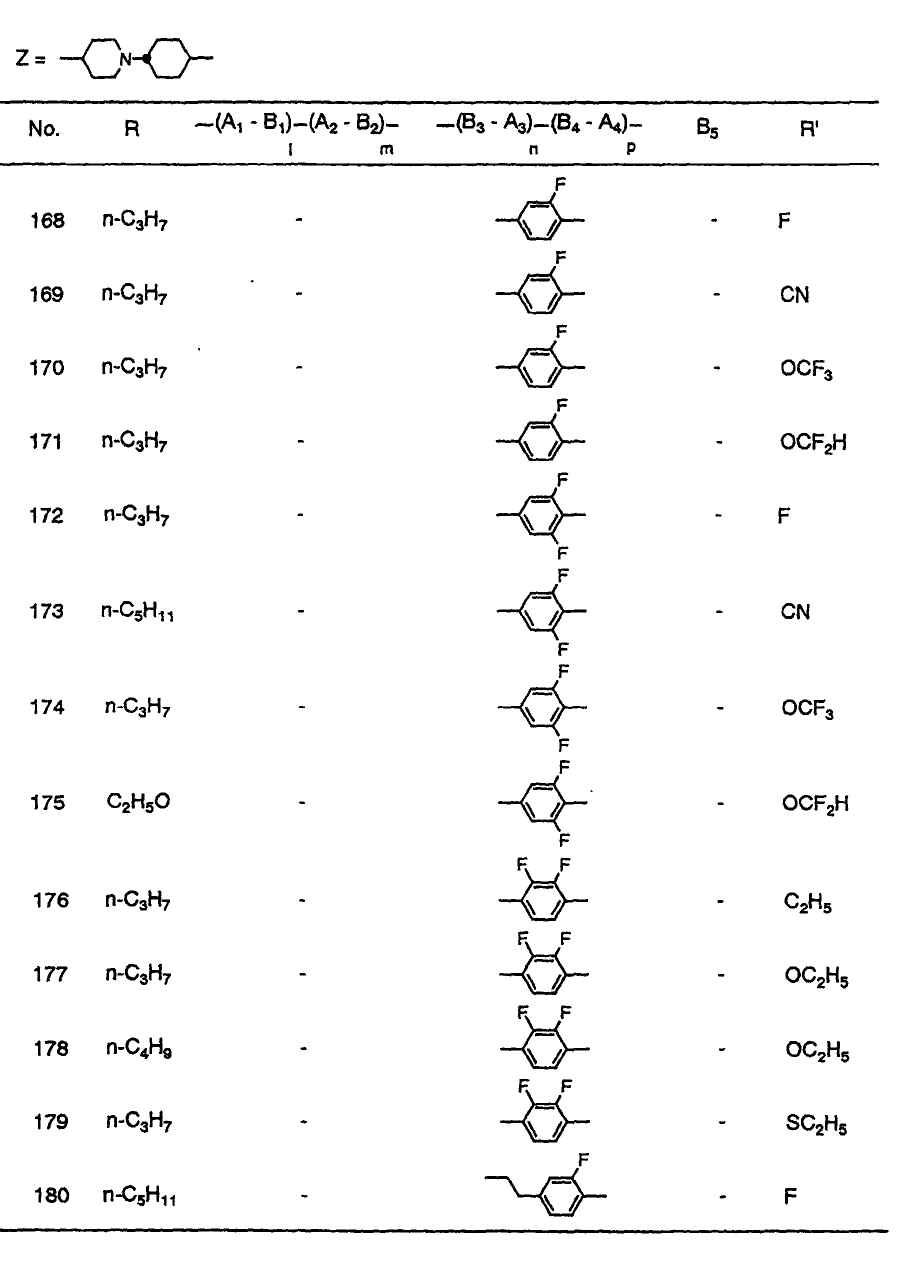

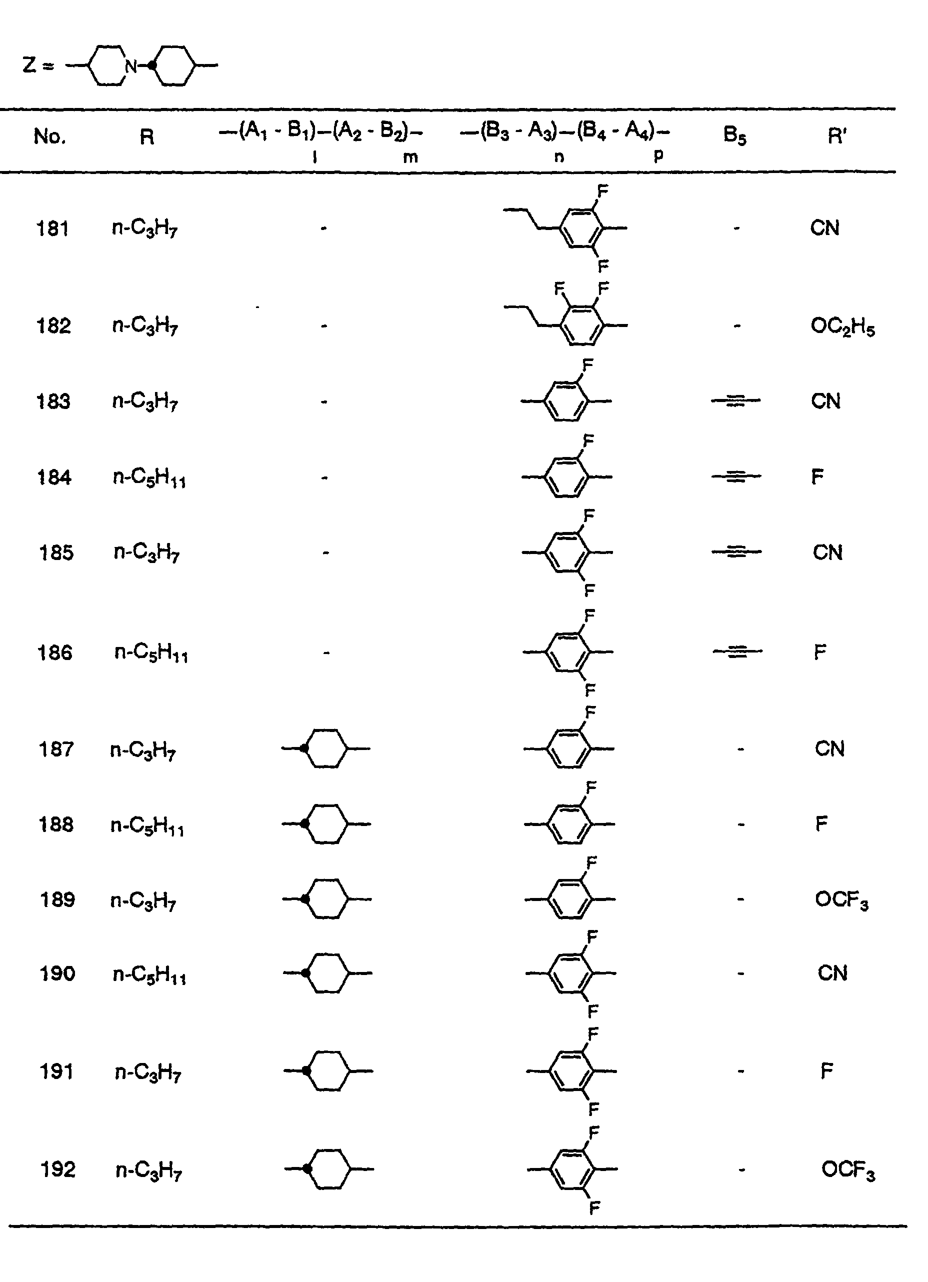

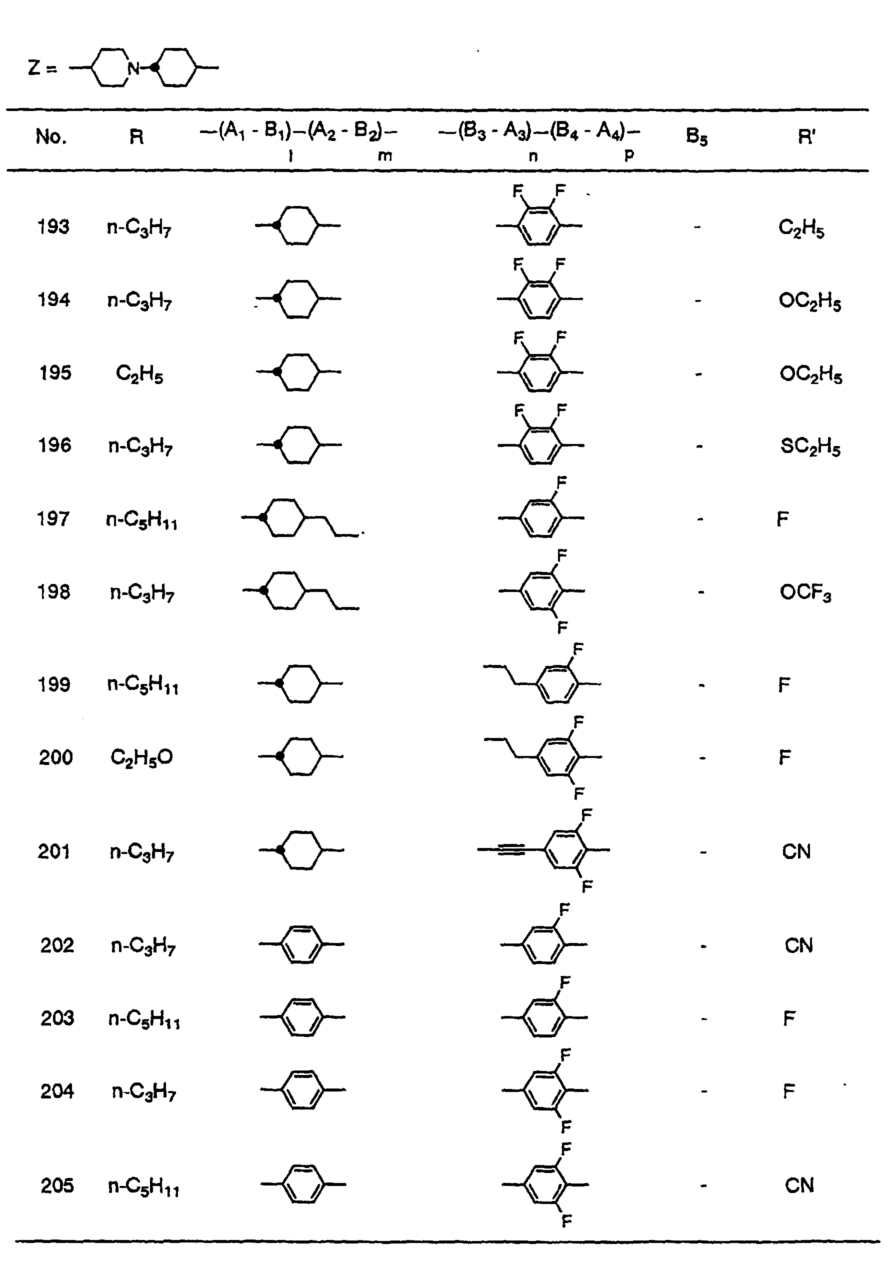

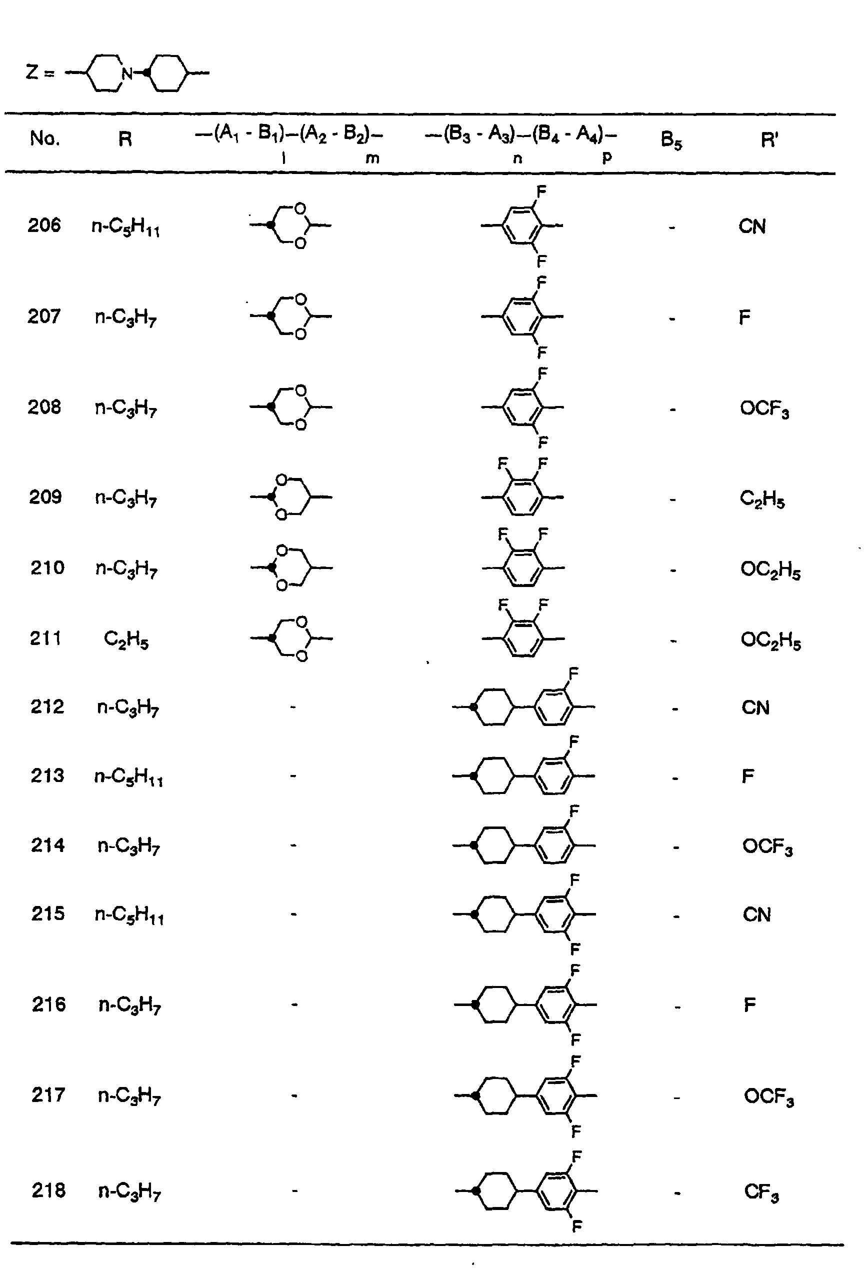

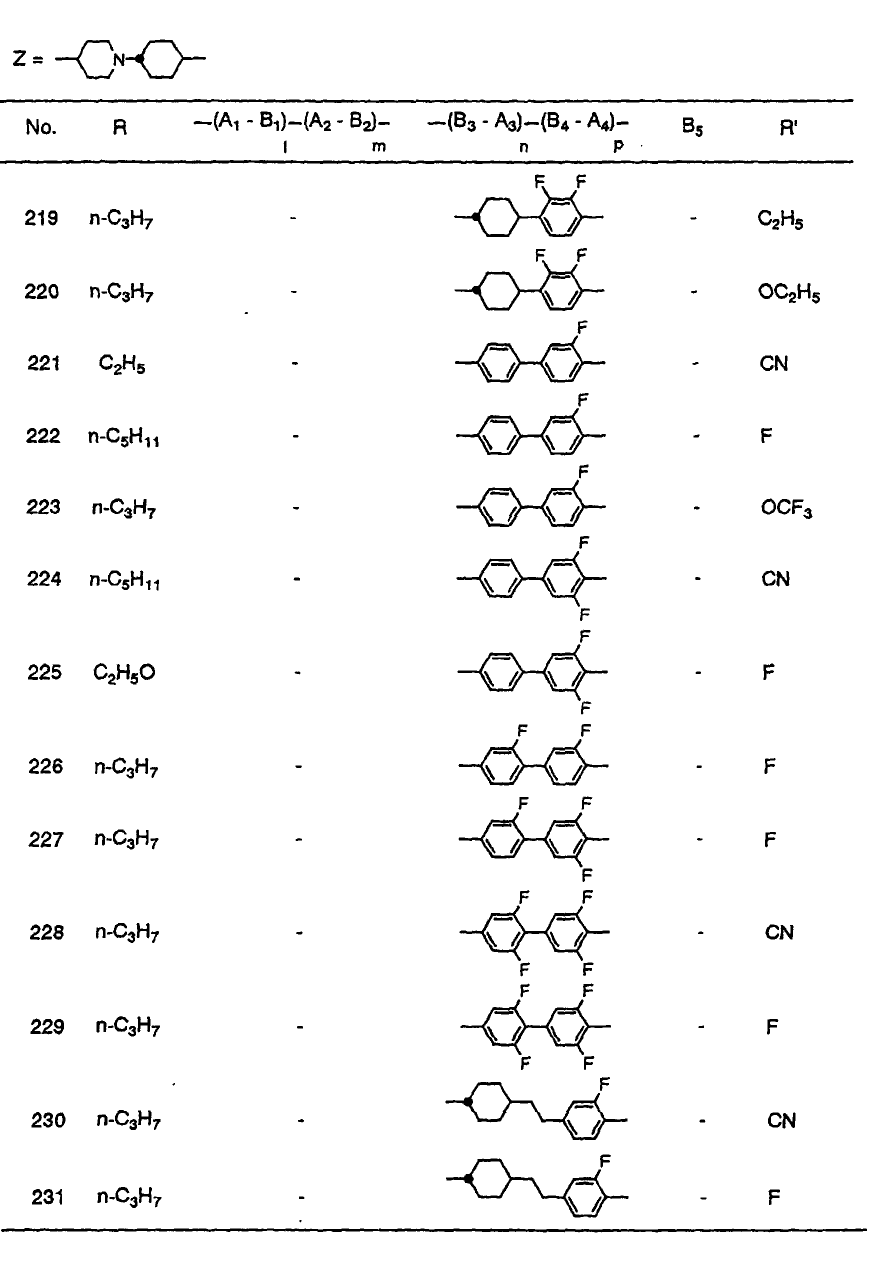

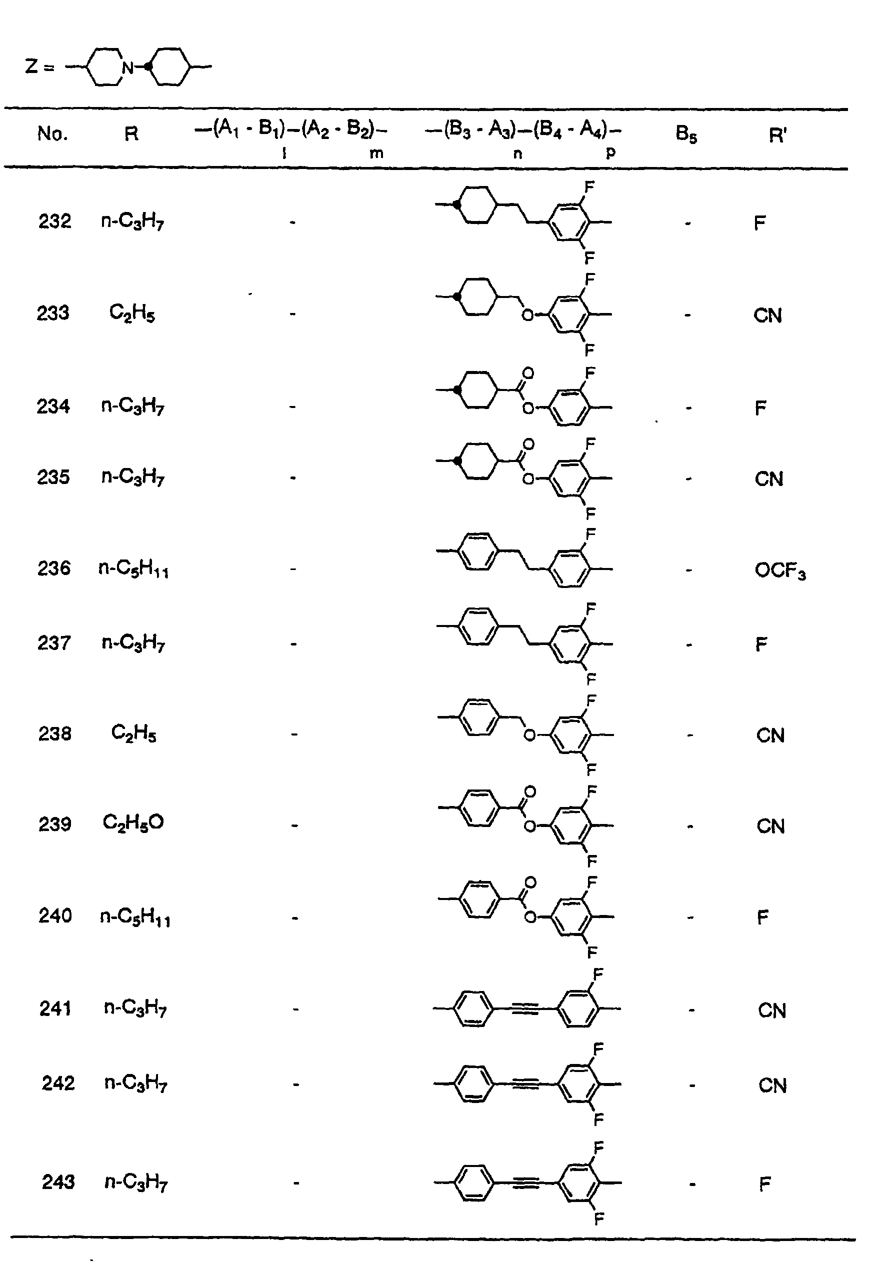

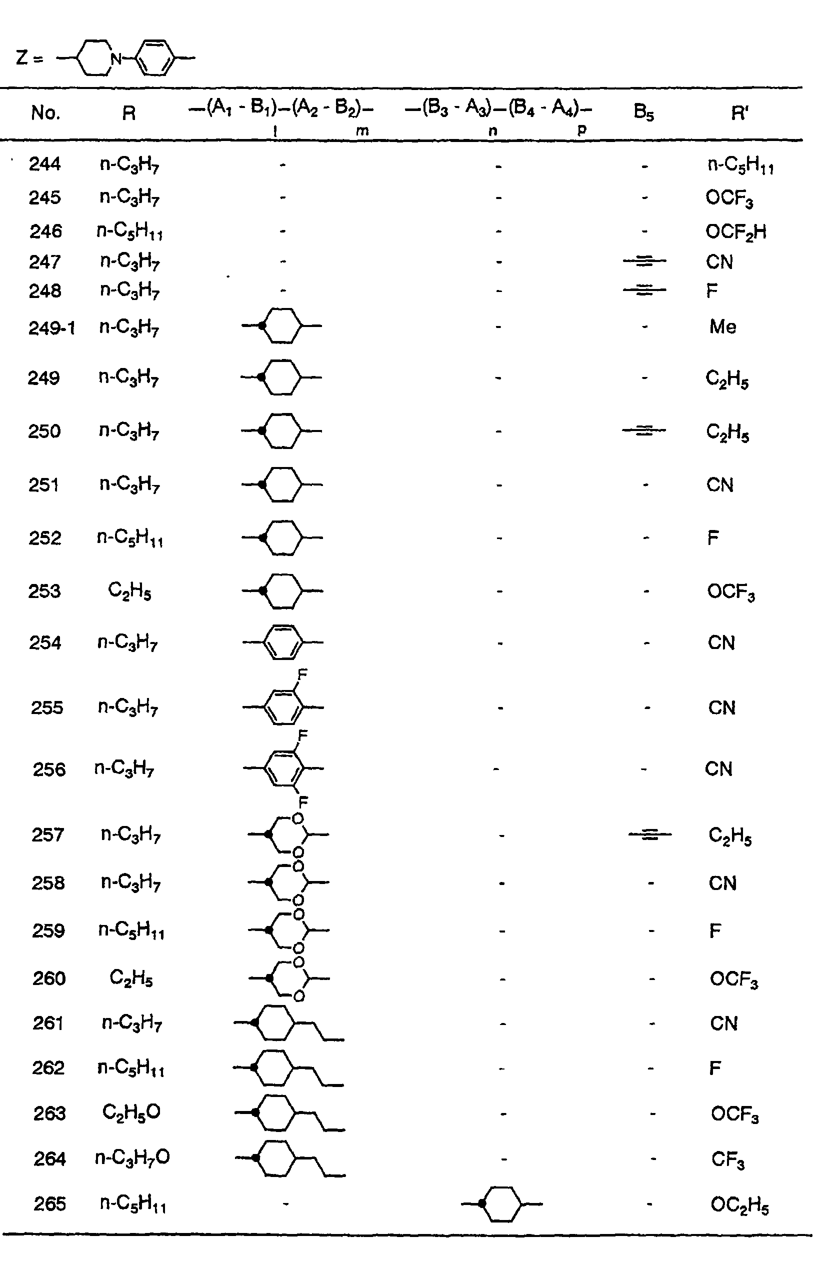

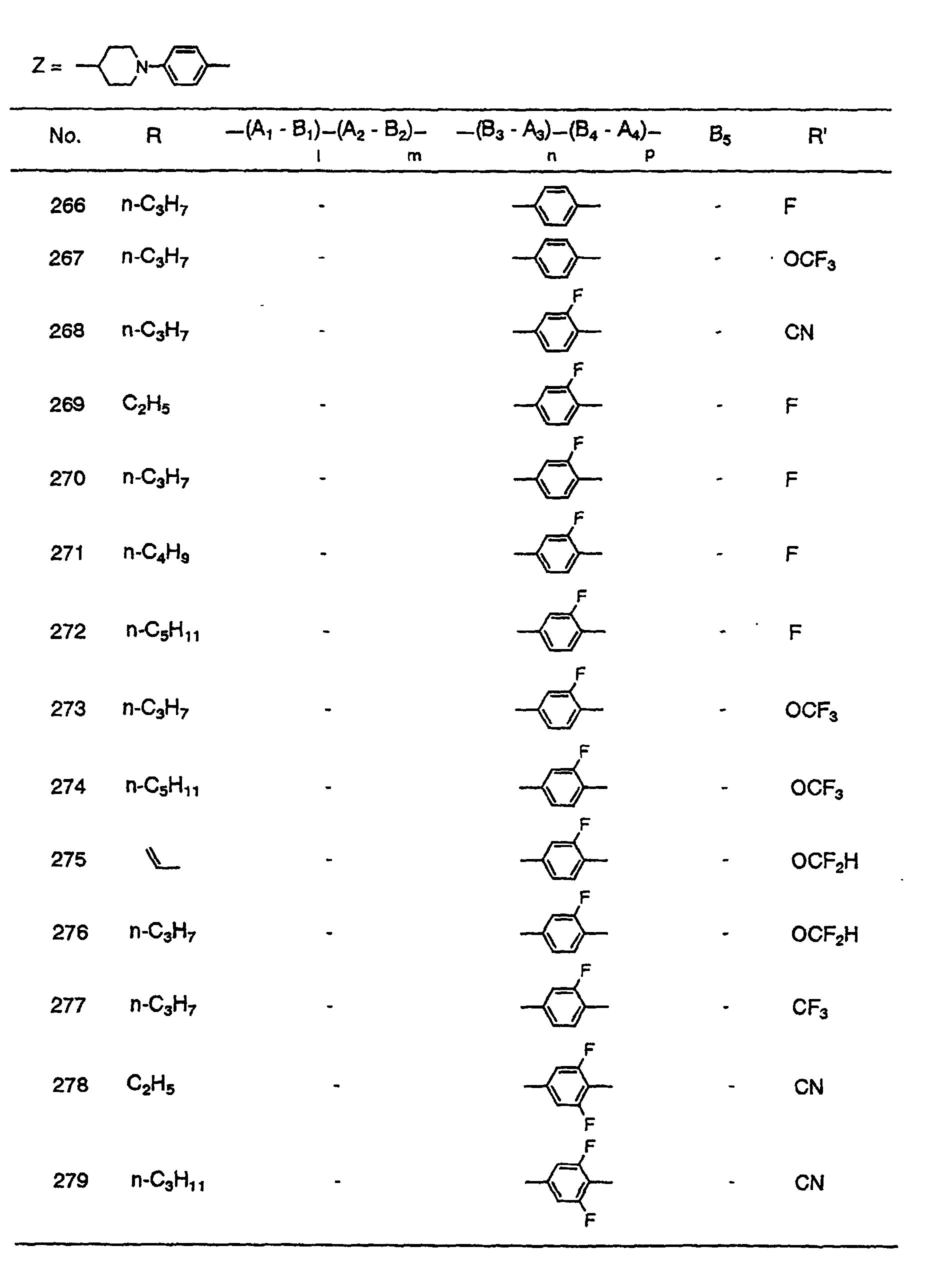

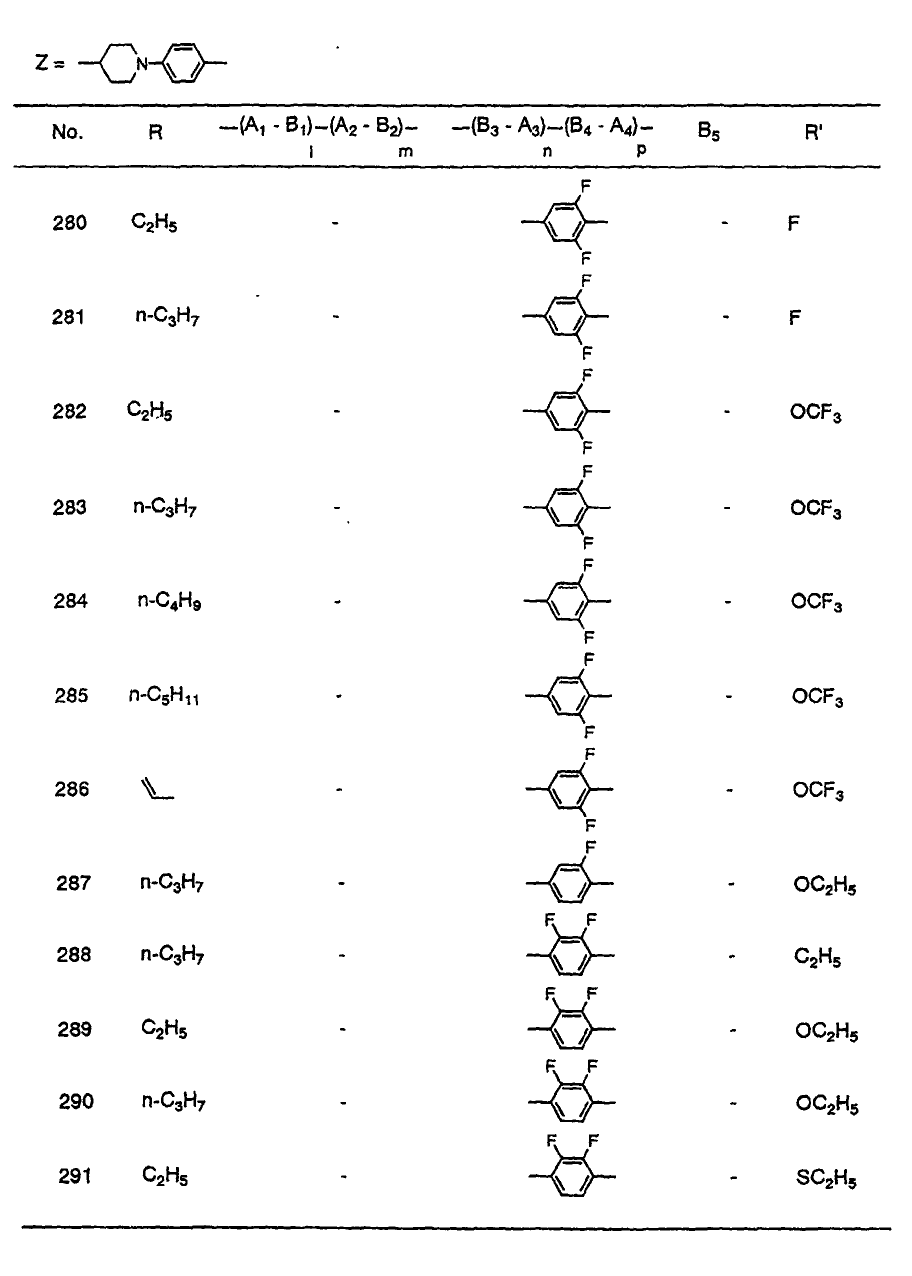

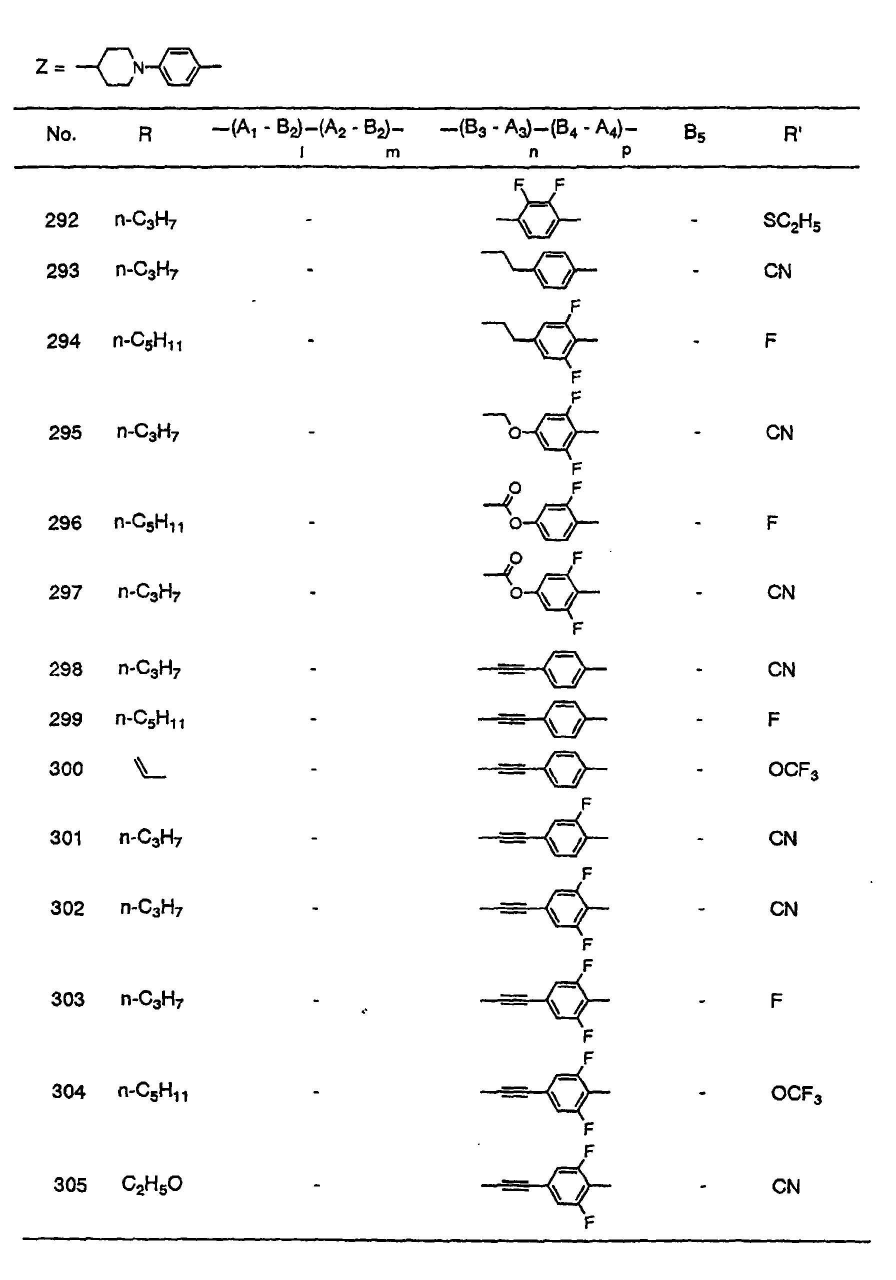

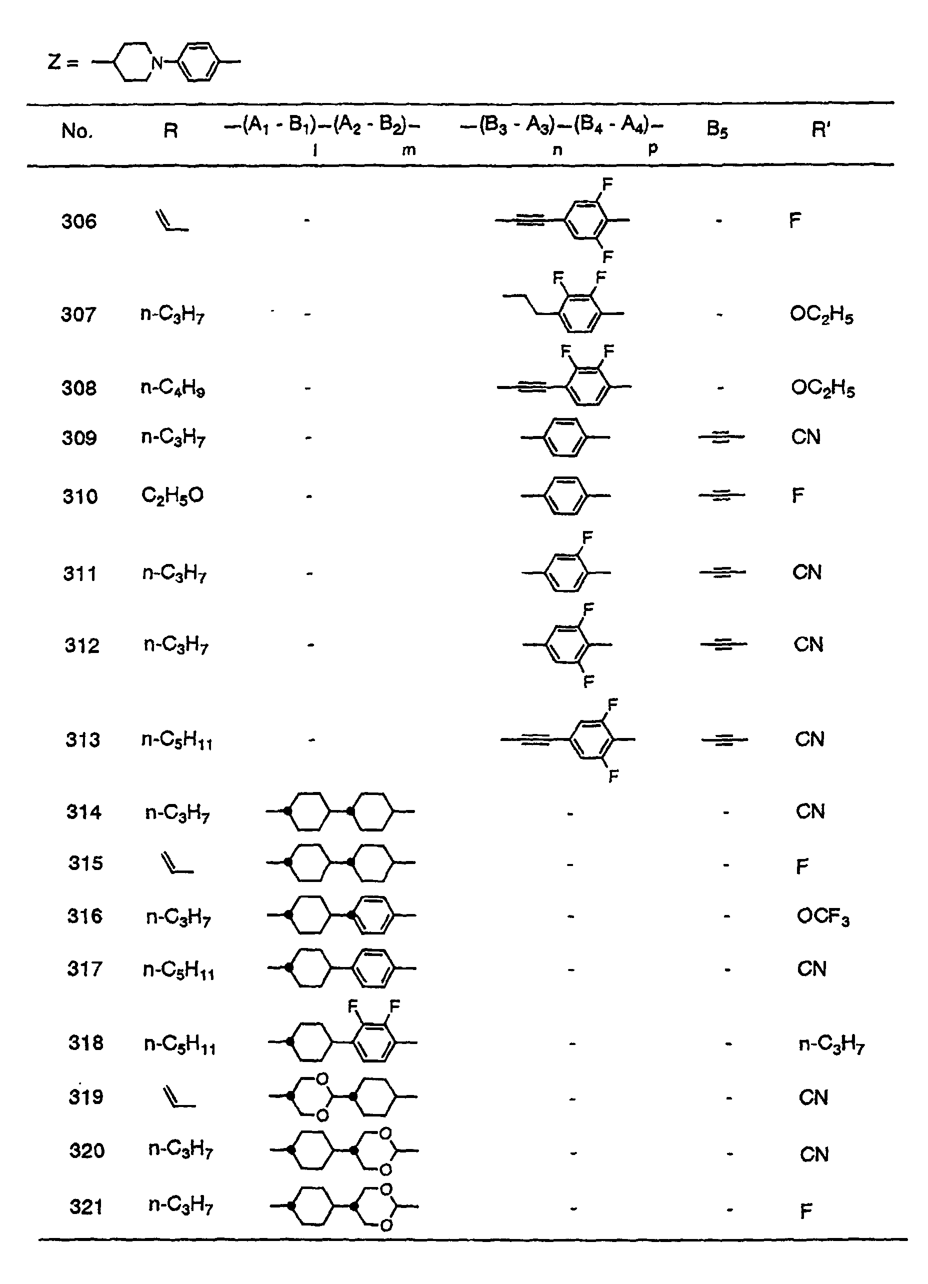

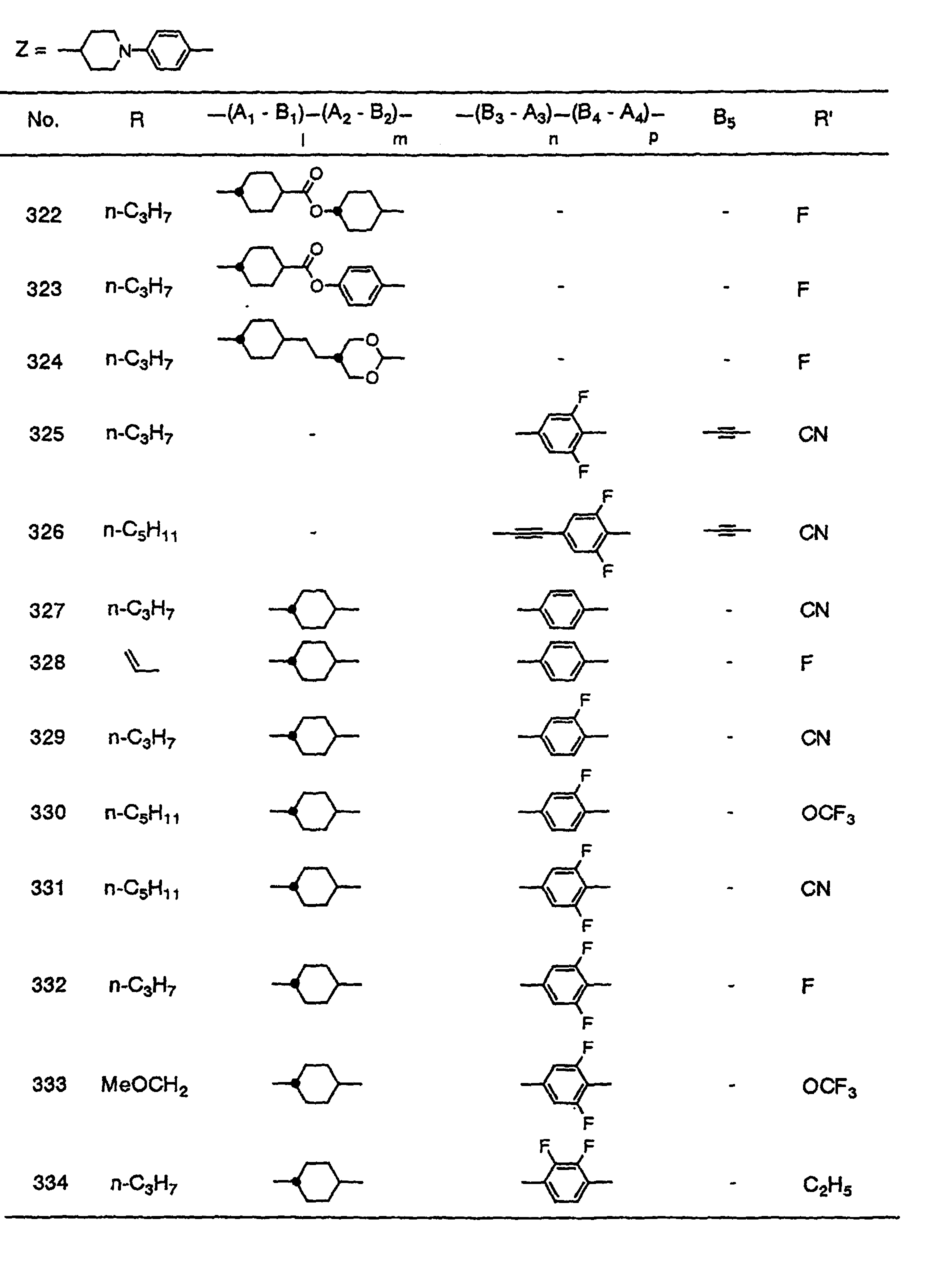

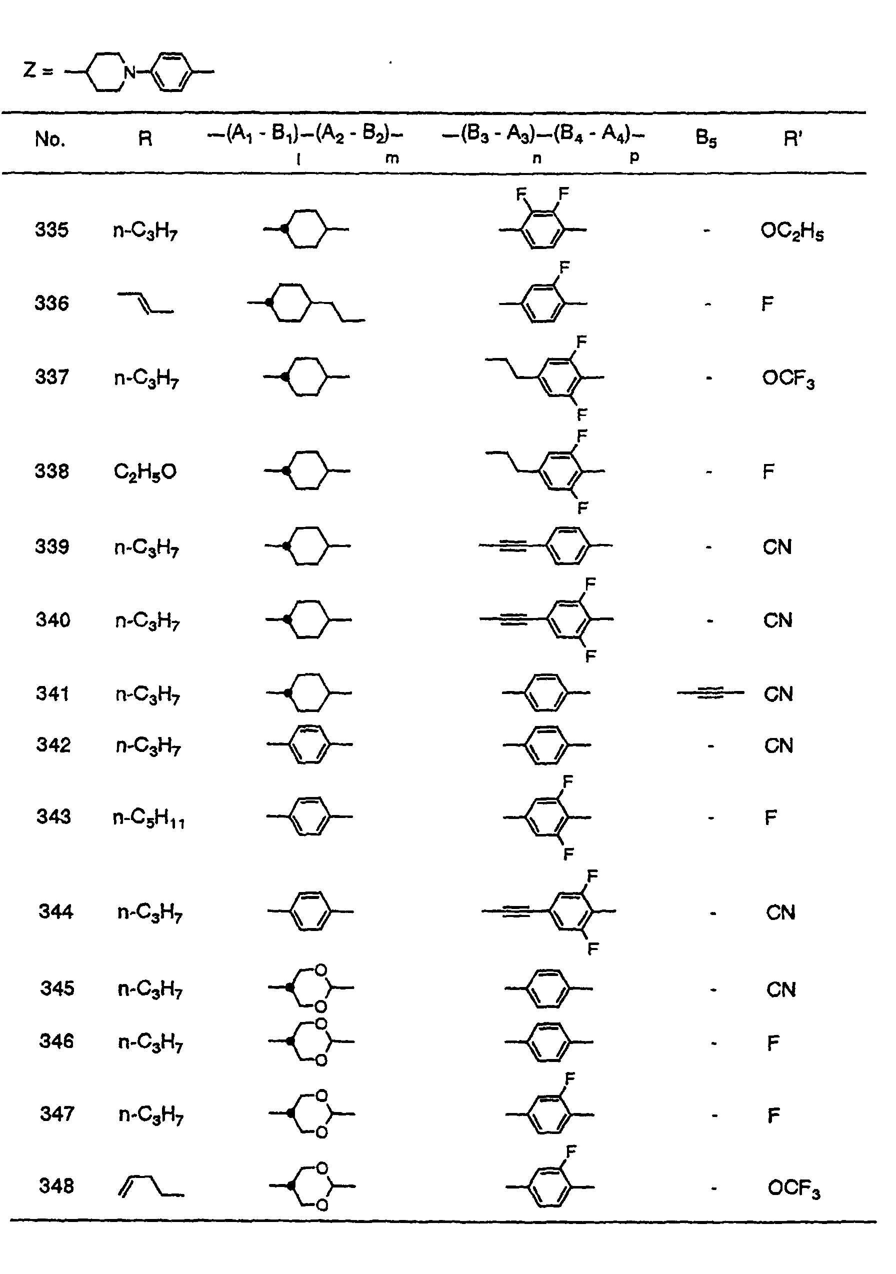

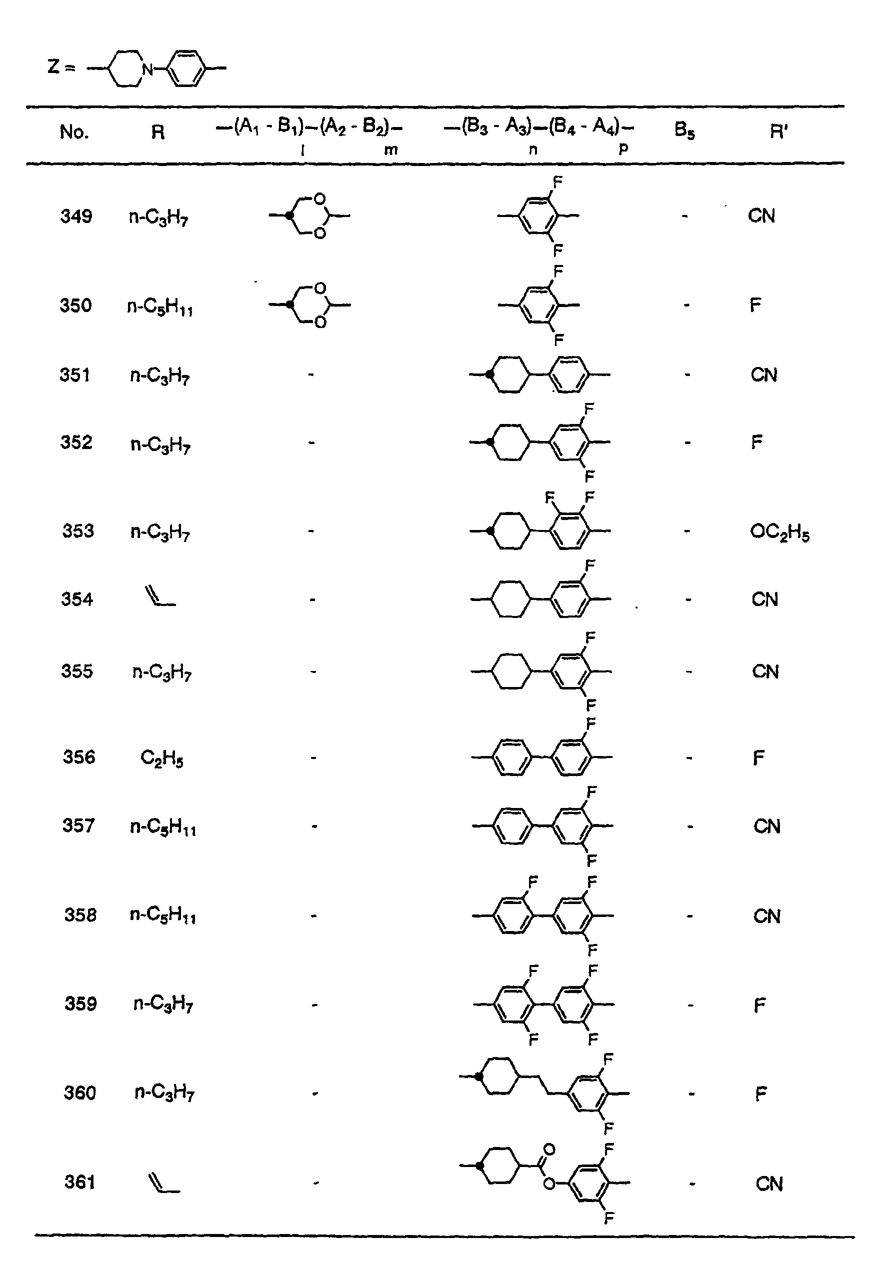

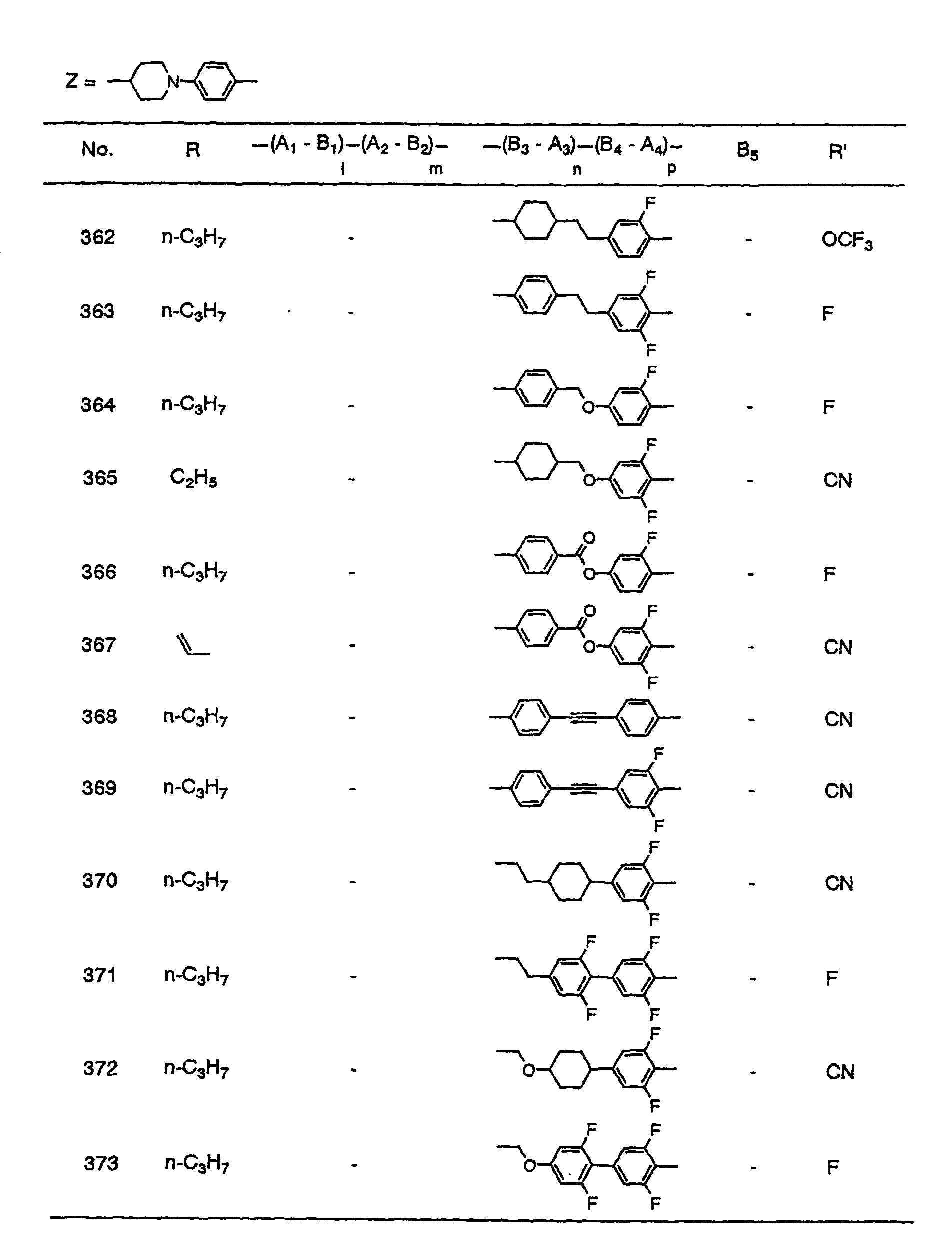

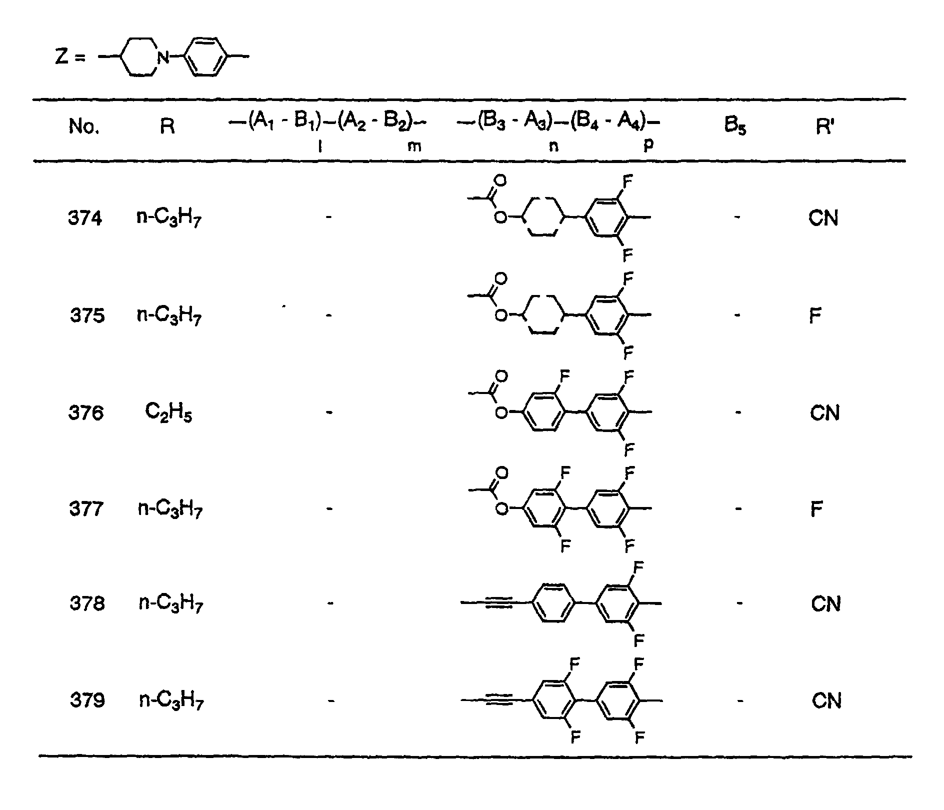

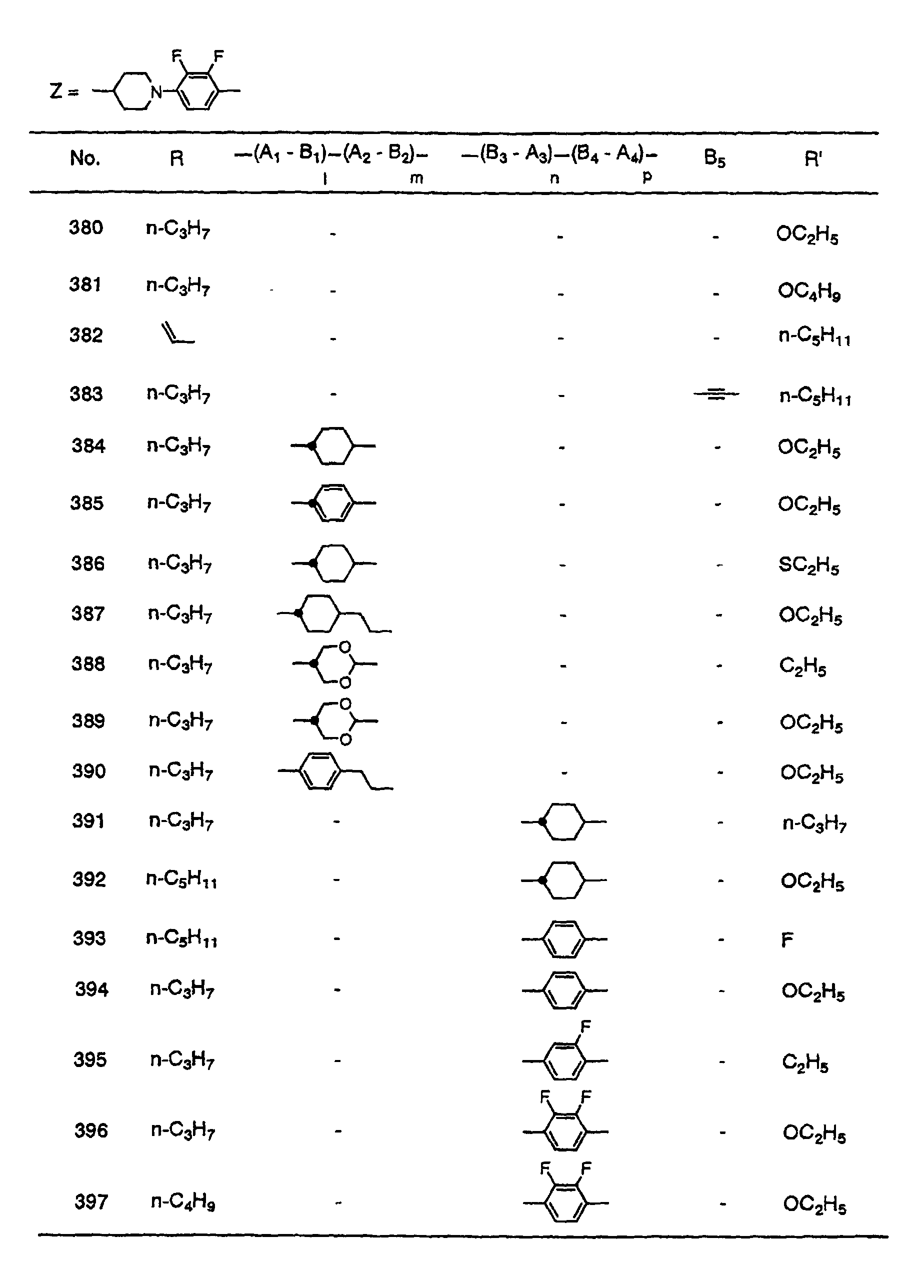

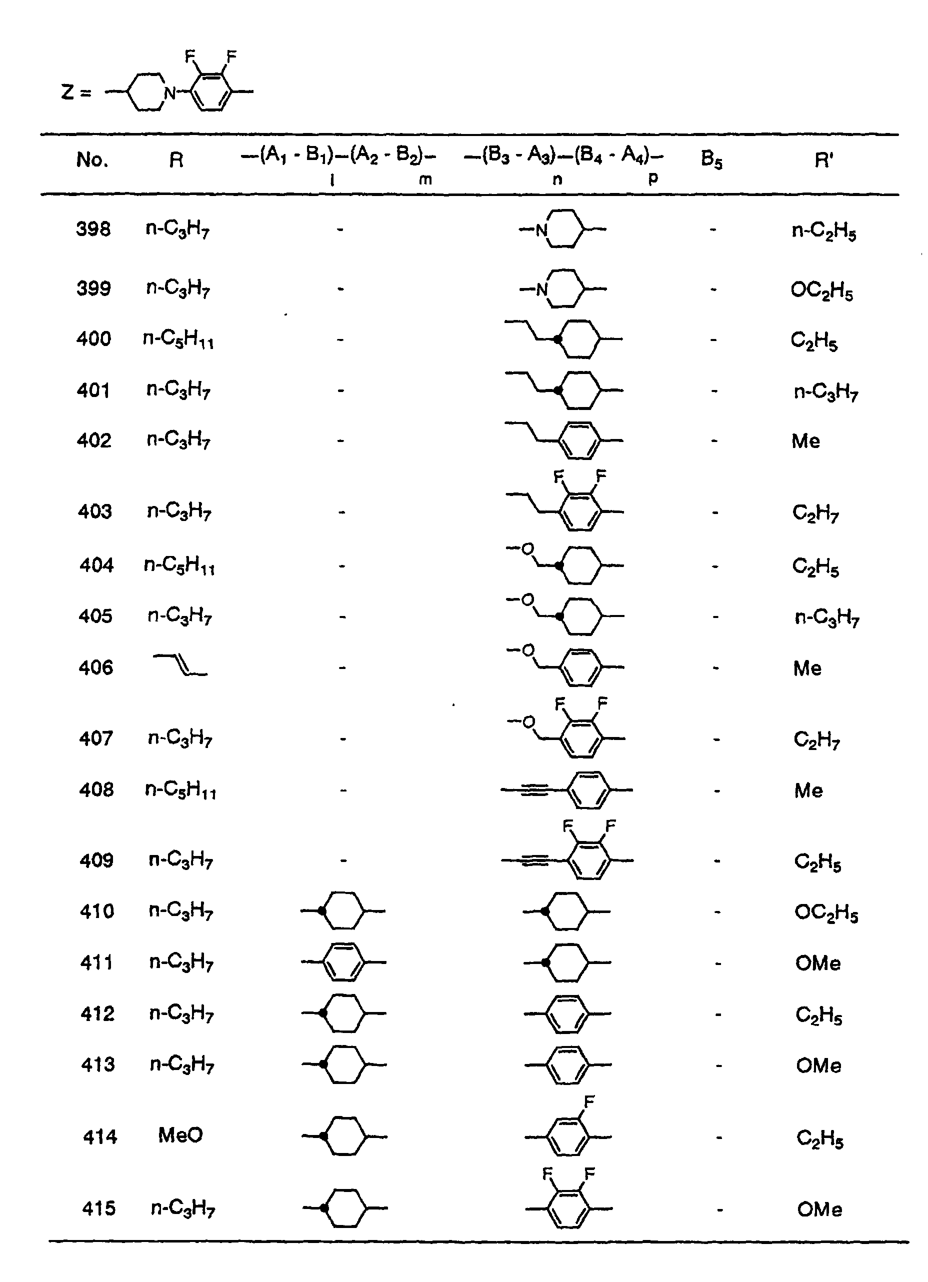

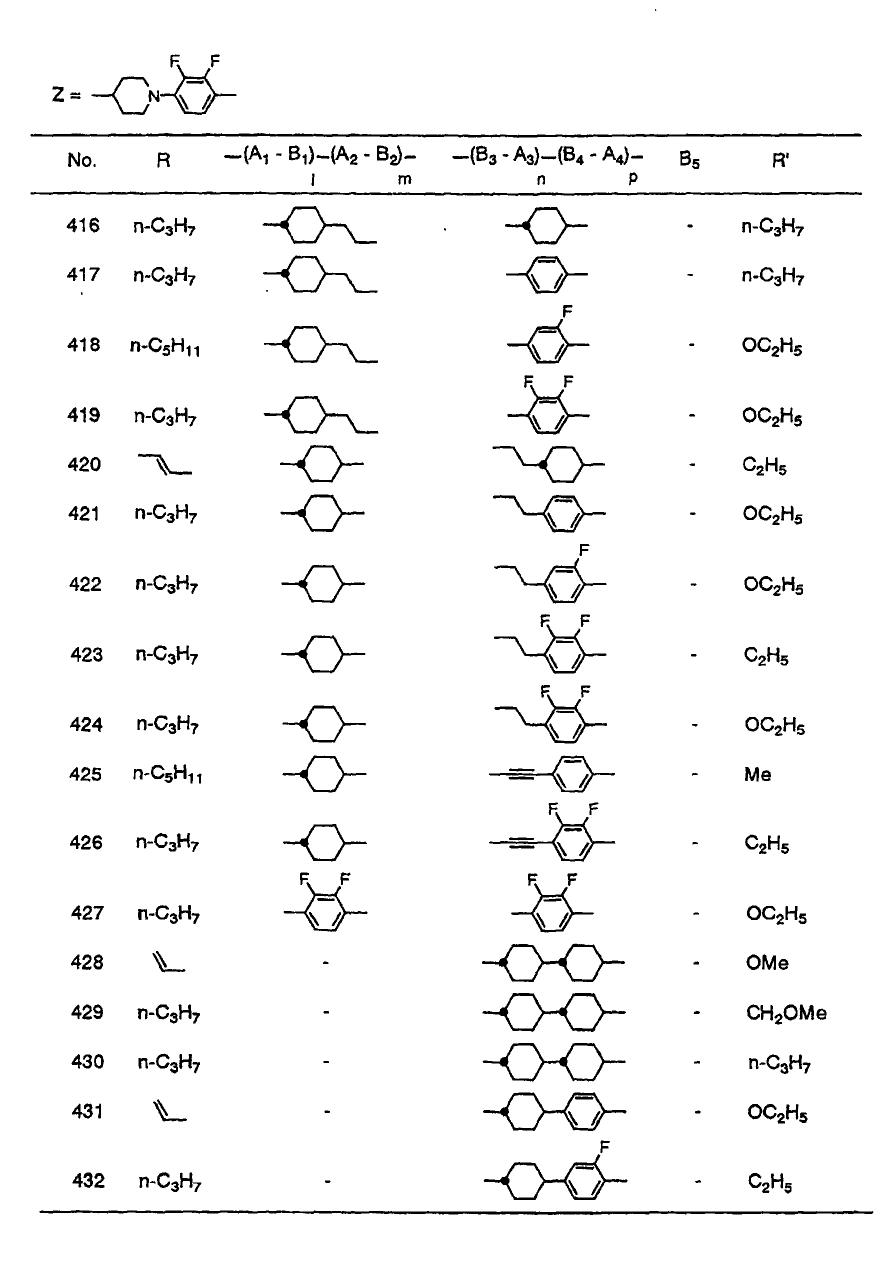

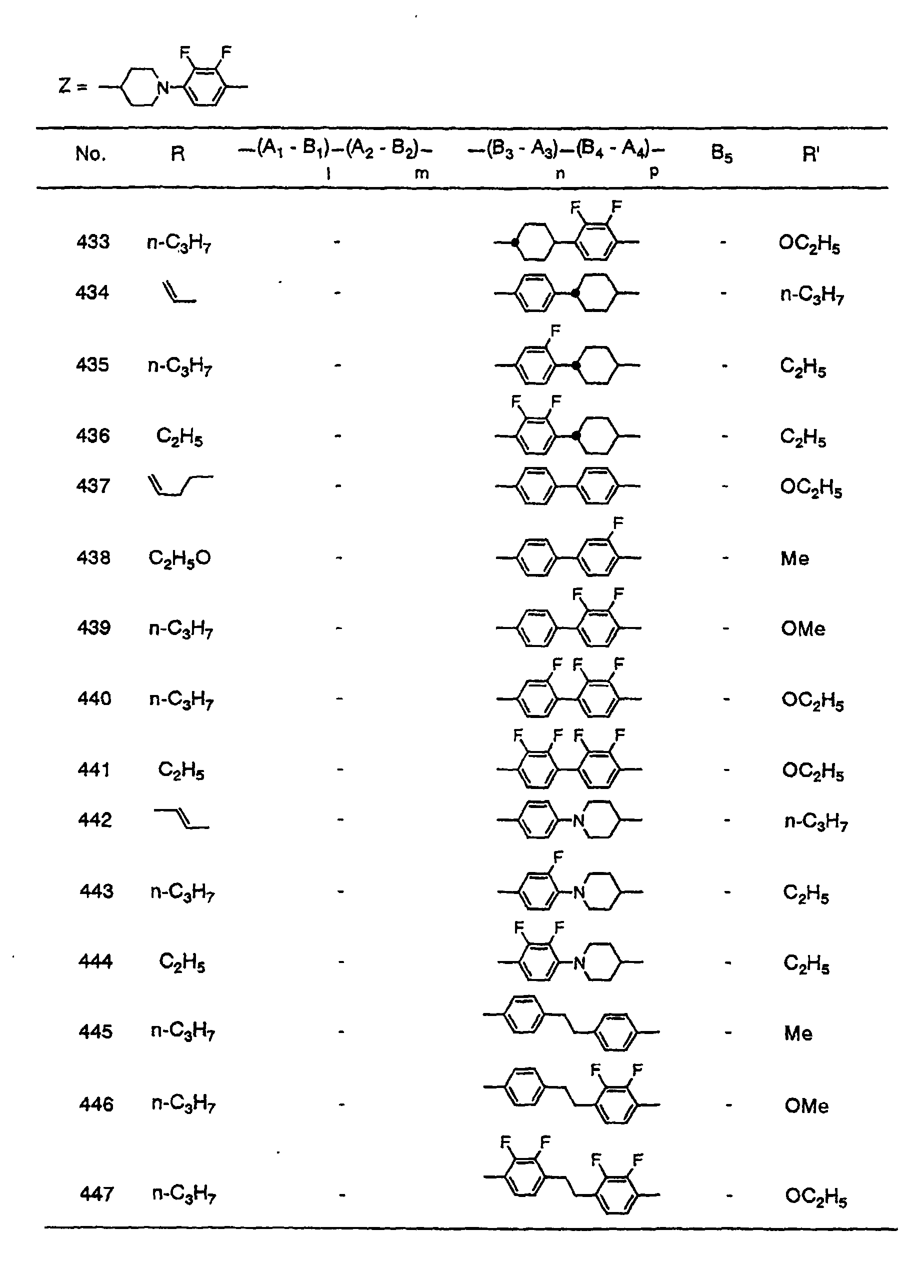

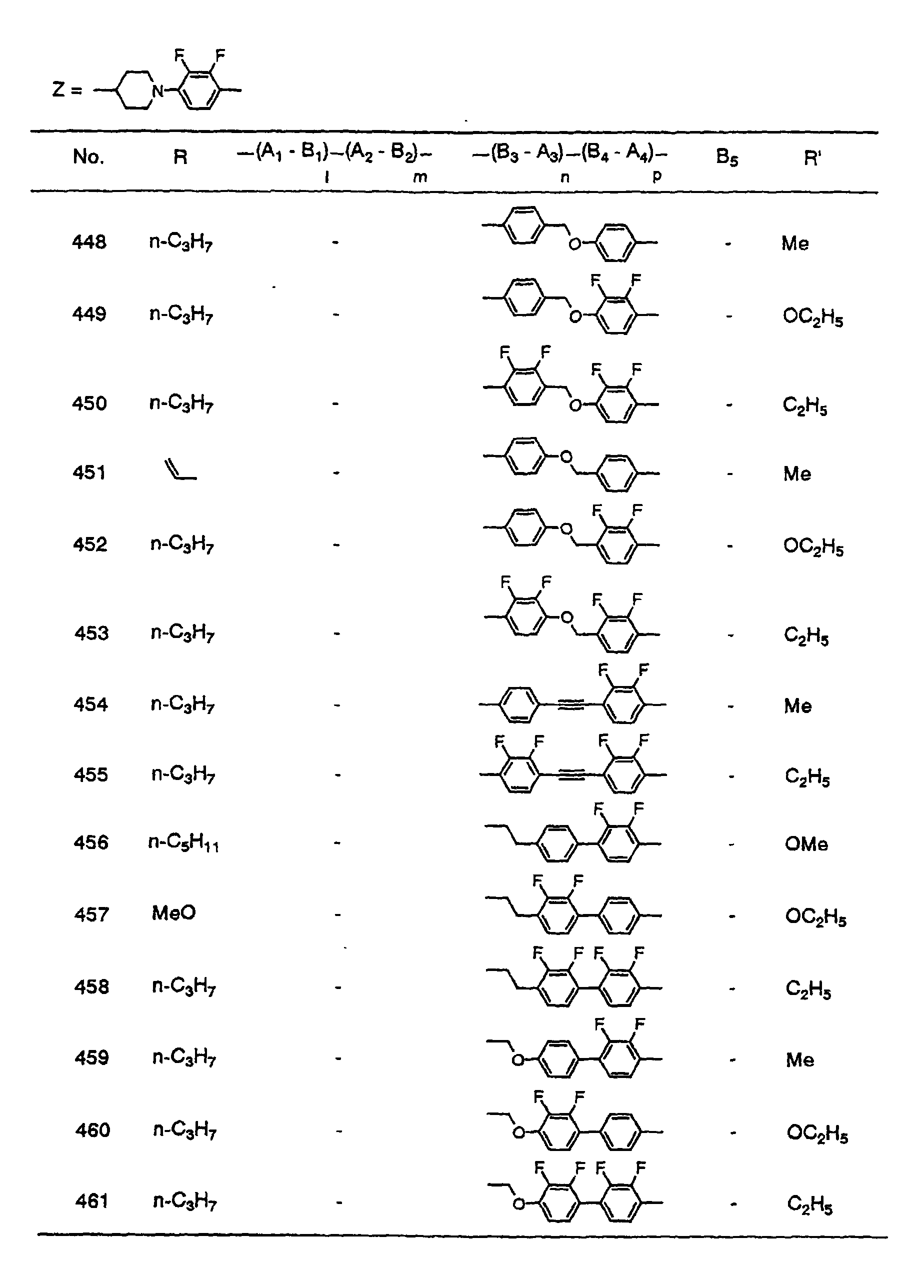

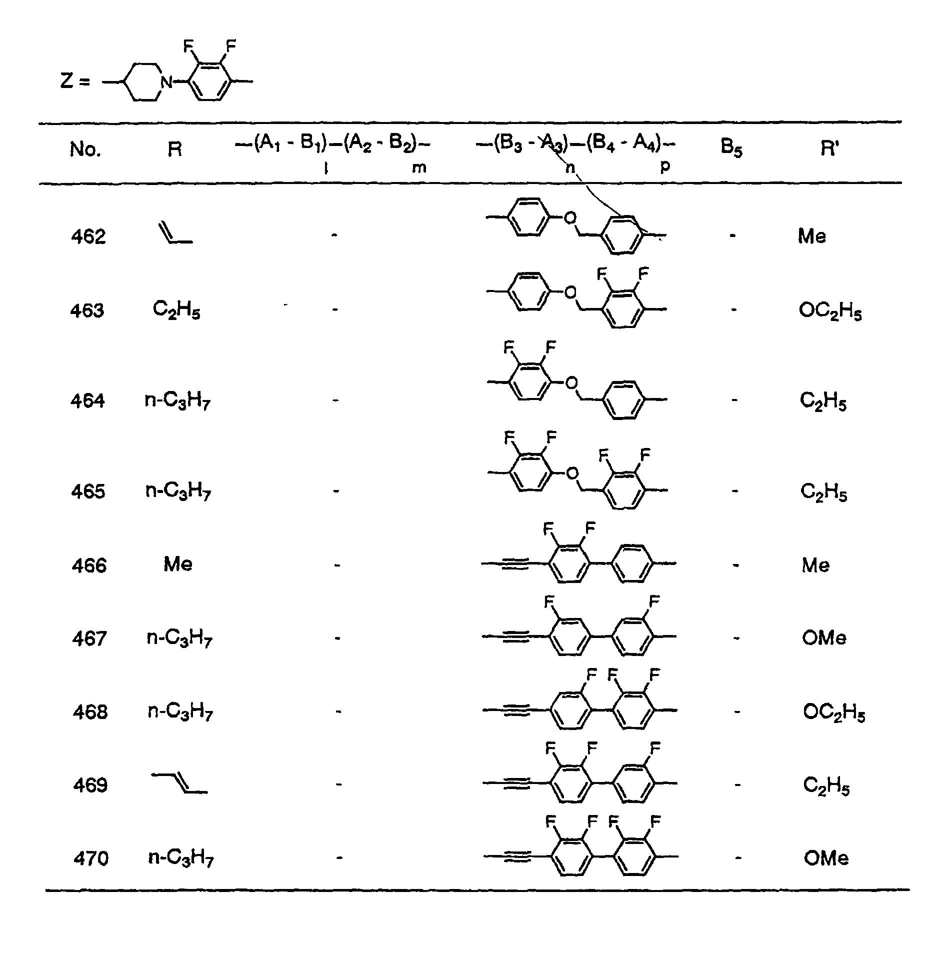

### Example 14 (Application Example 1)

The nematic liquid crystal composition (hereinafter referred to as liquid crystal composition A1) comprising:
- 4-(trans-4-propylcyclohexyl)benzonitrile: 24% by weight
- 4-(trans-4-pentylcyclohexyl)benzonitrile: 36% by weight
- 4-(trans-4-heptylcyclohexyl)benzonitrile: 25% by weight
- 4-(trans-4-propylphenyl)benzonitrile: 15% by weight
had the following characteristics:
Clearing point (Cp): 71.7°C,
Threshold voltage (Vth) in a cell thickness of 9 µm: 1.78 V,
Δε: 11.0,
Δn: 0.137, and
Viscosity (η20) at 20°C: 26.3 mPa·s.

85 parts by weight of this liquid crystal composition A1 and 15 parts by weight of 1-(4-cyanophenyl)-4-piperidone 2-propylpropyleneacetal (Compound No. 11) obtained in Example 1 were mixed to prepare liquid crystal composition B1. This liquid crystal composition B1 had the following physical properties:
Cp: 49.4°C, Vth: 1.37 V, Δε: 10.9, Δn: 0.116 and
η20: 38.3 mPa·s.

This liquid crystal composition B1 was left to stand in a freezer of -20°C for 30 days, but neither deposition of crystals nor presence of a smectic phase was observed.

### Example 15 (Application Example 2)

Liquid crystal composition B2 was prepared in the same manner as in Example 9, except that 1-(3,4-difluorophenyl)-4-piperidone 2-propylpropyleneacetal (Compound No. 18) obtained in Example 2 was substituted for Compound No. 11. This liquid crystal composition B2 had the following physical properties:
Cp: 47.2°C, Vth: 1.35 V, Δε: 10.9, Δn: 0.114 and
η20: 39.4 mPa·s.

This liquid crystal composition B2 was left to stand in a freezer of -20°C for 30 days, but neither deposition of crystals nor presence of a smectic phase was observed.

The respective compounds obtained in Examples 3 to 6 were used to prepare liquid crystal compositions B3 to B6 in the same manner. These liquid crystal compositions had the following physical properties.
- B3:: Cp: 49.4°C, Vth: 1.37 V, Δε: 10.9, Δn: 0.116,
η20: 38.3 mPa·s
- B4:: Cp: 48.5°C, Vth: 1.35 V, Δε: 11.9, Δn: 0.119,
η20: 39.2 mPa·s
- B5:: Cp: 73.1°C, Vth: 1.59 V, Δε: 11.4, Δn: 0.144,
η20: 25.1 mPa·s
- B6:: Cp: 69.3°C, Vth: 1.57 V, Δε: 13.1, Δn: 0.141,
η20: 33.2 mPa·s

### Example 16 (Application Example 3)

The compositions B7 to B9 were prepared from Compound Nos. 249-1, 254 and 297, respectively in the same manner, and measured for the physical properties, which were as follows.
- B7:: Cp: 79.7°C, Vth: 1.83 V, Δε: 10.8, Δn: 0.137,
η20: 26.7 mPa·s
- B8:: Cp: 70.3°C, Vth: 1.65 V, Δε: 12.7, Δn: 0.145,
η20: 32.8 mPa·s
- B9:: Cp: 74.5°C, Vth: 1.30 V, Δε: 16.7, Δn: 0.151,
η20: 39.8 mPa·s

### Example 17 (Application Example 4)

- 4-Ethoxyphenyl 4-propylcyclohexanecarboxylate: 17.2% by weight
- 4-Butoxyphenyl 4-propylcyclohexanecarboxylate: 27.6% by weight
- 4-Ethoxyphenyl 4-butylcyclohexanecarboxylate: 20.7% by weight
- 4-Methoxyphenyl 4-pentylcyclohexanecarboxylate: 20.7% by weight
- 4-Ethoxyphenyl 4-pentylcyclohexanecarboxylate: 13.8% by weight

The nematic liquid crystal composition (hereinafter referred to as liquid crystal composition A2) comprising the above component had the following characteristics:
Cp: 74.0°C, Δε: -1.3, Δn: 0.887 and η20: 18.9 mPa·s.

85 parts by weight of this liquid crystal composition A2 and 15 parts by weight of 1-(4-propylpiperidyl)-4-(trnas-4-propylcyclohexyl)-2,3-difluorobenzene (Compound No. 391) obtained in Example 7 were mixed to prepare liquid crystal composition B10. This liquid crystal composition B10 had the following physical properties:
Cp: 81.3°C, Δε: -1.58, Δn: 0.120 and η20: 39.1 mPa·s.

This composition was left to stand in a freezer of -20°C for 30 days, but neither deposition of crystals nor presence of a smectic phase was observed.

### Example 18 (Application Example 5)

Liquid crystal composition B11 was prepared in the same manner as in Example 11, except that 1-(4-propylpiperidyl)-4-butoxy-2,3-difluorobenzene (Compound No. 381) obtained in Example 8 was substituted for Compound No. 391. This liquid crystal composition B11 had the following physical properties:
Cp: 65.1°C, Δε: -21.5, Δn: 0.87 and η20: 20.3 mPa·s.

This liquid crystal composition B11 was left standing in a freezer of -20°C for 30 days, but neither deposition of crystals nor presence of a smectic phase was observed.

### Example 19 (Application Example 6)

Compounds No. 380 and No. 384, which were substituted for Compound No. 391, were blended with the liquid crystal composition A2 in the same manner as in Example 17 to prepare liquid crystal compositions B12 and B13, respectively. They had the following physical properties.
- B12:: Cp: 65.7°C, Δε: -2.29, Δn: 0.088 and η20: 20.7 mPa·s
- B13:: Cp: 86.5°C, Δε: -2.21, Δn: 0.088 and η20: 23.0 mPa·s

The compounds prepared in the respective examples were used to prepare the following compositions. In these cases, the compounds in the composition examples are represented by the symbols shown in Table 2.

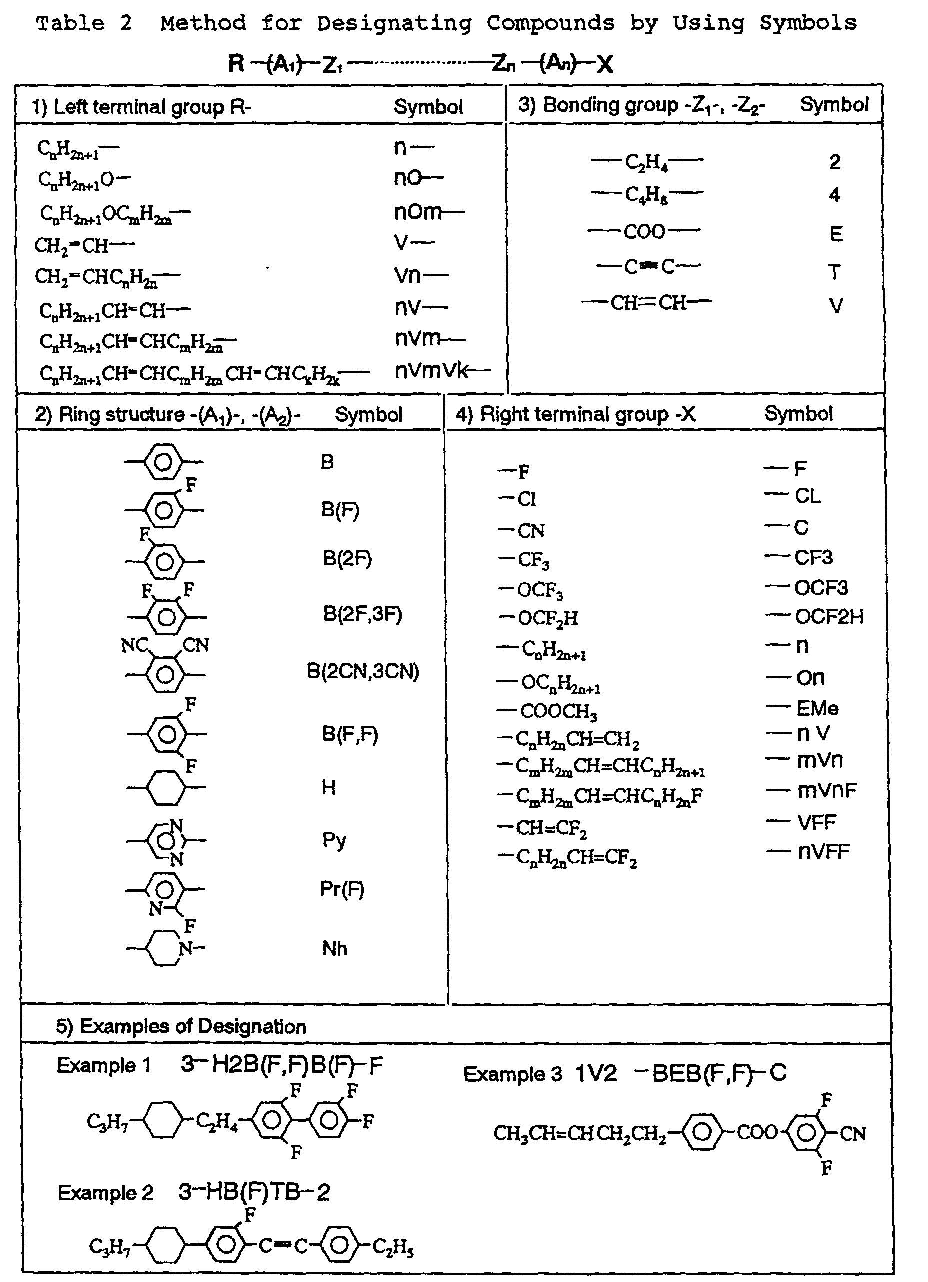

### Example 20 (Application Example 7)

| | |
|---|---|
| 7-NhB-2 | 5.0% |
| 3-NhB(2F,3F)H-3 | 4.0% |
| 3-HH-5 | 5.0% |
| 3-HH-O1 | 6.0% |
| 3-HH-O3 | 6.0% |
| 3-HB-O1 | 5.0% |
| 3-HB-O2 | 5.0% |
| 3-HB(2F,3F)-O2 | 10.0% |
| 5-HB(2F,3F)-O2 | 10.0% |
| 3-HHB(2F,3F)-O2 | 12.0% |
| 5-HHB(2F,3F)-O2 | 13.0% |
| 3-HHB(2F,3F)-2 | 4.0% |
| 3-HHEH-3 | 5.0% |
| 3-HHEH-5 | 5.0% |
| 4-HHEH-3 | 5.0% |

NI = 85.0 (°C)
Δn = 0.083
Δε = -3.0

### Example 21 (Application Example 8)

| | |
|---|---|
| 3-NhB(2F,3F)-O1 | 10.0% |
| 5-NhB(2F,3F)-O1 | 10.0% |
| 3-NhBB(2F,3F)-O2 | 14.0% |
| 5-NhBB(2F,3F)-O2 | 16.0% |
| 3-BB(2F,3F)-O2 | 12.0% |
| 2-BB(2F,3F)B-3 | 25.0% |
| 3-BB(2F,3F)B-5 | 13.0% |

NI = 104.5 (°C)
Δn = 0.157
Δε = -5.8

### Example 22 (Application Example 9)

| | |
|---|---|
| 3-NhB(2F,3F)-O2 | 3.0% |
| 3-BB(2F,3F)-O2 | 10.0% |
| 5-BB-5 | 9.0% |
| 5-BB-O6 | 9.0% |
| 5-BB-O8 | 8.0% |
| 1-BEB-5 | 6.0% |
| 3-BEB-5 | 6.0% |
| 3-HEB-O2 | 20.0% |
| 5-BBB(2F,3F)-7 | 9.0% |
| 3-H2BB(2F)-5 | 20.0% |

NI = 75.4 (°C)
Δn = 0.148
Δε = -3.2

### Example 23 (Application Example 10)

| | |
|---|---|
| 3-NhB(2F,3F)-O1 | 9.0% |
| 3-NhB(2F,3F)-O2 | 9.0% |
| 5-HNhB(2F,3F)-O1 | 6.0% |
| 5-HNhB(2F,3F)-O2 | 6.0% |
| 5-NhBB(2F,3F)-O2 | 6.0% |
| 3-HB-O1 | 15.0% |
| 3-HB-O2 | 6.0% |
| 3-HEB(2F,3F)-O2 | 9.0% |
| 2-BB2B-O2 | 6.0% |
| 1-B2BB(2F)-5 | 7.0% |
| 3-B2BB(2F)-5 | 7.0% |
| 5-B(F)BB-O2 | 7.0% |
| 3-BB(2F,3F)B-3 | 7.0% |

NI = 84.0 (°C)
η = 25.4 (mPa·s)
Δn = 0.131

### Example 24 (Application Example 11)

| | |
|---|---|
| 3-NhB(2F,3F)-O4 | 9.0% |
| 3-NhBB(2F,3F)-O2 | 5.0% |
| 5-NhBB(2F,3F)-O2 | 5.0% |
| 3-HB-O1 | 9.0% |
| 3-HB-O4 | 9.0% |
| 2-BTB-O1 | 5.0% |
| 1-BTB-O2 | 5.0% |
| 3-BTB(2F,3F)-O2 | 13.0% |
| 5-BTB(2F,3F)-O2 | 13.0% |
| 3-B(2F,3F)TB(2F,3F)-O4 | 4.0% |
| 5-B(2F,3F)TB(2F,3F)-O4 | 4.0% |
| 3-HBTB-O1 | 5.0% |
| 3-HHB(2F,3F)-O2 | 6.0% |
| 5-HBB(2F,3F)-O2 | 5.0% |
| 5-BPr(F)-O2 | 3.0% |

NI = 83.2 (°C)
η = 28.7 (mPa·s)
Δn = 0.212

### Example 25 (Application Example 12)

| | |
|---|---|
| 3-NhB(2F,3F)-O2 | 10.0% |
| 5-NhB(2F,3F)-O2 | 8.0% |
| 3-HNhB(2F,3F)-O2 | 12.0% |
| 3-HNhB(2F,3F)-O1 | 5.0% |
| 3-HB-O2 | 10.0% |
| 5-HB-3 | 8.0% |
| 5-BB(2F,3F)-O2 | 10.0% |
| 5-HHB(2F,3F)-O2 | 4.0% |
| 5-HHB(2F,3F)-1O1 | 4.0% |
| 3-HHB(2F,3F)-1 | 5.0% |
| 3-HBB-2 | 6.0% |
| 3-BB(2F,3F)B-3 | 8.0% |
| 5-B2BB(2F,3F)-O2 | 10.0% |

NI = 77.2 (°C)
Δn = 0.130
Δε = -4.9
Vth = 1.96 (V)

### Example 26 (Application Example 13)

| | |
|---|---|
| 3-HNhB(2F,3F)-O1 | 3.0% |
| 5-HNhB(2F,3F)-O1 | 4.0% |
| 3-HNhB(2F,3F)-O2 | 3.0% |
| 5-HNhB(2F,3F)-O2 | 2.0% |
| 3-NhBB(2F,3F)-O2 | 4.0% |
| 3-HB-O2 | 20.0% |
| 1O1-HH-5 | 6.0% |
| 1O1-HH-5 | 5.0% |
| 3-HB-EMe | 12.0% |
| 4-HEB-O1 | 9.0% |
| 4-HEB-O2 | 7.0% |
| 5-HEB-O1 | 8.0% |
| 3-HHB-1 | 6.0% |
| 3-HHB-3 | 6.0% |
| 4-HEB(2CN,3CN)-O4 | 3.0% |
| 2-HBEB(2CN,3CN)-O2 | 2.0% |

NI = 87.6 (°C)
η = 15.3 (mPa·s)
Δn = 0.092
Δε = -6.1
Vth = 1.43 (V)

### Example 27 (Application Example 14)

| | |
|---|---|
| 3-NhBEB(F,F)-C | 15.0% |
| 5-NhB-1 | 4.0% |
| 7-NhB-1 | 4.0% |
| 2-HNhB-1 | 6.0% |
| 3-HNhB-1 | 11.0% |
| 1V2-BEB(F,F)-C | 5.0% |
| 3-HB-C | 5.0% |
| V2-HB-C | 6.0% |
| 1-BTB-3 | 5.0% |
| 2-BTB-1 | 10.0% |
| 1O1-HH-3 | 3.0% |
| 3-HH-4 | 11.0% |
| 3-HHB-3 | 3.0% |
| 3-H2BTB-2 | 4.0% |
| 3-HB(F)TB-3 | 5.0% |
| 3-HHB-C | 3.0% |

NI = 83.2 (°C)
η = 27.1 (mPa·s)
Δn = 0.163
Δε = 17.0
Vth = 1.34 (V)

A composition prepared by adding 0.8 part by weight of the optically active compound of Formula (Op-4) to 100 parts by weight of the above composition had a pitch of 10.6 µm at 25°C.

### Example 28 (Application Example 15)

| | |
|---|---|
| 2-HNhB-1 | 4.0% |
| 5-HNhB-1 | 4.0% |
| 5-PyB-F | 4.0% |
| 3-PyB(F)-F | 4.0% |
| 2-BB-C | 5.0% |
| 4-BB-C | 4.0% |
| 5-BB-C | 5.0% |
| 2-PyB-2 | 2.0% |
| 3-PyB-2 | 2.0% |
| 4-PyB-2 | 2.0% |
| 6-PyB-O5 | 3.0% |
| 6-PyB-O6 | 3.0% |
| 6-PyB-O7 | 3.0% |
| 6-PyB-O8 | 3.0% |
| 3-PyBB-F | 6.0% |
| 4-PyBB-F | 6.0% |
| 5-PyBB-F | 6.0% |
| 3-HHB-1 | 6.0% |
| 2-H2BTB-2 | 4.0% |
| 2-H2BTB-3 | 4.0% |
| 2-H2BTB-4 | 5.0% |
| 3-H2BTB-2 | 5.0% |
| 3-H2BTB-3 | 5.0% |
| 3-H2BTB-4 | 5.0% |

NI = 93.5 (°C)
η = 35.8 (mPa·s)
Δn = 0.205
Δε = 7.2
Vth = 1.98 (V)

### Example 29 (Application Example 16)

| | |
|---|---|
| 3-HNhB(F)-F | 5.0% |
| 3-HNhB(F)-OCF3 | 4.0% |
| 5-NhB-2 | 6.0% |
| 7-NhB-1 | 6.0% |
| 5-HNhB-1 | 5.0% |
| 3O1-BEB(F)-C | 12.0% |
| 1V2-BEB(F,F)-C | 10.0% |
| 3-HEB-O4 | 4.0% |
| 3-HH-E1 | 6.0% |
| 3-HB-O2 | 6.0% |
| 7-HEB-F | 2.0% |
| 3-HHEB-F | 2.0% |
| 5-HHEB-F | 2.0% |
| 3-HBEB-F | 4.0% |
| 2O1-HBEB(F)-C | 2.0% |
| 3-HB(F)EB(F)-C | 2.0% |
| 3-HBEB(F,F)-C | 2.0% |
| 3-HHB-F | 4.0% |
| 3-HHB-O1 | 4.0% |
| 3-HHB-3 | 4.0% |
| 3-HEBEB-F | 2.0% |
| 3-HEBEB-1 | 2.0% |
| 3-HHB(F)-C | 4.0% |

NI = 88.2 (°C)
η = 33.2 (mPa·s)
Δn = 0.125
Δε = 21.0
Vth = 1.15 (V)

### Example 30 (Application Example 17)

| | |
|---|---|
| 3-HNhB(F)-F | 4.0% |
| 7-NhB-1 | 14.0% |
| 3-HB-CL | 10.0% |
| 5-HB-CL | 4.0% |
| 1O1-HH-5 | 5.0% |
| 2-HBB(F)-F | 8.0% |
| 3-HBB(F)-F | 8.0% |
| 4-HHB-CL | 8.0% |
| 5-HHB-CL | 8.0% |
| 3-H2HB(F)-CL | 4.0% |
| 3-HBB(F,F)-F | 10.0% |
| 5-H2BB(F,F)-F | 9.0% |
| 3-HB(F)VB-2 | 4.0% |
| 3-H2BTB-2 | 4.0% |

NI = 94.9 (°C)
η = 17.8 (mPa·s)
Δn = 0.132
Δε = 6.2
Vth = 1.89 (V)

### Example 31 (Application Example 18)

| | |
|---|---|
| 3-HNhB(F)-OCF3 | 5.0% |
| 5-HB-F | 12.0% |
| 6-HB-F | 9.0% |
| 7-HB-F | 7.0% |
| 2-HHB-OCF3 | 7.0% |
| 3-HHB-OCF3 | 7.0% |
| 4-HHB-OCF3 | 7.0% |
| 3-HH2B-OCF3 | 4.0% |
| 5-HH2B-OCF3 | 4.0% |
| 3-HHB(F,F)-OCF3 | 5.0% |
| 3-HBB(F)-F | 10.0% |
| 5-HBB(F)-F | 10.0% |
| 3-HH2B(F)-F | 3.0% |
| 3-HB(F)BH-3 | 3.0% |
| 5-HBBH-3 | 3.0% |
| 3-HHB(F,F)-OCF2H | 4.0% |

NI = 85.6 (°C)
η = 14.7 (mPa·s)
Δn = 0.094
Δε = 5.1
Vth = 2.21 (V)

### Comparative Example 1

A part of the physical properties of the compounds of the present invention measured according to the above examples is shown in Table 3. Also shown in Table 3 are physical properties of known compounds having a cyclohexane ring instead of a piperidine ring as comparative compounds. Among the respective physical properties, the Vth show measured values, and the others show extrapolated values.

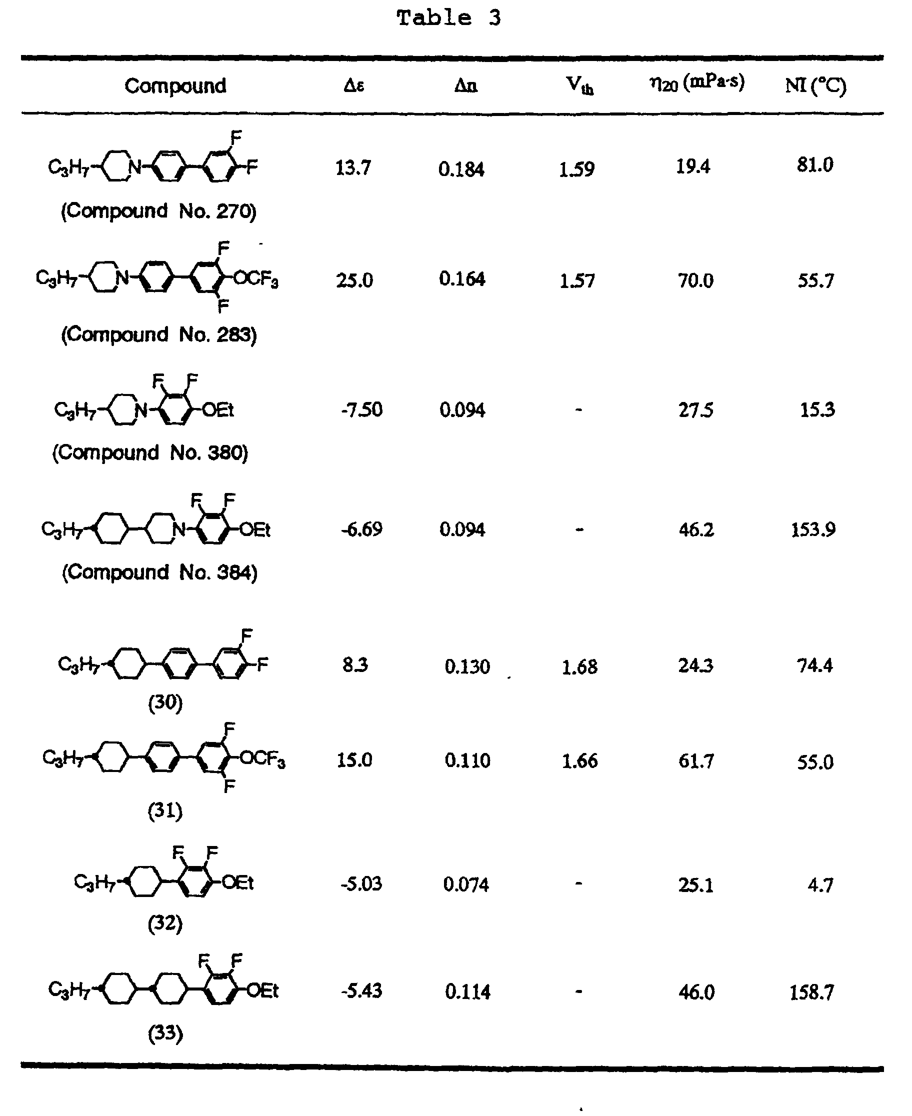

It can be found from the results shown in Table 3 that the compounds of the present invention have a large dielectric anisotropy (absolute value) as compared with the comparative compounds. It is known to the person skilled in the art that, for example, the compounds (30) and (31) have a biphenyl skeleton having a large Δn and that introduction of a fluorine atom thereinto enlarges the Δε. It has been found that Compounds No. 270 and No. 283 are increased in Δε by 5.4 and 10, respectively as compared with them. It has also been found that Compounds No. 270 and No. 283 are increased in Δn by 0.054 and 0.054, respectively as compared with the compounds (30) and (31). It has further been found that these compounds of the present invention are almost the same as or more improved than the comparative compounds in the viscosity and the clearing point. Further, it has been found that Compounds No. 380 and No. 384, which are N type compounds, are increased by 2.47 and 1.26 in terms of the absolute value as compared with the comparative compounds (32) and (33). It has also been found that these compounds are almost the same as or more improved than the comparative compounds in the viscosity and the clearing point.

It has been confirmed, though not described in the tables, that the compositions comprising the compounds of the present invention do not form crystals or a smectic phase in a freezer of -20°C for a month or longer as is the case with the compositions comprising the comparative compounds and that they have an excellent low temperature compatibility.

### Industrial Applicability The compound of the present invention represented by Formula (1) has very excellent characteristics such as:

(1) a very large dielectric anisotropy,
(2) a large refractive anisotropy, and
(3) a low viscosity and an excellent low temperature compatibility; and does not lower the clearing point of the composition when added thereto.

Accordingly, the liquid crystalline compound of the present invention as a component of a liquid crystal composition can provide a liquid crystal display element which can be driven at a low voltage and respond at a high speed and which can be used under a wide temperature condition.

## Claims

1. A novel liquid crystalline compound represented by Formula (1):
R―A¹―B¹(̵A²―B²)̵Z―B³―A³―B⁴―A⁴―B⁵―R' (1)
wherein R represents an alkyl group having 1 to 10 carbon atoms or a fluoroalkyl group having 1 to 10 carbon atoms, and R' represents an alkyl group having 1 to 10 carbon atoms, a fluoroalkyl group having 1 to 10 carbon atoms, a fluorine atom, a chlorine atom, a bromine atom or a cyano group, in which any methylene groups which are not adjacent to each other in these alkyl groups and any methylene groups and/or fluoromethylene groups which are not adjacent to each other in the fluoroalkyl groups may be substituted with oxygen atoms, sulfur atoms or -CH=CH-; the rings A¹, A², A³ and A⁴ each independently represent 1,4-cyclohexylene, 1,4-phenylene in which 1 to 2 hydrogen atoms may be substituted with fluorine atoms, dioxane-2,5-diyl, pyrimidine-2,5-diyl, pyridine-2,5-diyl, piperidine-1,4-diyl or 1-sila-1,4-cyclohexylene; B¹, B², B³, B⁴ and B⁵ each independently represent a single bond, -CH₂CH₂-, -CH=CH-, -OCH₂-, -CH₂O-, -(CH₂)₄-, -OCO- or -COO-; l, m, n and p each independently represent 0 or 1; and Z represents one group selected from the following moieties (I) to (V): 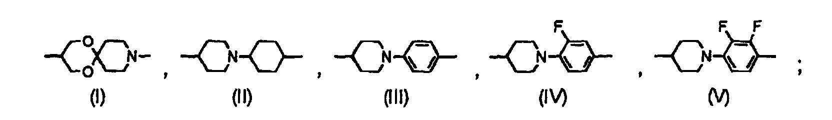 provided that
when Z is the moiety (II), l + m + n + p > 0, and the rings A¹, A², A³ and A⁴ each independently represent 1,4-phenylene in which 1 to 2 hydrogen atoms are substituted with fluorine atoms, pyrimidine-2,5-diyl, pyridine-2,5-diyl, piperidine-1,4-diyl or 1-sila-1,4-cyclohexylene; and that when Z is the moiety (III) and l = m = n = p = 0, R is an alkyl group, and R' is not a fluorine atom or a cyano group when B⁵ is a single bond.

2. The compound of Formula (1) as claimed in claim 1, wherein Z is the moiety (I).

3. The compound of Formula (1) as claimed in claim 2, wherein l + m + n + p > 0; the rings A¹, A², A³ and A⁴ each independently represent 1,4-cyclohexylene, 1,4-phenylene in which 1 to 2 hydrogen atoms may be substituted with fluorine atoms, or dioxane-2,5-diyl; B⁵ is a single bond; and R' is a fluorine atom, a cyano group or a fluoroalkoxy group.

4. The compound of Formula (1) as claimed in claim 1, wherein Z is the moiety (II).

5. The compound of Formula (1) as claimed in claim 4, wherein any of the rings A¹, A², A³ and A⁴ is 1,4-phenylene in which 1 to 2 hydrogen atoms are substituted with fluorine atoms; B⁵ is a single bond; and R' is a fluorine atom, a cyano group or a fluoroalkoxy group.

6. The compound of Formula (1) as claimed in claim 1, wherein Z is the moiety (III).

7. The compound of Formula (1) as claimed in claim 6, wherein n + p > 0; the ring A³ or A⁴ is 1,4-phenylene in which 1 to 2 hydrogen atoms may be substituted with fluorine atoms; and B⁵ is a single bond.

8. The compound of Formula (1) as claimed in claim 7, wherein R' is a fluorine atom, a cyano group or a fluoroalkoxy group.

9. The compound of Formula (1) as claimed in claim 6, wherein l + m > 0 and n = p = 0; B⁵ is a single bond; and R' is a fluorine atom, a cyano group or a fluoroalkoxy group.

10. The compound of Formula (1) as claimed in claim 1, wherein Z is the moiety (IV).

11. The compound of Formula (1) as claimed in claim 1, wherein Z is the moiety (V).

12. The compound of Formula (1) as claimed in claim 11, wherein n + p > 1; any of the rings A¹, A², A³ and A⁴ is 1,4-cyclohexylene, or 1,4-phenylene in which 1 to 2 hydrogen atoms may be substituted with fluorine atoms; B⁵ is a single bond; and R and R' each are independently alkyl groups having 1 to 10 carbon atoms, in which any methylene groups which are not adjacent to each other may be substituted with oxygen atoms or -CH=CH-, or fluoroalkyl groups having 1 to 10 carbon atoms, in which any methylene groups and/or fluoromethylene groups which are not adjacent to each other may be substituted with oxygen atoms or -CH=CH-.

13. The compound of Formula (1) as claimed in claim 11, wherein n = p = 0; B⁵ is a single bond; and R and R' each are independently alkyl groups having 1 to 10 carbon atoms, in which any methylene groups which are not adjacent to each other may be substituted with oxygen atoms or -CH=CH-, or fluoroalkyl groups, in which any methylene groups and/or fluoromethylene groups which are not adjacent to each other may be substituted with oxygen atoms or -CH=CH-.

14. A liquid crystal composition comprising at least one of the liquid crystal compounds as claimed in any one of claims 1 to 13.

15. A liquid crystal composition comprising at least one liquid crystalline compound as claimed in any one of claims 1 to 13 as a first component and at least one compound selected from the group consisting of compounds represented by Formulas (2), (3) and (4) as a second component: 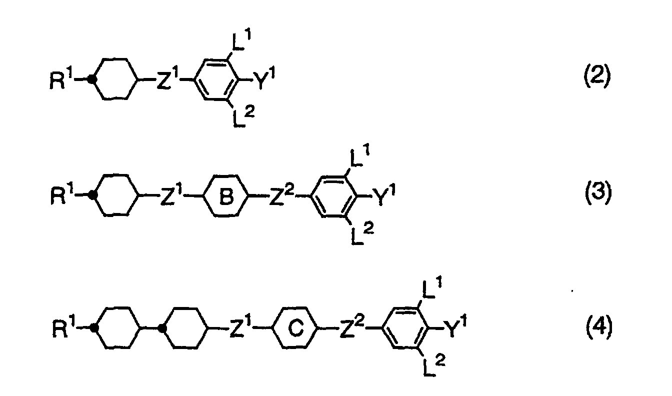 wherein R¹ represents an alkyl group having 1 to 10 carbon atoms, in which any methylene groups which are not adjacent to each other may be substituted with oxygen atoms or -CH=CH-, and in which any hydrogen atoms may be substituted with fluorine atoms; Y¹ represents a fluorine atom, a chlorine atom, -OCF₃, -OCF₂H, -CF₃, -CF₂H, -CFH₂, -OCF₂CF₂H or -OCF₂CFHCF₃; L¹ and L² each independently represent a hydrogen atom or a fluorine atom; Z¹ and Z² each independently represent -(CH₂)₂-, -(CH₂)₄-, -COO-, -CF₂O-, -OCF₂-, -CH=CH- or a single bond; the ring B represents trans-1,4-cyclohexylene, 1,3-dioxane-2,5-diyl, or 1,4-phenylene in which hydrogen atoms may be substituted with fluorine atoms; and the ring C represents trans-1,4-cyclohexylene, or 1,4-phenylene in which hydrogen atoms may be substituted with fluorine atoms.

16. A liquid crystal composition comprising at least one compound as claimed in any one of claims 1 to 13 as a first component and at least one compound selected from the group consisting of compounds represented by Formulas (5) and (6) as a second component: 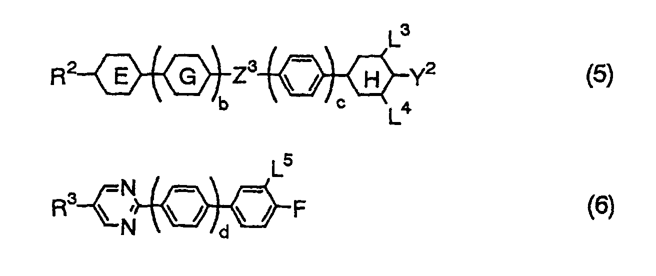 wherein R² and R³ each independently represent an alkyl group having 1 to 10 carbon atoms, in which any methylene groups which are not adjacent to each other may be substituted with oxygen atoms or -CH=CH-, and in which any hydrogen atoms may be substituted with fluorine atoms; Y² represents -CN or -C≡C-CN; the ring E represents trans-1,4-cyclohexylene, 1,4-phenylene, 1,3-dioxane-2,5-diyl or pyrimidine-2,5-diyl; the ring G represents trans-1,4-cyclohexylene, 1,4-phenylene in which hydrogen atoms may be substituted with fluorine atoms, or pyrimidine-2,5-diyl; the ring H represents trans-1,4-cyclohexylene or 1,4-phenylene; Z³ represents 1,2-ethylene, -COO- or a single bond; L³, L⁴ and L⁵ each independently represent a hydrogen atom or a fluorine atom; and b, c and d each independently represent 0 or 1.

17. A liquid crystal composition comprising at least one compound as claimed in any one of claims 1 to 13 as a first component, at least one compound selected from the group consisting of the compounds represented by Formulas (2), (3) and (4) as a second component and at least one compound selected from the group consisting of compounds represented by Formulas (7), (8) and (9) as a third component: 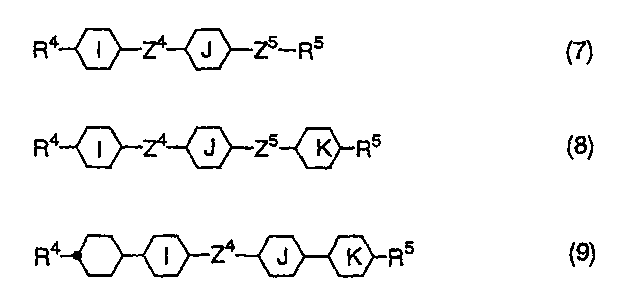 wherein R⁴ and R⁵ each independently represent an alkyl group having 1 to 10 carbon atoms, in which any methylene groups which are not adjacent to each other may be substituted with oxygen atoms or -CH=CH-, and in which any hydrogen atoms may be substituted with fluorine atoms; I, J and K each independently represent trans-1,4-cyclohexylene, pyrimidine-2,5-diyl, or 1,4-phenylene in which hydrogen atoms may be substituted with fluorine atoms; and Z⁴ and Z⁵ each independently represent -C≡C-, -COO-, -CH₂CH₂-, -CH=CH- or a single bond.

18. A liquid crystal composition comprising at least one compound as claimed in any one of claims 1 to 13 as a first component and at least one compound selected from the group consisting of compounds represented by Formulas (10), (11) and (12) as a second component: 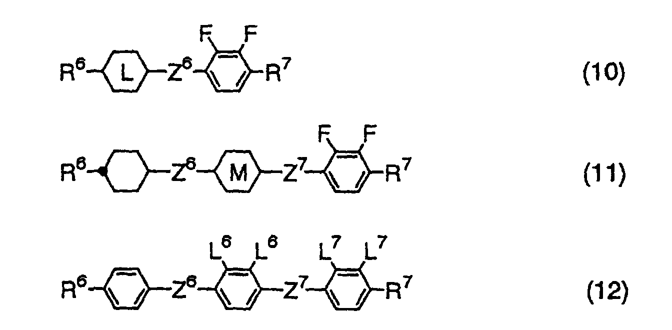 wherein R⁶ and R⁷ each independently represent an alkyl group having 1 to 10 carbon atoms, in which any methylene groups which are not adjacent to each other may be substituted with oxygen atoms or -CH=CH-, and in which any hydrogen atoms may be substituted with fluorine atoms; the rings L and M each independently represent trans-1,4-cyclohexylene or 1,4-phenylene; L⁶ and L⁷ each independently represent a hydrogen atom or a fluorine atom but do not represent hydrogen atoms at the same time; and Z⁶ and Z⁷ each independently represent -CH₂CH₂-, -CH₂O- or a single bond.

19. A liquid crystal composition comprising at least one compound as claimed in any one of claims 1 to 13 as a first component, at least one compound selected from the group consisting of the compounds represented by Formulas (7), (8) and (9) as a second component and at least one compound selected from the group consisting of the compounds represented by Formulas (10), (11) and (12) as a third component.

20. A liquid crystal composition comprising at least one compound as claimed in any one of claims 1 to 13 as a first component, at least one compound selected from the group consisting of the compounds represented by Formulas (5) and (6) as a second component and at least one compound selected from the group consisting of the compounds represented by Formulas (7), (8) and (9) as a third component.

21. A liquid crystal composition comprising at least one compound as claimed in any one of claims 1 to 13 as a first component, at least one compound selected from the group consisting of the compounds represented by Formulas (2), (3) and (4) as a second component, at least one compound selected from the group consisting of the compounds represented by Formulas (5) and (6) as a third component and at least one compound selected from the group consisting of the compounds represented by Formulas (7), (8) and (9) as a fourth component.

22. A liquid crystal composition further comprising at least one optically active compound in addition to the liquid crystal composition as claimed in any one of claims 14 to 21.

23. A liquid crystal display element comprising the liquid crystal composition as claimed in any one of claims 14 to 22.

## Amended claims

### Amended claims under Art. 19.1 PCT

1. (Amended) A novel liquid crystalline compound represented by Formula (1): 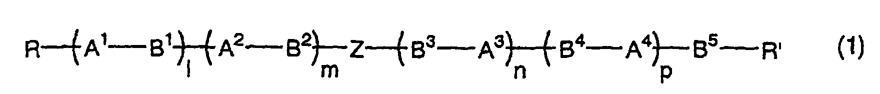 wherein R represents an alkyl group having 1 to 10 carbon atoms or a fluoroalkyl group having 1 to 10 carbon atoms, and R' represents an alkyl group having 1 to 10 carbon atoms, a fluoroalkyl group having 1 to 10 carbon atoms, a fluorine atom, a chlorine atom, a bromine atom or a cyano group, in which any methylene groups which are not adjacent to each other in these alkyl groups and any methylene groups and/or fluoromethylene groups which are not adjacent to each other in the fluoroalkyl groups may be substituted with oxygen atoms, sulfur atoms or -CH=CH-; the rings A¹, A², A³ and A⁴ each independently represent 1,4-cyclohexylene, 1,4-phenylene in which 1 to 2 hydrogen atoms may be substituted with fluorine atoms, dioxane-2,5-diyl, pyrimidine-2,5-diyl, pyridine-2,5-diyl, piperidine-1,4-diyl or 1-sila-1,4-cyclohexylene; B¹, B², B³, B⁴ and B⁵ each independently represent a single bond, -CH₂CH₂-, -CH=CH-, -OCH₂-, -CH₂O-, -(CH₂)₄-, -OCO- or -COO-; l, m, n and p each independently represent 0 or 1; and Z represents one group selected from the following moieties (I) to (V):
